# EUROPEAN PATENT APPLICATION

(11) **EP 4 039 808 A1**
(43) Date of publication of application: **10.08.2022**
(21) Application number: 21155865.5
(22) Date of filing: 08.02.2021
(51) Int. Cl.: C12N 15/113, A61K 39/00, A61P 35/00

(54) **GUIDE RNAS AND USES THEREOF**

(71) Applicant: Ospedale San Raffaele S.r.l., 20132 Milano (IT); Fondazione Centro San Raffaele, 20132 Milano (IT)
(72) Inventor: Bonini, Maria Chiara, 20132 Milano (IT); Cianciotti, Beatrice Claudia, 20132 Milano (IT); Ruggiero, Eliana, 20132 Milano (IT); Potenza, Alessia, 20132 Milano (IT)
(74) Representative: Turri, Elisa

(57) **Abstract**

The present invention is related to an isolated guide ribonucleic acid (gRNA) comprising a guide sequence targeting an inhibitory receptor (IR), a TCR α (TRAC) constant region or a β chain (TRBC1/2) constant region target sequence, wherein said guide sequence is selected from the group consisting of SEQ ID NOs: 1-27.

SEQUENCE LISTING

<110>  Ospedale San Raffaele S.r.l.
       Fondazione Centro San Raffaele

<120>  GUIDE RNAS AND USES THEREOF

<130>  BE 146281

<160>  121

<170>  PatentIn version 3.5

<210>  1
<211>  20
<212>  RNA
<213>  Artificial Sequence

<220>
<223>  guide sequence

<400>  1
gagucacauu cucuaugguc                                                   20


<210>  2
<211>  20
<212>  RNA
<213>  Artificial Sequence

<220>
<223>  guide sequence

<400>  2
aauguggcaa cguggugcuc                                                   20


<210>  3
<211>  20
<212>  RNA
<213>  Artificial Sequence

<220>
<223>  guide sequence

<400>  3
cuaaaugggg auuuccgcaa                                                   20


<210>  4
<211>  20
<212>  RNA
<213>  Artificial Sequence

<220>
<223>  guide sequence

<400>  4
auccccauuu agccaguauc                                                   20


<210>  5
<211>  20

<212>  RNA
<213>  Artificial Sequence

<220>
<223>  guide sequence

<400>  5
cucucugccg agucggugca                                                   20


<210>  6
<211>  20
<212>  RNA
<213>  Artificial Sequence

<220>
<223>  guide sequence

<400>  6
gugaagucuc ucugccgagu                                                   20


<210>  7
<211>  20
<212>  RNA
<213>  Artificial Sequence

<220>
<223>  guide sequence

<400>  7
ugccccaugc auaguuaccu                                                   20


<210>  8
<211>  20
<212>  RNA
<213>  Artificial Sequence

<220>
<223>  guide sequence

<400>  8
aggucacccc ugcaccgacu                                                   20


<210>  9
<211>  20
<212>  RNA
<213>  Artificial Sequence

<220>
<223>  guide sequence

<400>  9
uggcccaggu aacuaugcau                                                   20


<210>  10
<211>  20
<212>  RNA
<213>  Artificial Sequence

<220>
<223>  guide sequence

<400>  10
ccaaggaugc uuaccaccag                                                   20


<210>  11
<211>  20
<212>  RNA
<213>  Artificial Sequence

<220>
<223>  guide sequence

<400>  11
caccgcggcg cgguacucgc                                                   20


<210>  12
<211>  20
<212>  RNA
<213>  Artificial Sequence

<220>
<223>  guide sequence

<400>  12
gcucagcacc guguagcggc                                                   20


<210>  13
<211>  20
<212>  RNA
<213>  Artificial Sequence

<220>
<223>  guide sequence

<400>  13
accuucgucu guaugcuguu                                                   20


<210>  14
<211>  20
<212>  RNA
<213>  Artificial Sequence

<220>
<223>  guide sequence

<400>  14
accaaacagc auacagacga                                                   20


<210>  15
<211>  20
<212>  RNA
<213>  Artificial Sequence

<220>
<223>  guide sequence

<400>  15
aguugagaaa ccccgccuac                                                   20


<210>  16
<211>  20
<212>  RNA
<213>  Artificial Sequence

<220>
<223>  guide sequence

<400>  16
guugagaaac cccgccuaca                                                   20


<210>  17
<211>  20
<212>  RNA
<213>  Artificial Sequence

<220>
<223>  guide sequence

<400>  17
uugagaaacc ccgccuacag                                                   20


<210>  18
<211>  20
<212>  RNA
<213>  Artificial Sequence

<220>
<223>  guide sequence

<400>  18
uguagcaccg cccagacgac                                                   20


<210>  19
<211>  20
<212>  RNA
<213>  Artificial Sequence

<220>
<223>  guide sequence

<400>  19
cgucugggcg gugcuacaac                                                   20


<210>  20
<211>  20
<212>  RNA
<213>  Artificial Sequence

<220>
<223>  guide sequence

<400>  20
aggugccgcu gucauugcgc                                                   20

<210>  21
<211>  20
<212>  RNA
<213>  Artificial Sequence

<220>
<223>  guide sequence

<400>  21
ccugcucgug gugaccgaag                                                   20


<210>  22
<211>  20
<212>  RNA
<213>  Artificial Sequence

<220>
<223>  guide sequence

<400>  22
aaggauggcu aggauauucu                                                   20


<210>  23
<211>  20
<212>  RNA
<213>  Artificial Sequence

<220>
<223>  guide sequence

<400>  23
aagaauaucc uagccauccu                                                   20


<210>  24
<211>  20
<212>  RNA
<213>  Artificial Sequence

<220>
<223>  guide sequence

<400>  24
gagaaucaaa aucggugaau                                                   20


<210>  25
<211>  20
<212>  RNA
<213>  Artificial Sequence

<220>
<223>  guide sequence

<400>  25
ucuccgagag cccguagaac                                                   20


<210>  26
<211>  20

<212>  RNA
<213>  Artificial Sequence

<220>
<223>  guide sequence

<400>  26
caaacacagc gaccucgggu                                                    20


<210>  27
<211>  20
<212>  RNA
<213>  Artificial Sequence

<220>
<223>  guide sequence

<400>  27
ugacagcgga agugguugcg                                                    20


<210>  28
<211>  23
<212>  DNA
<213>  Homo sapiens

<400>  28
gagtcacatt ctctatggtc agg                                                23


<210>  29
<211>  23
<212>  DNA
<213>  Homo sapiens

<400>  29
ctaaatgggg atttccgcaa agg                                                23


<210>  30
<211>  23
<212>  DNA
<213>  Homo sapiens

<400>  30
atccccattt agccagtatc tgg                                                23


<210>  31
<211>  23
<212>  DNA
<213>  Homo sapiens

<400>  31
caccgcggcg cggtactcgc cgg                                                23


<210>  32
<211>  23
<212>  DNA
<213>  Homo sapiens

<400>  32
agttgagaaa ccccgcctac agg                                              23


<210>  33
<211>  23
<212>  DNA
<213>  Homo sapiens

<400>  33
gagaatcaaa atcggtgaat agg                                              23


<210>  34
<211>  23
<212>  DNA
<213>  Homo sapiens

<400>  34
caaacacagc gacctcgggt ggg                                              23


<210>  35
<211>  23
<212>  DNA
<213>  Homo sapiens

<400>  35
aatgtggcaa cgtggtgctc agg                                              23


<210>  36
<211>  23
<212>  DNA
<213>  Homo sapiens

<400>  36
ctctctgccg agtcggtgca ggg                                              23


<210>  37
<211>  23
<212>  DNA
<213>  Homo sapiens

<400>  37
gtgaagtctc tctgccgagt cgg                                              23


<210>  38
<211>  23
<212>  DNA
<213>  Homo sapiens

<400>  38
tgccccatgc atagttacct ggg                                              23


<210>  39
<211>  23
<212>  DNA
<213>  Homo sapiens

<400>  39
aggtcacccc tgcaccgact cgg                                               23


<210>  40
<211>  23
<212>  DNA
<213>  Homo sapiens

<400>  40
tggcccaggt aactatgcat ggg                                               23


<210>  41
<211>  23
<212>  DNA
<213>  Homo sapiens

<400>  41
ccaaggatgc ttaccaccag ggg                                               23


<210>  42
<211>  23
<212>  DNA
<213>  Homo sapiens

<400>  42
gcaaagtggc cgtcgtggct ggg                                               23


<210>  43
<211>  23
<212>  DNA
<213>  Homo sapiens

<400>  43
acagagcaaa gtggccgtcg tgg                                               23


<210>  44
<211>  23
<212>  DNA
<213>  Homo sapiens

<400>  44
gctcagcacc gtgtagcggc ggg                                               23


<210>  45
<211>  23
<212>  DNA
<213>  Homo sapiens

<400>  45
gaacggcatc ccagccacga cgg                                               23


<210>  46
<211>  23
<212>  DNA
<213>  Homo sapiens

<400>  46
accttcgtct gtatgctgtt tgg                                               23


<210>  47
<211>  23
<212>  DNA
<213>  Homo sapiens

<400>  47
accaaacagc atacagacga agg                                               23


<210>  48
<211>  23
<212>  DNA
<213>  Homo sapiens

<400>  48
ctcccgagat gctaaccaca tgg                                               23


<210>  49
<211>  23
<212>  DNA
<213>  Homo sapiens

<400>  49
gttgagaaac cccgcctaca ggg                                               23


<210>  50
<211>  23
<212>  DNA
<213>  Homo sapiens

<400>  50
ttgagaaacc ccgcctacag ggg                                               23


<210>  51
<211>  23
<212>  DNA
<213>  Homo sapiens

<400>  51
aaggatggct aggatattct tgg                                               23


<210>  52
<211>  23
<212>  DNA
<213>  Homo sapiens

<400>  52
aagaatatcc tagccatcct tgg                                               23


<210>  53
<211>  23
<212>  DNA
<213>  Homo sapiens

<400>  53
tctccgagag cccgtagaac tgg                                              23


<210>  54
<211>  23
<212>  DNA
<213>  Homo sapiens

<400>  54
tgacagcgga agtggttgcg ggg                                              23


<210>  55
<211>  23
<212>  DNA
<213>  Homo sapiens

<400>  55
tgtagcaccg cccagacgac tgg                                              23


<210>  56
<211>  23
<212>  DNA
<213>  Homo sapiens

<400>  56
cgtctgggcg gtgctacaac tgg                                              23


<210>  57
<211>  23
<212>  DNA
<213>  Homo sapiens

<400>  57
aggtgccgct gtcattgcgc cgg                                              23


<210>  58
<211>  23
<212>  DNA
<213>  Homo sapiens

<400>  58
cctgctcgtg gtgaccgaag ggg                                              23


<210>  59
<211>  23
<212>  DNA
<213>  Homo sapiens

<400>  59
accggtctgg gcggtgctac aac                                              23


<210>  60
<211>  23
<212>  DNA
<213>  Artificial Sequence

<220>
<223>  oligonucleotide

<400>  60
accgggtgcc gctgtcattg cgc                                              23


<210>  61
<211>  23
<212>  DNA
<213>  Artificial Sequence

<220>
<223>  oligonucleotide

<400>  61
accgctgctc gtggtgaccg aag                                              23


<210>  62
<211>  24
<212>  DNA
<213>  Artificial Sequence

<220>
<223>  oligonucleotide

<400>  62
accggcagtt gtgtgacacg gaag                                             24


<210>  63
<211>  23
<212>  DNA
<213>  Artificial Sequence

<220>
<223>  oligonucleotide

<400>  63
accgtaaatg gggatttccg caa                                              23


<210>  64
<211>  23
<212>  DNA
<213>  Artificial Sequence

<220>
<223>  oligonucleotide

<400>  64
accgatgtgg caacgtggtg ctc                                              23


<210>  65
<211>  23
<212>  DNA
<213>  Artificial Sequence

<220>
<223>  oligonucleotide

<400>  65
accgtcccca tttagccagt atc                                              23


<210>  66
<211>  23
<212>  DNA
<213>  Artificial Sequence

<220>
<223>  oligonucleotide

<400>  66
accgagtcac attctctatg gtc                                              23


<210>  67
<211>  23
<212>  DNA
<213>  Artificial Sequence

<220>
<223>  oligonucleotide

<400>  67
accgtctctg ccgagtcggt gca                                              23


<210>  68
<211>  23
<212>  DNA
<213>  Artificial Sequence

<220>
<223>  oligonucleotide

<400>  68
accgtgaagt ctctctgccg agt                                              23


<210>  69
<211>  23
<212>  DNA
<213>  Artificial Sequence

<220>
<223>  oligonucleotide

<400>  69
accggcccca tgcatagtta cct                                              23


<210>  70
<211>  23
<212>  DNA
<213>  Artificial Sequence

<220>
<223>  oligonucleotide

<400>  70
accgggtcac ccctgcaccg act                                              23

<210>  71
<211>  23
<212>  DNA
<213>  Artificial Sequence

<220>
<223>  oligonucleotide

<400>  71
accgggccca ggtaactatg cat                                               23


<210>  72
<211>  23
<212>  DNA
<213>  Artificial Sequence

<220>
<223>  oligonucleotide

<400>  72
accgcaagga tgcttaccac cag                                               23


<210>  73
<211>  24
<212>  DNA
<213>  Artificial Sequence

<220>
<223>  oligonucleotide

<400>  73
accgagaaat caaaatcggt gaat                                              24


<210>  74
<211>  23
<212>  DNA
<213>  Artificial Sequence

<220>
<223>  oligonucleotide

<400>  74
aaacgtcgtc tgggcggtgc tac                                               23


<210>  75
<211>  23
<212>  DNA
<213>  Artificial Sequence

<220>
<223>  oligonucleotide

<400>  75
aaacgttgta gcaccgccca gac                                               23


<210>  76
<211>  23

<212>  DNA
<213>  Artificial Sequence

<220>
<223>  oligonucleotide

<400>  76
aaacgcgcaa tgacagcggc acc                                              23


<210>  77
<211>  23
<212>  DNA
<213>  Artificial Sequence

<220>
<223>  oligonucleotide

<400>  77
aaaccttcgg tcaccacgag cag                                              23


<210>  78
<211>  24
<212>  DNA
<213>  Artificial Sequence

<220>
<223>  oligonucleotide

<400>  78
aaacgaaggc acagtgtgtt gacg                                             24


<210>  79
<211>  23
<212>  DNA
<213>  Artificial Sequence

<220>
<223>  oligonucleotide

<400>  79
aaacttgcgg aaatccccat tta                                              23


<210>  80
<211>  23
<212>  DNA
<213>  Artificial Sequence

<220>
<223>  oligonucleotide

<400>  80
aaacgagcac cacgttgcca cat                                              23


<210>  81
<211>  23
<212>  DNA
<213>  Artificial Sequence

<220>
<223>  oligonucleotide

<400>  81
aaacgatact ggctaaatgg gga                                              23


<210>  82
<211>  23
<212>  DNA
<213>  Artificial Sequence

<220>
<223>  oligonucleotide

<400>  82
aaacgaccat agagaatgtg act                                              23


<210>  83
<211>  23
<212>  DNA
<213>  Artificial Sequence

<220>
<223>  oligonucleotide

<400>  83
aaactgcacc gactcggcag aga                                              23


<210>  84
<211>  23
<212>  DNA
<213>  Artificial Sequence

<220>
<223>  oligonucleotide

<400>  84
aaacactcgg cagagagact tca                                              23


<210>  85
<211>  23
<212>  DNA
<213>  Artificial Sequence

<220>
<223>  oligonucleotide

<400>  85
aaacaggtaa ctatgcatgg ggc                                              23


<210>  86
<211>  23
<212>  DNA
<213>  Artificial Sequence

<220>
<223>  oligonucleotide

<400>  86
aaacagtcgg tgcaggggtg acc                                               23


<210>  87
<211>  23
<212>  DNA
<213>  Artificial Sequence

<220>
<223>  oligonucleotide

<400>  87
aaacatgcat agttacctgg gcc                                               23


<210>  88
<211>  23
<212>  DNA
<213>  Artificial Sequence

<220>
<223>  oligonucleotide

<400>  88
aaacctggtg gtaagcatcc ttg                                               23


<210>  89
<211>  24
<212>  DNA
<213>  Artificial Sequence

<220>
<223>  oligonucleotide

<400>  89
aaacattcac cgattttgat ttct                                              24


<210>  90
<211>  18
<212>  DNA
<213>  Artificial Sequence

<220>
<223>  oligonucleotide

<400>  90
agtttccctt ccgctcac                                                     18


<210>  91
<211>  20
<212>  DNA
<213>  Artificial Sequence

<220>
<223>  oligonucleotide

<400>  91
cctgacccgt cattctacag                                                   20

<210>  92
<211>  18
<212>  DNA
<213>  Artificial Sequence

<220>
<223>  oligonucleotide

<400>  92
gagggatgtg agcaggtg                                                    18


<210>  93
<211>  20
<212>  DNA
<213>  Artificial Sequence

<220>
<223>  oligonucleotide

<400>  93
gcatctctgt cctctagctc                                                  20


<210>  94
<211>  20
<212>  DNA
<213>  Artificial Sequence

<220>
<223>  oligonucleotide

<400>  94
gcctatctgc cctgcttcta                                                  20


<210>  95
<211>  20
<212>  DNA
<213>  Artificial Sequence

<220>
<223>  oligonucleotide

<400>  95
ttacaggctc ccgtaaccat                                                  20


<210>  96
<211>  20
<212>  DNA
<213>  Artificial Sequence

<220>
<223>  oligonucleotide

<400>  96
tatccaggca ggtttggaag                                                  20


<210>  97
<211>  20

<212>  DNA
<213>  Artificial Sequence

<220>
<223>  oligonucleotide

<400>  97
atccaatgtg tcagttccca                                                   20


<210>  98
<211>  20
<212>  DNA
<213>  Artificial Sequence

<220>
<223>  oligonucleotide

<400>  98
gggcaaagag ggaaatgaga                                                   20


<210>  99
<211>  20
<212>  DNA
<213>  Artificial Sequence

<220>
<223>  oligonucleotide

<400>  99
caatggataa ggccgagacc                                                   20


<210>  100
<211>  23
<212>  DNA
<213>  Artificial Sequence

<220>
<223>  oligonucleotide

<400>  100
accggtagca ccgcccagac gac                                               23


<210>  101
<211>  336
<212>  DNA
<213>  Homo sapiens

<400>  101
ggtcctcaga agtggaatac agagcggagg tcggtcagaa tgcctatctg ccctgcttct      60

acaccccagc cgccccaggg aacctcgtgc ccgtctgctg gggcaaagga gcctgtcctg     120

tgtttgaatg tggcaacgtg gtgctcagga ctgatgaaag ggatgtgaat tattggacat     180

ccagatactg gctaaatggg gatttccgca aaggagatgt gtccctgacc atagagaatg     240

tgactctagc agacagtggg atctactgct gccggatcca aatcccaggc ataatgaatg     300

atgaaaaatt taacctgaag ttggtcatca aaccag                               336

<210>  102
<211>  110
<212>  DNA
<213>  Homo sapiens

<400>  102
ccaaggtcac ccctgcaccg actcggcaga gagacttcac tgcagccttt ccaaggatgc      60

ttaccaccag gggacatggc ccaggtaact atgcatgggg cagaggaggg                110


<210>  103
<211>  305
<212>  DNA
<213>  Homo sapiens

<400>  103
tggcccgccc gctgccgccc ccggccatcc cctggccccc ggccctcacc cggcggcgcc      60

ctcctcctgg gggcccaggc cccgccgcta cacggtgctg agcgtgggtc ccggaggcct     120

gcgcagcggg aggctgcccc tgcagccccg cgtccagctg gatgagcgcg gccggcagcg     180

cggggacttc tcgctatggc tgcgcccagc ccggcgcgcg gacgccggcg agtaccgcgc     240

cgcggtgcac ctcagggacc gcgccctctc ctgccgcctc cgtctgcgcc tgggccaggc     300

ctcga                                                                 305


<210>  104
<211>  407
<212>  DNA
<213>  Homo sapiens

<400>  104
acaggggtga aggcccagag accagcagaa cggcatccca gccacgacgg ccactttgct      60

ctgtctgctc tccgccacgg ccctgctctg ttccctggga cacccccgcc cccacctcct     120

caggctgcct gatctgccca gctttccagc tttcctctgg attccggcct ctggtcatcc     180

ctccccaccc tctctccaag gccctctcct ggtctccctt cttctagaac cccttcctcc     240

acctccctct ctgcagaact tctcctttac cccccacccc ccaccactgc cccctttcct     300

tttctgacct ccttttggag ggctcagcgc tgcccagacc ataggagaga tgtgggaggc     360

tcagttcctg ggcttgctgt ttctgcagcc gctttgggtg gctccag                   407


<210>  105
<211>  318
<212>  DNA
<213>  Homo sapiens

<400>  105
gatgccaggg atcagctgac catgtggtta gcatctcggg agtgcctctt cagttacaac      60

caaacagcat acagacgaag gttgacagca ttgcatggaa gaagttgctg ccctcacaaa     120

atggatttca tcacatattg aagtgggaga atggctcttt gccttccaat acttccaatg 180

atagattcag ttttatagtc aagaacttga gtcttctcat caaggcagct cagcagcagg 240

acagtggcct ctactgcctg gaggtcacca gtatatctgg aaaagttcag acagccacgt 300

tccaggtttt tgtatttg 318

<210> 106
<211> 276
<212> DNA
<213> Homo sapiens

<400> 106
ataaagttga gaaaccccgc ctacaggggc aggggaagat cctggacaga gggagatgcc 60

aagtggctct gtcttgcttg gtctccaggg atggcaatgt gtcctatgct tggtacagag 120

ggagcaagct gatccagaca gcagggaacc tcacctacct ggacgaggag gttgacatta 180

atggcactca cacatatacc tgcaatgtca gcaatcctgt tagctgggaa agccacaccc 240

tgaatctcac tcaggactgt cagaatgccc atcagg 276

<210> 107
<211> 144
<212> DNA
<213> Homo sapiens

<400> 107
agtttccctt ccgctcacct ccgcctgagc agtggagaag gcggcactct ggtggggctg 60

ctccaggcat gcagatccca caggcgccct ggccagtcgt ctgggcggtg ctacaactgg 120

gctggcggcc aggatggttc ttag 144

<210> 108
<211> 360
<212> DNA
<213> Homo sapiens

<400> 108
actccccaga caggccctgg aaccccccca ccttctcccc agccctgctc gtggtgaccg 60

aaggggacaa cgccaccttc acctgcagct tctccaacac atcggagagc ttcgtgctaa 120

actggtaccg catgagcccc agcaaccaga cggacaagct ggccgccttc cccgaggacc 180

gcagccagcc cggccaggac tgccgcttcc gtgtcacaca actgcccaac gggcgtgact 240

tccacatgag cgtggtcagg gcccggcgca atgacagcgg cacctacctc tgtggggcca 300

tctccctggc ccccaaggcg cagatcaaag agagcctgcg ggcagagctc agggtgacag 360

<210> 109
<211> 128
<212> DNA
<213> Homo sapiens

<400>  109
agtctaacgt gaagacattt tgctccaaga atatcctagc catccttggc ttctcctcta      60

tcatagctgt gatagctttg cttgctgtgg ggttgaccca gaacaaagca ttgccagaaa     120

acgttaag                                                              128


<210>  110
<211>  273
<212>  DNA
<213>  Homo sapiens

<400>  110
atatccagaa ccctgaccct gccgtgtacc agctgagaga ctctaaatcc agtgacaagt      60

ctgtctgcct attcaccgat tttgattctc aaacaaatgt gtcacaaagt aaggattctg     120

atgtgtatat cacagacaaa actgtgctag acatgaggtc tatggacttc aagagcaaca     180

gtgctgtggc ctggagcaac aaatctgact ttgcatgtgc aaacgccttc aacaacagca     240

ttattccaga agacaccttc ttccccagcc cag                                  273


<210>  111
<211>  333
<212>  DNA
<213>  Homo sapiens

<400>  111
gtgttcccac ccgaggtcgc tgtgtttgag ccatcagaag cagagatctc ccacacccaa      60

aaggccacac tggtgtgcct ggccacaggc ttcttccctg accacgtgga gctgagctgg     120

tgggtgaatg ggaaggaggt gcacagtggg gtcagcacgg acccgcagcc cctcaaggag     180

cagcccgccc tcaatgactc cagatactgc ctgagcagcc gcctgagggt ctcggccacc     240

ttctggcaga acccccgcaa ccacttccgc tgtcaagtcc agttctacgg gctctcggag     300

aatgacgagt ggacccagga tagggccaaa ccc                                  333


<210>  112
<211>  36
<212>  RNA
<213>  Artificial Sequence

<220>
<223>  guide sequence

<400>  112
ucuccgagag cccguagaac guuuuagagc uaugcu                                36


<210>  113
<211>  36
<212>  RNA
<213>  Artificial Sequence

<220>
<223>  guide sequence

<400>  113
caaacacagc gaccucgggu guuuuagagc uaugcu                                36


<210>  114
<211>  36
<212>  RNA
<213>  Artificial Sequence

<220>
<223>  guide sequence

<400>  114
ugacagcgga agugguugcg guuuuagagc uaugcu                                36


<210>  115
<211>  36
<212>  RNA
<213>  Artificial Sequence

<220>
<223>  guide sequence

<400>  115
gagaaucaaa aucggugaau guuuuagagc uaugcu                                36


<210>  116
<211>  36
<212>  RNA
<213>  Artificial Sequence

<220>
<223>  guide sequence

<400>  116
aguugagaaa ccccgccuac guuuuagagc uaugcu                                36


<210>  117
<211>  36
<212>  RNA
<213>  Artificial Sequence

<220>
<223>  guide sequence

<400>  117
caccgcggcg cgguacucgc guuuuagagc uaugcu                                36


<210>  118
<211>  36
<212>  RNA
<213>  Artificial Sequence

<220>
<223>  guide sequence

<400>  118
gcucagcacc guguagcggc guuuuagagc uaugcu                                36

<210>  119
<211>  36
<212>  RNA
<213>  Artificial Sequence

<220>
<223>  guide sequence

<400>  119
cuaaaugggg auuuccgcaa guuuuagagc uaugcu                                36


<210>  120
<211>  36
<212>  RNA
<213>  Artificial Sequence

<220>
<223>  guide sequence

<400>  120
auccccauuu agccaguauc guuuuagagc uaugcu                                36


<210>  121
<211>  36
<212>  RNA
<213>  Artificial Sequence

<220>
<223>  guide sequence

<400>  121
gagucacauu cucuaugguc guuuuagagc uaugcu                                36

## Description

### Field of the invention

The present invention is related to guide RNAs (gRNAs) directed to at least one inhibitory receptor (IR) gene and/or to α and/or β TCR chain (TRAC and TRBC) gene, to Clustered Regularly Interspaced Short Palindromic Repeats (CRISPR)-CRISPR-associated protein 9 (Cas9) (CRISPR/Cas9) system comprising said guide RNAs, to T cells or TCR redirected T cells or CAR T cells and to their use in the treatment of cancer or tumor, inflammatory and/or autoimmune diseases.

### Background of the invention

Cancer immunotherapy represents one of the most recent advances in biomedical research, and largely relies on the ability of T lymphocytes to recognize and kill their targets and generate antigen-specific memory T cells. Adoptive T cell therapy (ACT) with lymphocytes genetically engineered to express a tumor-specific T-cell Receptor (TCR) or a chimeric antigen receptor (CAR) has shown promising results in clinical trials. However, short T cell persistence and T cell exhaustion limit the efficacy of ACT. The use of early differentiated T cells, such as memory stem T cells (T_{SCM}) and central memory (T_{CM}) lymphocytes instead of effector memory (T_{EM}) or terminal effector cells (T_{EMRA}), may address the first issue. T_{SCM} proved to be endowed by self-renewal potential, high expansion and long-term persistence (Gattinoni Nat Med 2011; Oliveira at el., STM 2015; Gattinoni Review in Nat Med 2017). The present inventors developed a clinically compliant protocol to generate genetically engineered T_{SCM} cellular products endowed with multipotency and differentiation, expansion and persistence capacities.

Once tumor-specific T lymphocytes infiltrate the tumor, chronic antigen stimulation drives T cell exhaustion, a dysfunctional state characterized by a loss of effector functions. Hallmark of exhaustion is the upregulation of a panel of inhibitory receptors (IRs) including PD-1, CTLA-4, LAG-3, Tim-3 and 2B4. Inhibitory receptors (IRs) are a variety of molecules expressed by T cells, involved in the regulation of motility, cytokine production and effector functions, thus orchestrating peripheral self-tolerance maintenance and acting as breaks for T cell responses. Their ligands are widely upregulated on a broad spectrum of tumor cells and other cells of the tumor microenvironment, thus the interaction between ligands and inhibitory receptors dampens anti-tumor immunity and results in T cell exhaustion and tumor escape (Wherry & Kurachi, 2015). The co-expression of several IRs such as PD-1, LAG-3 or Tim-3, in the absence of activation markers, on antigen-specific T cells represents a hallmark of T cell exhaustion. Physiologically, co-stimulatory and co-inhibitory molecules co-evolved to regulate T cell activation upon TCR triggering. Pivotal initial studies in mice demonstrated the importance of IRs in modulating immune responses. CTLA-4 knock-out mice develop lymphoproliferative disorders, that cause extensive tissue damage and death in the first month of age. PD-1 deficient mice develop autoimmune diseases. These evidences showed that CTLA-4 plays a role in T cell priming, while PD-1 regulation is important in effector T cells in order to prevent excessive tissue damage. Phenotypic characterization of human T cells from healthy subjects point to a dynamic and variegate expression of the IR profile, that varies according to T cell subset, differentiation and activation. For instance, 2B4, KLRG1 and CD160 are highly expressed in effector cells, while BTLA is mainly expressed by naive T cells. Other IRs, including CTLA-4, Tim-3 and LAG-3, are not expressed by steady state CD8 T cells. Importantly, IRs can be also expressed by early differentiated T_{SCM}, as shown in subjects vaccinated against yellow fever. On the other hand, non-exhausted, activated T cells also express several IRs, including PD-1, LAG-3, Tim-3 and CTLA-4, but in a strong association with activation markers, such as 4-1BB, CD69, HLA-DR and CD25. Thus, IR expression does not coincide with exhaustion and other molecules, such as differentiation and activation markers need to be studied on T cells, to fully capture their functional status. Three major mechanisms of action have been described for IRs: (i) competition with co-stimulatory receptors, ie: CTLA-4 competes with CD28 for the binding to CD80/CD86; (ii) interference with downstream pathways from TCR or co-stimulatory receptors; (iii) upregulation of genes involved in T cell dysfunction. However, a comprehensive characterization of IRs downstream signaling pathways remains to be fully revealed. CTLA-4 and PD-1 were the first, if not the best, IRs described and characterized. CTLA-4 was initially discovered by Linsley and Golstein in 1996, when they found that it binds to B7 with higher affinity than CD28. A major contribution was reported in 1995 by Krummel and Allison, who demonstrated the immunosuppressive role of CTLA-4 (Leach et al 1996). A seminal paper then demonstrated that the blockade of CTLA-4 in tumor bearing mice provides durable tumor regressions. In 2010, a fully human monoclonal antibody blocking CTLA-4 (ipilimumab) entered clinical investigation and improved overall survival of patients with metastatic melanoma. Although only 15% of patients displayed objective responses, those responses were durable. From that moment, a new class of drugs, called "immune checkpoint blockades (ICBs)" entered clinical practice. In parallel, physicians had to phase a new class of side effect, named immune related adverse events, characterized by tissue-specific autoimmune reactions. Indeed, ICBs do not act only on tumor-specific T cells, but instead on the entire T-cell repertoire, posing the risk of autoimmunity. Clinical data demonstrated that dual blockade of anti-CTLA4 and anti-PD-1 produce a synergistic effect (Larkin et al, 2015; Wolchok et al, 2013). In preclinical models, combinatorial blockade of IRs demonstrated increased reinvigoration of exhausted cells. The simultaneous blockade of PD-1 with LAG-3 or CTLA-4 or Tim-3 increases the recovery of effector functions (Kaufmann et al, 2007; Blackburn et al, 2009; Nakamoto et al, 2009; Jin et al, 2010; Kassu et al, 2010) and in some combinations produced synergistic effects in antigen-specific T cells in models of chronic infections and cancer (Sakuishi et al, 2010; Fourcade et al, 2010). Currently, an anti-LAG-3 monoclonal antibody is under investigation in clinical trials. Several preclinical and clinical data postulated that the upregulation of alternative immune checkpoints might be a possible resistance mechanism upon anti-PD-1 therapy. Upregulation of TIGIT on NY-ESO-1-specific CD8+T cells has been reported upon PD-1 blockade in vitro and the upregulation of multiple alternative immune checkpoints including Tim-3, Lag-3, and CTLA-4 upon anti-PD-1 treatment was described in a murine lung cancer model. The use of monoclonal antibody blocking the interactions between inhibitory receptors and their ligands can rescue T cell exhaustion and effector functions. Up to now, anti-PD-1 and anti-CTLA-4 monoclonal antibodies has shown impressive results in clinical trials for the treatment of both solid and hematological malignancies and received approval for market authorization. However, the most common side effects using checkpoint blockade are autoimmune-related side effects, mainly caused by the effect of such molecules on the entire T cell repertoire rather than on tumor-specific T cells. Actually, seven monoclonal antibody blocking LAG-3 are under development: among these, Relatilimab is currently under clinical investigation in 18 phase I/II trials. Combination of Nivolumab and Relatilimab showed a 20% of response rate in melanoma patients with a frequency of LAG-3+ TILs greater than 1% (NCT01968109). Preliminary results are also shown for clinical trials with MK-4280 in patients with advanced solid tumors (NCT02720068). Combination of MK-4280 with Pembrolizumab showed 27% of Objective Response Rate in 15 patients. In all trials, anti-LAG-3 mAb proved safe and well tolerated. Currently, 11 clinical trials testing Tim-3 blockade alone or in combination with PD-1 blockade are ongoing with encouraging first results.

### TCR gene transfer and TCR gene editing

Beside CARs, the transfer of tumor-specific TCRs into mature T lymphocytes represents a promising approach to redirect T-cell specificity toward cancer cells. Compared to CARs, T cell Receptors TCRs have the great advantage of recognizing also intracellular antigens, in the form of epitopes presented on HLA molecules, this feature allowing to broaden the range of antigens that can be targeted by ACT. The TCR gene transfer approach was reported in the nineties by Clay et al., who efficiently transduced human T cells with a melanoma-specific TCR by means of a retroviral (RV) vector. This approach starts with the isolation of a T cell clone with high affinity for a specific target antigen: its α and β chains are isolated, cloned into viral vectors and expressed in patients T cells. The first clinical study demonstrating the efficacy of TCR transferred lymphocytes in mediating tumor regression was published in 2006, when T cells expressing an anti-MART-1 (Melanoma-Melanocyte antigen or Melan-A) TCR were infused to cancer patients. Other intracellular proteins, such as Wilms tumor antigen-1 (WT1), the cancer-testis antigens NY-ESO-1 and minor histocompatibility antigens have been validated as potential target antigens for TCR transferred lymphocytes, both in solid tumors and in hematological malignancies. TCR gene transfer has to deal with some limitations intrinsic to T cell biology. The transfer of high-avidity tumor-specific T cell receptor (TCR) occurs in mature lymphocytes, each expressing a distinct endogenous TCR. Consequently, the transferred tumor-specific TCR chains might mispair with the endogenous ones and compete for components of the CD3 complex. This results in tumor-specific TCR dilution, reduced anti-tumor activity and novel, potentially harmful specificities. To completely and permanently abrogate the expression of the endogenous TCR and the risk of mispairing, a novel approach, termed TCR gene editing, was developed by the present inventors. The protocol is based on the combination of endogenous α and/or β TCR chain (TRAC and TRBC) gene disruption coupled with transfer of tumor specific TCR genes. TCR edited T cells express uniquely the exogenous TCR at high levels and are then completely redirected against the antigen of choice. Notably, phenotype, function, and proliferative potential of T cells are not affected by gene editing. Overall, TCR edited cells displayed substantial anti-tumor efficacy and, being devoid of off-target reactivity, were superior to TCR-transferred lymphocytes in promoting GvHD-free survival in humanized murine models of cancer. Thus, TCR edited cells have the ability to recognize tumor antigens and are endowed with long-term persisting capacity, but, in principle, cannot resist exhaustion signals.

There remains a need in the art for cellular therapy product genetically engineered to recognize antigens, e.g. tumor antigens, endowed with long-term persisting capacity and with the ability to resist exhaustion mechanisms.

### Summary of the invention

Inventors hypothesized that the genetic disruptions of one or more of inhibitory molecules on tumor-specific T cells to be adoptively transferred to cancer patients, could enhance their antitumor activity, and allow to overcome T cell exhaustion. In order to develop an innovative T cell product to treat cancer, inventors investigated the effect of genomic disruption of a panel of inhibitory receptor (IRs) relevant in hematological and solid tumors. More specifically, the present inventors have previously demonstrated that Tim-3, LAG-3, PD-1, CTLA-4 and 2B4 were significantly overexpressed by T cell infiltrating the bone marrow of AML patients relapsing after allogeneic hematopoietic stem cell transplantation (Noviello et al, 2019; Toffalori et al, 2019).

The present inventors have herein genetically engineered T cells with CRISPR/Cas9 technology and lentiviral vectors to simultaneously redirect the T cell specificity against a tumor-antigen and to permanently disrupt inhibitory receptors. Inventors also dissected the role of distinct IR disruptions on T cell phenotype and effector functions upon antigen recognition and validated both *in vitro* and *in vivo* in multiple myeloma models the safety and the efficacy of TCR-edited IRs-disrupted T cells.

Thus, inventors herein tested the efficacy and the safety profile of an innovative tumor specific T cell product devoid of at least one inhibitory molecule overexpressed by exhausted T cells in the context of hematological malignancies.

Inventors demonstrated that:
- CRISPR/Cas9 reagents allowed simultaneous disruption of both TCR chains, with more than 90% disruption at both genomic loci, as assessed at molecular level.
- CRISPR/Cas9 technology allows for a high efficiency of Tim-3, LAG-3, 2B4 gene disruption (at least 80% of NHEJ at IRs loci) in T cells, including T_{SCM} /T_{CM} cells and more than 50% of the cells were efficiently redirected against the HLA-A*0201 restricted NY-ESO-1₁₅₇₋₁₆₅ epitope.
- CRISPR/Cas9 technology allows for a high efficiency of CD39 gene disruption (at least 75% of NHEJ at CD39 loci)
- Neither Tim3, Lag3 nor 2B4 are required for T cell expansion, activation, differentiation and acquisition of effector functions, since none of these functions were reduced in IR KO cells compared to IR competent counterparts.
- Tim-3, LAG-3 and 2B4 disruption does not affect the memory differentiation of edited cells.
- IRs-disrupted T cells retain their killing capacity, thus proving that Tim-3, LAG-3 or 2B4 are not required by T cells for effective effector functions.

Inventors tested TCR-edited IR-disrupted cells both in vitro and in vivo against antigen positive tumor cells. They demonstrated that the disruption of either Tim-3 or 2B4, but not LAG3, in redirected cells increased the production of cytotoxic cytokines and cytolytic molecules. In preclinical models, TCR-edited LAG3-disrupted cells significantly controlled tumor burden and showed a higher T cell fitness compared to TCR-edited LAG3pos cells. In addition, mice treated with TCR-edited LAG3-disrupted, but not with TCR-edited-LAG3 competent cells, were resistant to secondary tumor challenges.

The disruption of multiple IRs (including Tim-3-LAG-3-2B4 triple disruption, Tim-3-LAG-3 or Tim-3-2B4 or LAG-3-2B4 double disruption) or single disruption can be beneficial for the anti-tumor activity of tumor specific T cells (either TCR redirected T cells or CAR T cells). Specifically, tumor specific T cells disrupted for either one or two or three IRs can enhance the production of cytotoxic molecules in the immunosuppressive tumor microenvironment and can induce long-lasting anti-tumor responses upon tumor recurrences. Thus, triple, double or single IR disruption can be beneficial for cellular products (either TCR redirected or CAR T cells) to be used for the treatment of both hematological and solid tumors.

In addition, IR disruption can be effective alone if applied to antigen specific T cells isolated from tumor samples or peripheral blood or lymph nodes or other biological samples. IR disruption can be efficiently combined to a TRAC or TRBC disruption in CAR T cells and TCR redirected T cells.

It is therefore an object of the invention an isolated guide ribonucleic acid (gRNA) comprising a guide sequence targeting an inhibitory receptor (IR), a TCR α (TRAC) constant region or a β chain (TRBC 1/2) constant region target sequence, wherein said guide sequence is selected from the group consisting of SEQ ID NOs:1-27 or wherein said guide sequence comprises a sequence selected from the group consisting of SEQ ID NOs:1-27.

A further object of the invention is an isolated guide ribonucleic acid (gRNA) comprising a guide sequence selected from the group consisting of:
- at least 17, 18, 19, 20 contiguous nucleotides of a sequence selected from SEQ ID NOs:1-27, or
- a sequence that is at least 90% identical to a sequence selected from SEQ ID NOs: 1-27.

Another object of the invention is a composition comprising at least one gRNA as defined above or a nucleic acid molecule encoding said at least one gRNA.

Preferably, the composition comprises:
- at least one gRNA comprising a guide sequence selected from SEQ ID NOs: 1-10, and
- at least one further gRNA comprising a guide sequence selected from SEQ ID NOs:11-23 and optionally
- at least one further gRNA comprising a guide sequence of SEQ ID NO:24 and/or at least one further gRNA comprising a guide sequence selected from SEQ ID NOs:25-27.

Preferably, the composition comprises:
- at least one gRNA comprising a guide sequence of SEQ ID NOs:1, and
- at least one further gRNA comprising a guide sequence of SEQ ID NO:24.

Preferably, the composition of the invention comprises:
- at least one gRNA comprising a guide sequence selected from SEQ ID NOs: 1-23, and
- at least one further gRNA comprising a guide sequence of SEQ ID NO:24 and/or at least one further gRNA comprising a guide sequence selected from SEQ ID NOs:25-27.

Preferably, the composition according to the invention comprises:
(ii) a gRNA comprising a guide sequence of SEQ ID NO:24 and/or a gRNA comprising a guide sequence comprising a guide sequence selected from SEQ ID NOs:25-27 and
(ii) a gRNA comprising a guide sequence of SEQ ID NO: 1 or
   a gRNA comprising a guide sequence of SEQ ID NO:3 or
   a gRNA comprising a guide sequence of SEQ ID NO:11 or
   a gRNA comprising a guide sequence of SEQ ID NO: 15.

The composition of the invention may comprise the nucleic acid molecules encoding the above gRNAs and combinations thereof.

The composition of the invention preferably further comprises an RNA-guided DNA binding agent or a nucleic acid encoding an RNA-guided DNA binding agent, preferably wherein said RNA-guided DNA binding agent is Cas9.

A further object of the invention is a composition comprising a ribonucleoprotein (RNP) complex comprising the composition of the invention, preferably further comprising an enhancer.

Another object of the invention is a nucleic acid molecule encoding at least one gRNA as defined above or combinations thereof, preferably wherein said nucleic acid is operably linked to a promoter sequence that is recognized by a phage RNA polymerase for in vitro RNA synthesis, preferably wherein said nucleic acid is part of a vector.

Another object of the invention is a Clustered Regularly Interspaced Short Palindromic Repeats (CRISPR)-CRISPR-associated protein 9 (Cas9) system comprising:
a. a Cas9 protein or a nucleic acid molecule encoding a Cas9 protein, and
b. at least one gRNA or the composition or at least one nucleic acid molecule as above defined, preferably wherein the system and or a. or b. are comprised in a ribonucleoprotein (RNP) complex, preferably delivered by electroporation, and/or packaged into a viral vector.

Another object of the invention is a vector comprising or encoding at least one gRNA as defined or at least one gRNA or at least one nucleic acid molecule or comprising a CRISPR-Cas system as defined above, preferably said vector is or comprises a plasmid vector or a viral vector, such as a retroviral vector, gammaretroviral vector, herpesvirus vector, lentiviral vector, adenoviral vector or adeno-associated vector.

A further object of the invention is an engineered cell comprising at least one gRNA, or the composition or the nucleic acid molecule or the CRISPR/Cas system or the vector as defined above, preferably said cell being an immune cell, such as a T cell (e.g., a CD2+, a CD3+, a CD4+ or CD8+ T cell, CAR-T cell, NK T cell, iNKT, alpha beta T cell or gamma delta T cell, regulatory T cells), MAIT, more preferably being a memory stem T cells (T_{SCM}), a central memory (T_{CM}) lymphocytes, T naive, mixed T naive (TN) and stem memory T cells (TSCM), effector memory or effector T cells or regulatory T cell.

Preferably, the engineered cell further comprises a recombinant protein, or a polynucleotide encoding a recombinant protein,
or has been or will be engineered to express a recombinant protein,
preferably said recombinant protein being a recombinant receptor, more preferably a receptor expressed on the surface of the immune cell receptor
preferably said recombinant receptor specifically binds to an antigen,
and preferably wherein the immune cell is capable of inducing cytotoxicity, proliferating and/or secreting a cytokine upon binding of the recombinant receptor to the antigen,
preferably wherein the recombinant receptor is a recombinant T cell receptor (TCR) or a chimeric antigen receptor (CAR) or a T cell redirected for universal cytokine killing (TRUCK).

Another object of the invention is a pharmaceutical composition comprising at least one guide RNA or the composition or at least one nucleic acid molecule, or a CRISPR/Cas9 system, or at least one vector or at least one cell as defined above and at least one pharmaceutically acceptable carrier and/or excipient.

A further object of the invention is an in vitro or ex-vivo method of altering expression of at least one IR gene and optionally of TRAC and/or TRBC1/2 gene, in an isolated cell, said method comprising introducing into an isolated cell a guide RNA, or the composition or at least one nucleic acid molecule as defined above, in a CRISPR/Cas9 system, wherein the guide RNA targets the gene and the Cas protein cleaves the genomic loci of the DNA molecules encoding the one or more gene products, whereby expression of the one or more gene products is decreased or abolished, preferably said cell being an immune cell, such as a T cell (e.g., a CD2+, a CD3+, a CD4+ or CD8+ T cell, CAR-T cell, NK T cell, iNKT, alpha beta T cell or gamma delta T cell, regulatory T cells), MAIT, more preferably being a memory stem T cells (T_{SCM}), a central memory (T_{CM}) lymphocytes, T naive, mixed T naive (TN) and stem memory T cells (TSCM), effector memory or effector T cells or regulatory T cell.

Another object of the invention is the gRNA, or the composition or the nucleic acid molecule or the CRISPR/Cas system or the vector or the pharmaceutical composition or the cell as defined above for use as a medicament, preferably for use in gene therapy, preferably for use in the treatment and/or prevention of a condition selected from the group consisting of: cancer or tumor, an inflammatory disease, an autoinflammatory disease, an autoimmune disease, an allergic disease, an infectious disease, graft versus host disease, organ rejection and/or immune response induced by gene therapy.

The cancer or tumor is preferably selected from the group consisting of: a leukemia, lymphoma, chronic lymphocytic leukemia (CLL), lymphoma, chronic lymphocytic leukemia (CLL), B cell acute lymphocytic leukemia (B-ALL), acute lymphoblastic leukemia (ALL), acute myeloid leukemia, myelodysplasia, myeloproliferative neoplasm, Hodgkin's lymphoma, non-Hodgkin's lymphoma (NHL), diffuse large cell lymphoma (DLCL), indolent B cell lymphoma, refractory follicular lymphoma, mantle cell lymphoma, multiple myeloma, renal cell carcinoma (RCC), B cell malignancies, colon cancer, neuroblastoma, cervical carcinoma, colorectal cancer, breast cancer, bone cancer, ovarian cancer, skin cancer, melanoma, sarcoma, prostate cancer, lung cancer, liver cancer, esophageal cancer, hepatocellular carcinoma, pancreatic cancer, gastric cancer, astrocytoma, brain cancer, epithelial cancers, renal cell carcinoma (RCC), Ewing sarcoma, neuroblastoma, pancreatic adenocarcinoma, mesothelioma, glioblastoma, osteosarcoma, synovial sarcoma, head and neck cancer, medulloblastoma.

The autoimmune disease is preferably selected from the group consisting of: type 1 diabetes mellitus, autoimmune enteropathy, rheumatoid arthritis, systemic lupus erythematosus, multiple sclerosis, autoimmune myositis, psoriasis, Addison's disease, Grave's disease, Sjogren's syndrome, Hashimoto's thyroiditis, myasthenia gravis, vasculitis, pernicious anemia, celiac disease, autoimmune hepatitis, alopecia areata, pemphigus vulgaris, vitiligo, aplastic anemia, autoimmune uveitis, Alopecia Areata, Amyotrophic Lateral Sclerosis (Lou Gehrig's), Ankylosing Spondylitis, Anti-GBM Nephritis, Antiphospholipid Syndrome, Osteoarthritis, Autoimmune Active Chronic Hepatitis, Autoimmune Inner Ear Disease (AIED), Balo Disease, Behcet's Disease, Berger's Disease, Bullous Pemphigoid, Cardiomyopathy, Chronic Fatigue Immune Dysfunction Syndrome, Churg Strauss Syndrome, Cicatricial Pemphigoid, Cold Agglutinin Disease, Colitis Cranial Arteritis, Crest Syndrome, Crohn's Disease, Dego's Disease, Dermatomyositis & JDM, Devic Disease, Eczema, Essential Mixed Cryoglobulinemia, Eoscinophilic Fascitis, Fibromyalgia - Fibromyositis, Fibrosing Alveolitis, Giant Cell Arteritis, Glomerulonephritis, Goodpasture's Disease, Guillain-Barre Syndrome, Hashimoto's Thyroiditis, Hepatitis, Hughes Syndrome, Idiopathic Pulmonary Fibrosis, Idiopathic Thrombocytopenic Purpura, Irritable Bowel Syndrome, Kawasaki's Disease, Lichen Planus, Lupoid Hepatitis, Lupus / SLE, Lyme Disease, Meniere's Disease, Mixed Connective Tissue Disease, Myositis: Juvenile Myositis (JM), Juvenile dermatomyositis (JDM), and Juvenile Polymyositis (JPM), Osteoporosis, Pars Planitis, Pemphigus Vulgaris, Polyglandular Autoimmune Syndromes, Polymyalgia Rheumatica, Polymyositis, Primary Biliary Cirrhosis, Primary Sclerosis Cholangitis, Psoriasis, Raynaud's Syndrome, Reiter's Syndrome, Rheumatic Fever, Rheumatoid Arthritis, Scleritis, Scleroderma, Sticky Blood Syndrome, Still's Disease, Stiff Man Syndrome, Sydenham's Chorea, Takayasus Arteritis, Temporal Arteritis, Ulcerative Colitis, Uveitis, Vasculitis, Wegener's Granulomatosis and Wilson's Syndrome, preferably the autoimmune disease is vasculitis such as catastrophic anti-phospholipid syndrome (also named Asherson's syndrome), Giant Cell Arteritis and anti- ANCA vasculitis or myasthemia gravis, refractory celiac disease, autoimmune uveitis such as Behcet's Disease, pemphigus vulgaris, giant cell myocarditis, Graves' disease, Addison's disease and granulomatosis with polyangiitis.

The inflammatory or autoinflammatory disease is preferably a chronic inflammatory disease, preferably the chronic inflammatory disease is selected from the group consisting of: inflammatory bowel disease, Chron's disease, ulcerative colitis, celiac disease.

The engineered cell of the invention is preferably for use in cancer immunotherapy.

The present invention will be illustrated by means of non-limiting examples in reference to the following figures.

### Brief Description of the Figures

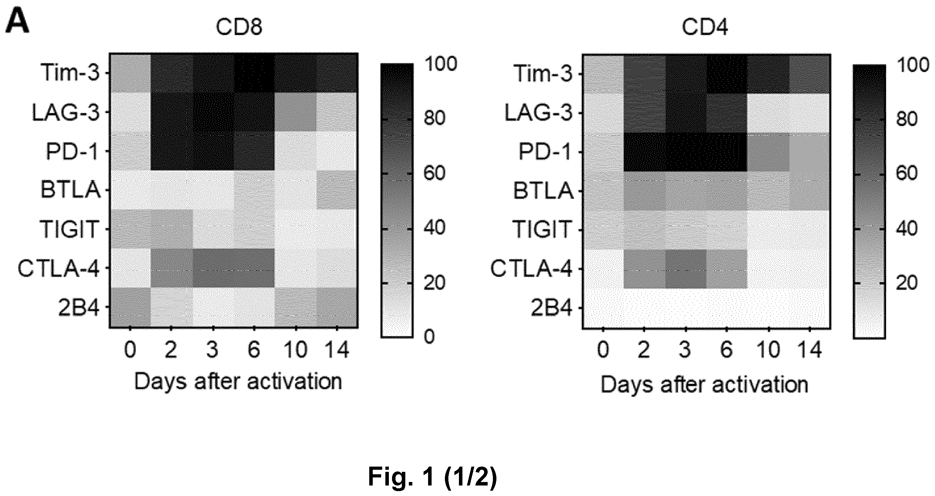

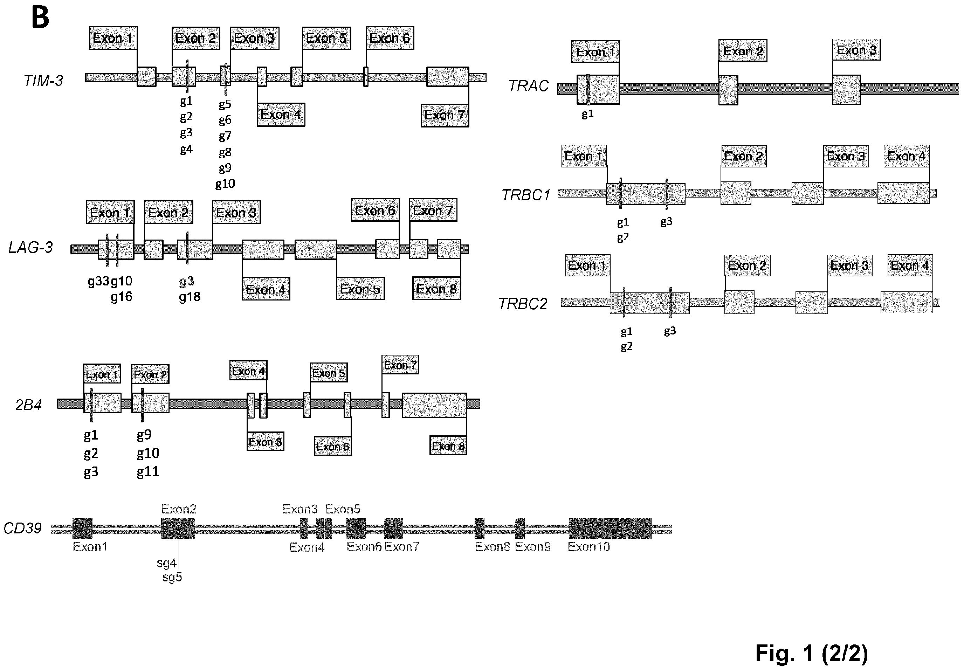

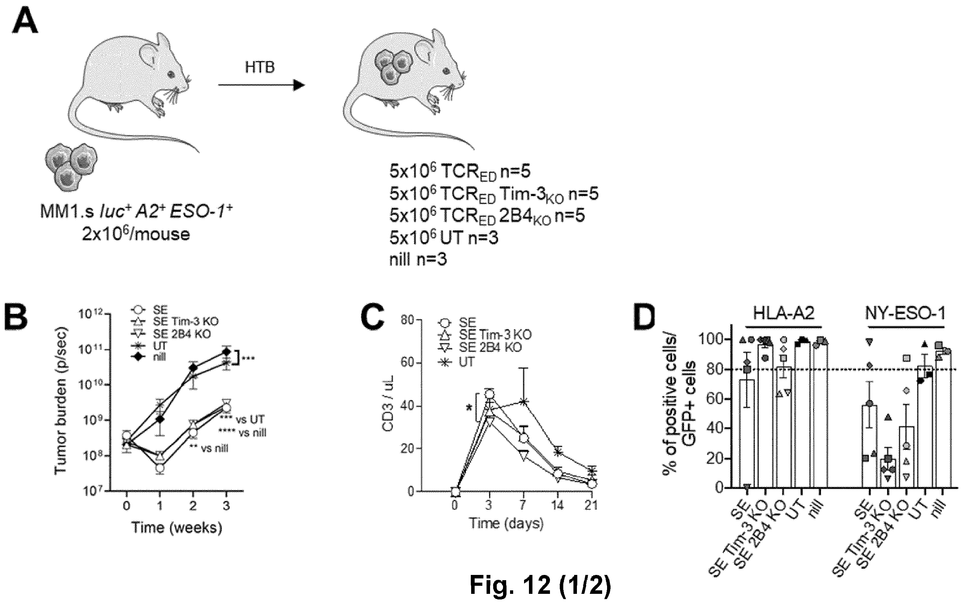

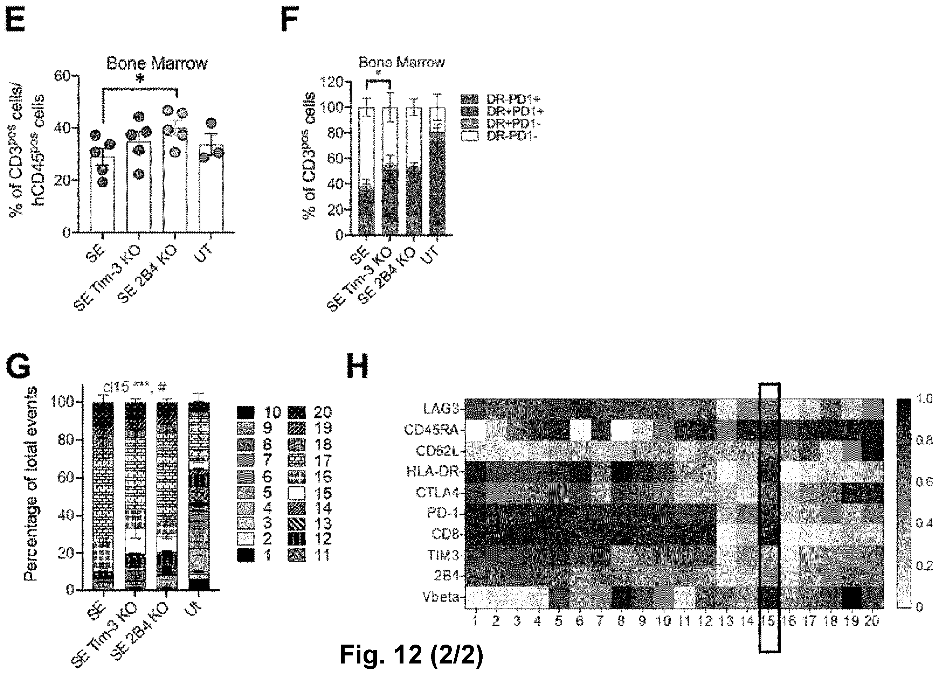

**Figure1****. Inhibitory receptor expression on resting and activated primary T cells and design of gRNAs targeting the selected IRs.** Percentage of Tim-3, LAG-3, PD-1, CTLA-4, BTLA, 2B4 or TIGIT positive cells gated on CD8 (left panel) or CD4 (right panel) T cells. Data are shown as mean of three healthy donors ± SEM **(A).**
   Cartoons depicting targeted loci of gRNAs at Tim-3, LAG-3, 2B4, PD-1, CD39, T cell receptor alpha chain and the shared common region of T cell receptor beta chain 1 and 2 coding regions **(B).** In TRBC1 and TRBC2 loci, the shared common region of TRBC1 and TRBC2 is shown in dark gray.

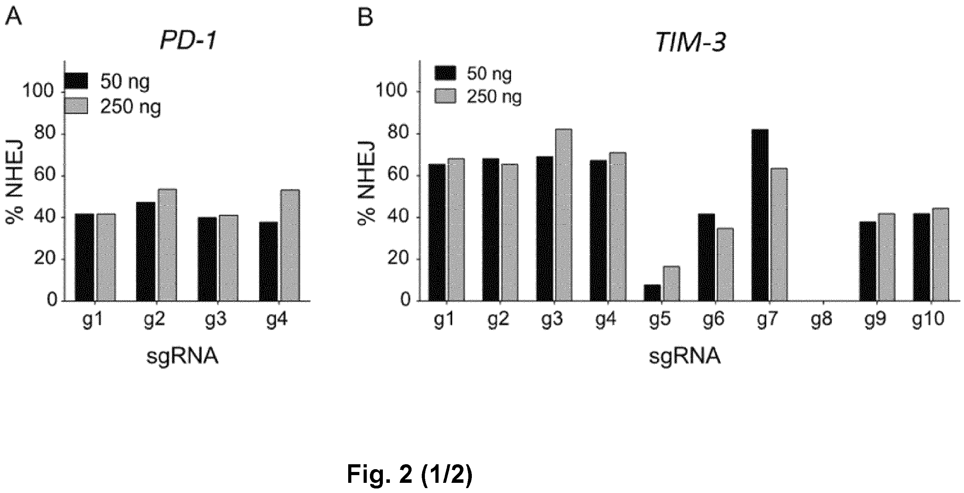

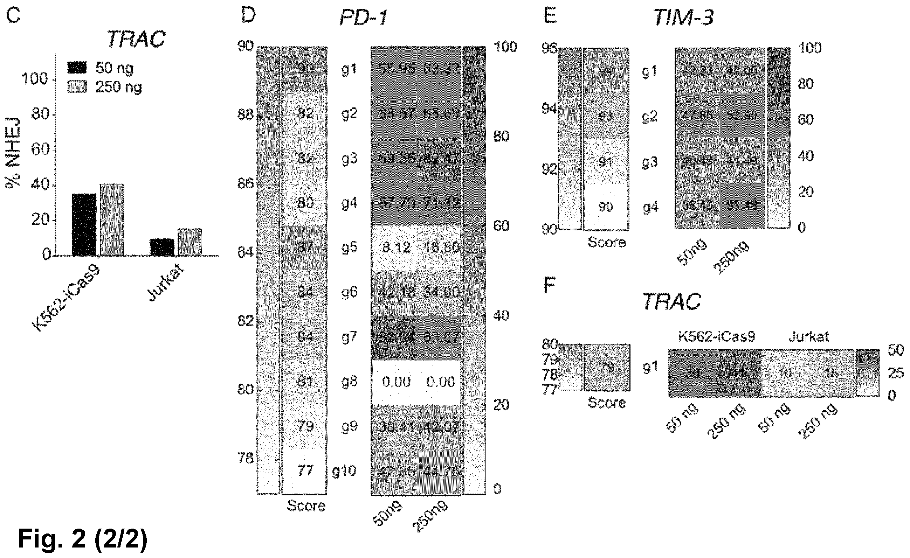

**Figure 2** **Screening of gRNAs in K562-iCas9 and Jurkat cell line.** Percentages of NHEJ at targeted genomic loci using 50ng (black bar) or 250ng (light grey bar) of gRNAs targeting PD-1 **(A),** Tim-3 **(B)** and TRAC **(C)** in K562-iCas9 or Jurkat cells (only for TRAC disruption). Heatmaps indicating the score predictive of targeting efficiency (from white to dark grey scale) and percentage of NHEJ of each tested gRNA obtained in the screening phase (from white to dark grey color scale) at Tim-3, PD-1 or TRAC locus **(D, E and F).**

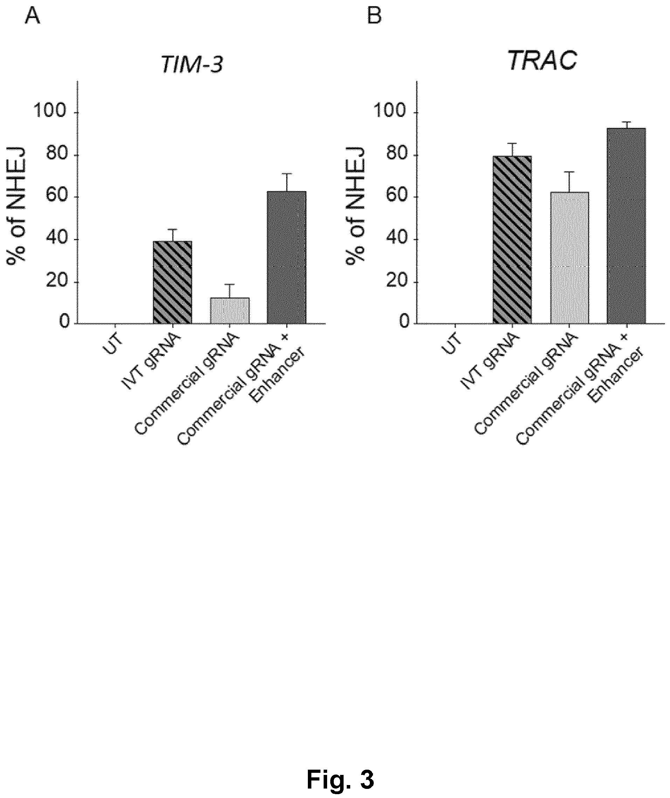

**Figure 3****. Gene disruption efficiencies of gRNAs:Cas9 ribonucleoproteins assembled with different reagents.** Percentages of NHEJ at Tim-3 **(A)** or TRAC loci **(B)** using in vitro transcribed gRNAs (grey bar with black diagonal line), commercial gRNA (light grey bar) or commercial gRNA in combination with the enhancer (dark grey bar). Untreated cell (UT), black bar) are shown as control. Data are shown as mean of at least 3 healthy donors ± S.E.M

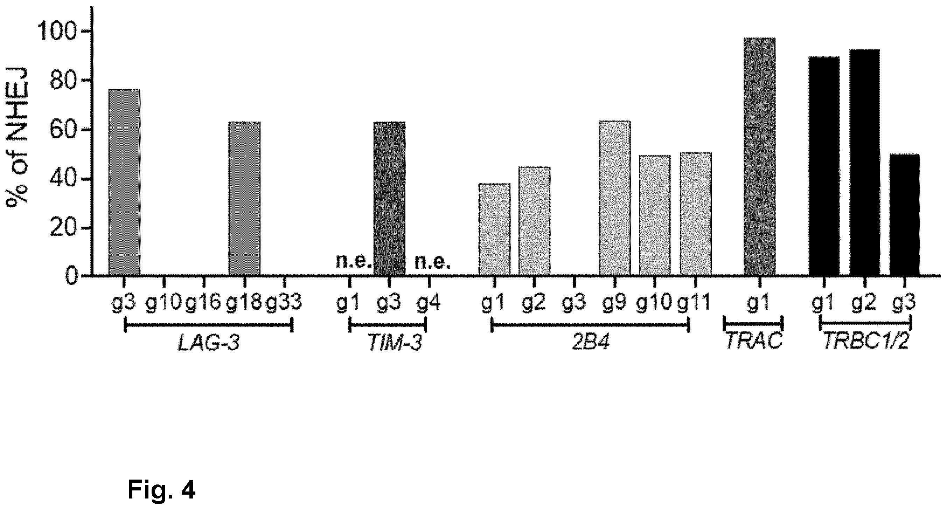

**Figure 4** **Screening of gRNAs targeting LAG-3, Tim-3, 2B4, TRAC and TRBC1/2 in primary T lymphocytes.** Percentages of NHEJ at targeted loci obtained with 5 for LAG-3, 6 gRNAs for 2B4, 1 gRNAs for Tim-3, 1 gRNAs for TRAC and 3 different gRNAs for TRBC1/2.

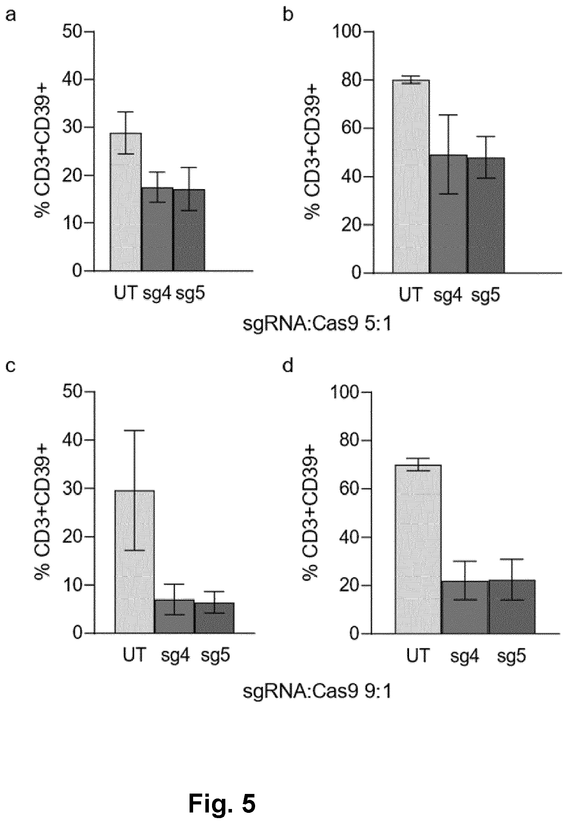

**Figure 5** **CD39 gene disruption in primary T cells: flow cytometry assessment.** Frequency of CD39⁺ T cells after CD39 disruption with a sgRNA:Cas9 5:1 ratio in control and edited cells stimulated with aCD3/CD28 beads **(A)** or after Rapid Expansion Protocol (REP) **(B);** T cells electroporated with RNPs in a sgRNA:Cas9 9:1 ratio in control and edited cells stimulated with aCD3/CD28 beads **(C)** or after Rapid Expansion Protocol (REP) **(D);** data are shown as mean ± SEM of 3 biological replicates.

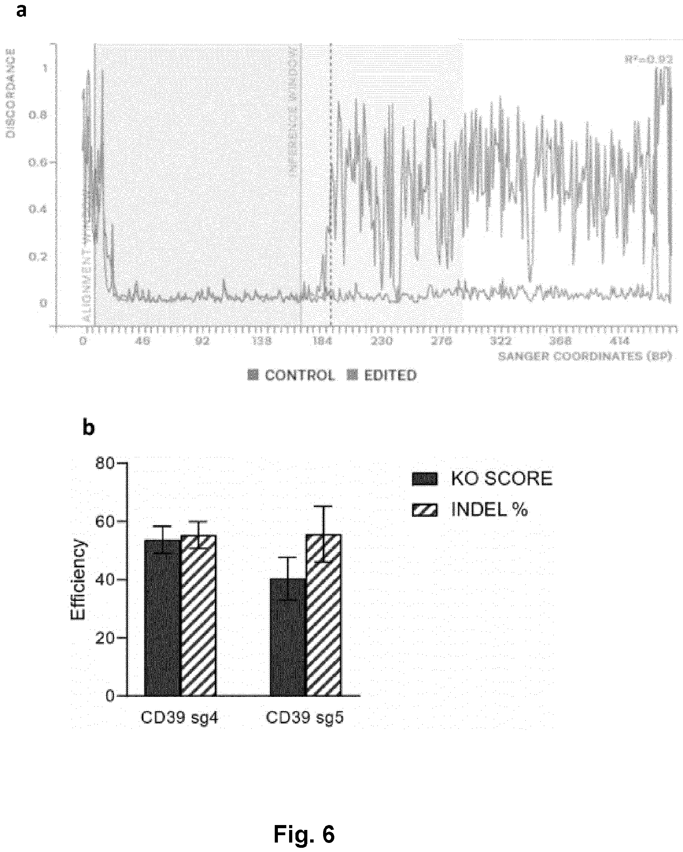

**Figure 6** **CD39 gene disruption: molecular analysis.** Traces of control and edited cells after Sanger Sequencing **(A);** knock-out and indels frequencies in T cells edited with sg4 and sg5 **(B);** data are shown as mean ± SEM of 3 biological replicates.
**Figure 1** **Single and complete TCR gene editing with CRISPR/Cas9 and lentiviral vector.** Percentages of cells expressing CD3 on cell surface in unmanipulated cells (UT, black bar) or cells transduced with TRAC-ZFN IDLVs (ZFN) or electroporated with CRISPR/Cas9 targeting trac locus **(A).** Percentages of Non-Homologous End-Joining (NHEJ) at targeted trac locus in unmanipulated cells (UT, black bar) or cells electroporated with CRISPR/Cas9 targeting trac **(B).** Percentages of cells expressing CD3 on cell surface in unmanipulated cells (UT), electroporated with CRISPR/Cas9 targeting only trac locus (gRNA TRAC) or targeting simultaneously trac and trbc1/2 loci (gRNA TRAC + gRNAs TRBC1/2) **(C).** Percentages of NHEJ at trac **(D)** or trbc1/2 loci **(E)** in unmanipulated cells (UT), T cells electroporated with CRISPR/Cas9 targeting trac locus (gRNA TRAC) or targeting simultaneously trac and trbc1/2 loci (gRNAs TRAC + gRNA TRBC1/2). Percentages of CD3 negative cells (white bars), CD3 positive TCR NY-ESO-1 negative (grey) and CD3 positive TCR NY-ESO-1 positive (dark grey) measured by flow cytometry in unmanipulated cells (UT), T cells transduced with an LV encoding for a HLA-A*02:01 restricted NY-ESO-1₁₅₇₋₁₆₅ specific TCR (transfer cells, TR), TRAC disrupted cells and transduced with an LV encoding for a HLA-A*02:01 restricted NY-ESO-1₁₅₇₋₁₆₅ specific TCR (Single edited cells, SE), TRAC and TRBC disrupted cells and transduced with an LV encoding for a HLA-A*02:01 restricted NY-ESO-1₁₅₇₋₁₆₅ specific TCR (CE, complete edited cells) (F). Representative plots of unmanipulated cell, single edited cells and complete edited cells (G). Data are shown as mean ± SEM of at least 2 biological replicates.

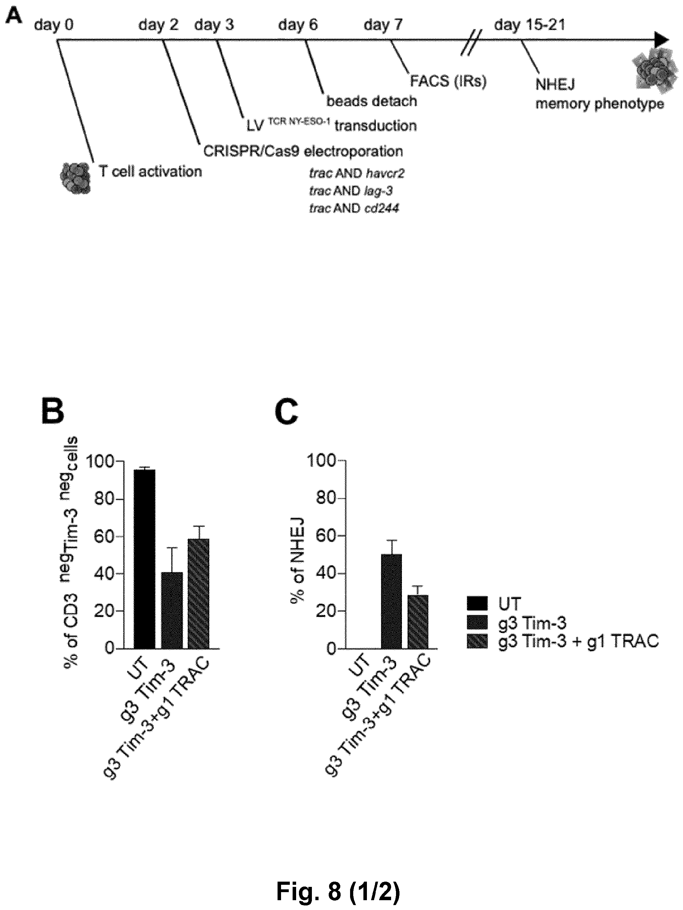

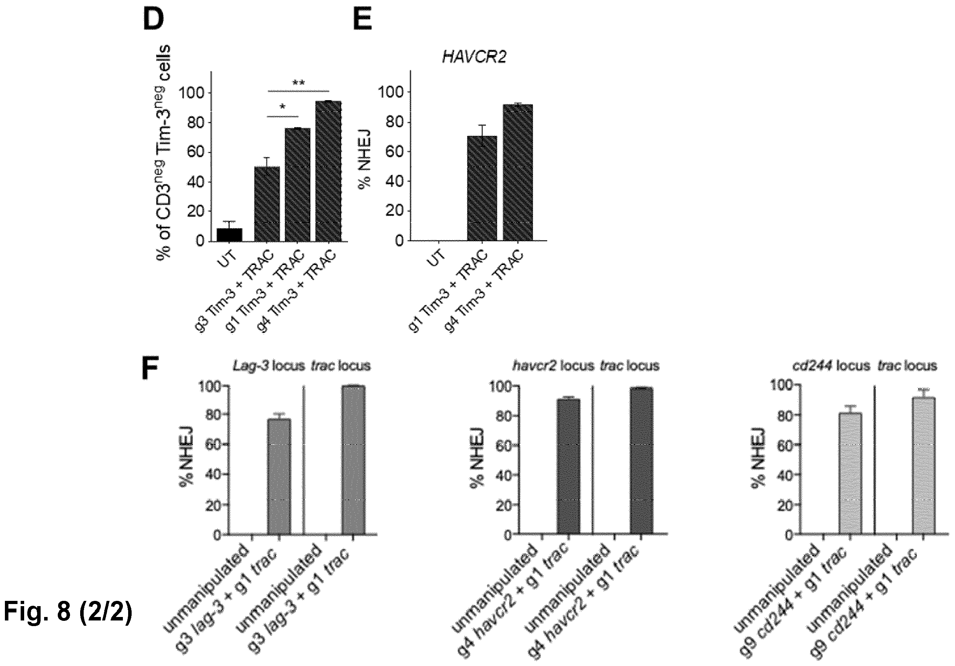

**Figure 8** **Multiplex IR and TCR gene editing with CRISPR/Cas9 and lentiviral (LV) vectors in human T cells.** Schematic representation of the protocol for the generation of Tim-3-, LAG-3, or 2B4-disrupted single edited (SE) T cells **(A).** Percentages of CD3^{neg}Tim-3^{neg} live cells measured by flow cytometry in T cells electroporated with CRISPR/Cas9 targeting Tim-3 with g3 alone or in combination with CRISPR/Cas9 targeting TRAC. Unmanipulated cells (UT) are shown as control **(B).** Percentages of NHEJ at Tim-3 locus in unmanipulated cells (UT), T cells electroporated with CRISPR/Cas9 targeting Tim-3 locus with g3 alone or in combination with CRISPR/Cas9 targeting trac locus **(C).** Percentages of CD3^{neg}Tim3^{neg} live cells in unmanipulated cells (UT) or T cells electroporated with CRISPR/Cas9 targeting Tim-3 with different gRNAs in combination with TRAC-targeting CRISPR/Cas9 **(D).** NHEJ at Tim-3 locus in unmanipulated cells (UT) or in T cells electroporated with CRISPR/Cas9 targeting Tim-3 with different gRNAs in combination with trac-targeting CRISPR/Cas9 **(E).** Quantification of Non-Homologous-End-Joining (NHEJ) at *trac, lag-3, havcr2 (tim-3)* and *2b4* loci in unmanipulated T cells or disrupted T cells **(F).** Data are shown as mean ± SEM of at least 2 biological replicates.

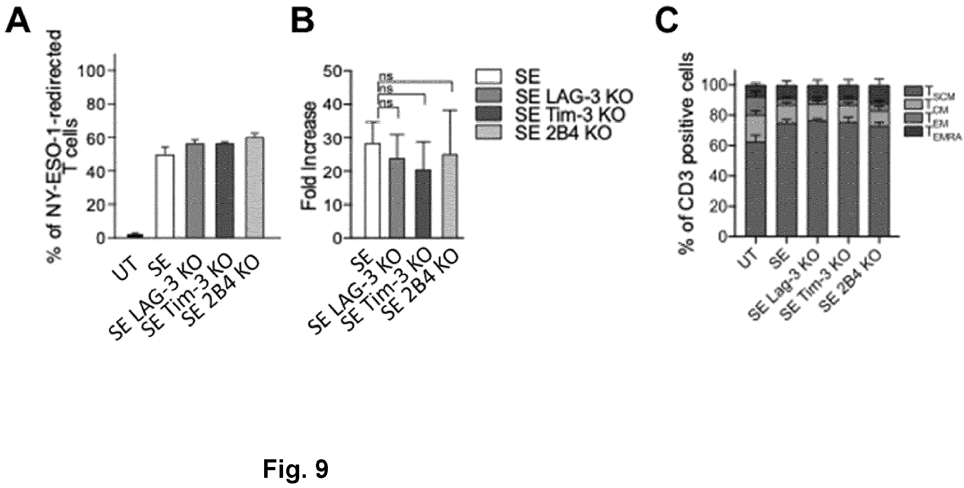

**Figure 9** **The disruption of Tim-3, LAG-3 or 2B4 in single edited (SE) T cells preserve their expansion capacity and early differentiated memory phenotype.** Frequency of cells redirected against NY-ESO-1 upon lentiviral transduction of unmanipulated cells (UT, black bar) or in T cells electroporated with CRISPR/Cas9 targeting Trac alone (SE) or in combination with gRNAs targeting lag3 (SE LAG-3 KO), tim3 (SE TIM-3 KO) or 2B4 (SE 2B4 KO) with different gRNAs in combination with trac-targeting CRISPR/Cas9. Transduction efficiency is measured by HLA-A*0201-NY-ESO-1₁₅₇₋₁₆₅ Dextramer staining **(A).** Fold Increase at day 21 of SE T cells, SE LAG-3 KO T cells, SE Tim-3 KO T cells and SE 2B4 KO T cells **(B).** Memory phenotype of unmanipulated (UT) T cells, SE, SE LAG-3 KO, SE Tim3-KO and SE 2B4 KO T cells at the end of cell culture **(C).** Data are shown as mean ± SEM of 3 biological replicates; ns= not statistically significant.

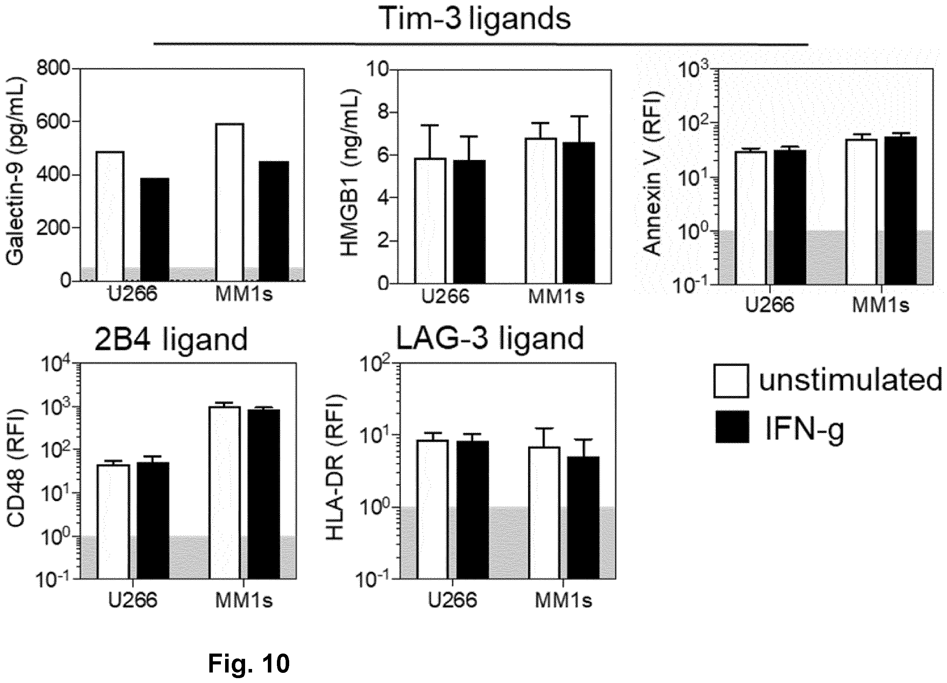

**Figure 10** **Inhibitory receptors ligands expressed by antigen positive and antigen negative multiple myeloma cell lines.**
   Concentration of Galectin-9 and HMGB1 secreted in cell culture media and surface expression of Annexin V (as surrogate marker for phosphatidyl serin), CD48 and HLA-DR in U266 and MM1s in resting conditions (white), or 48 hours (black) after IFNγ stimulation. Relative fluorescence intensity (RFI) = mean of fluorescence of stained sample / mean of fluorescence in unstained sample.

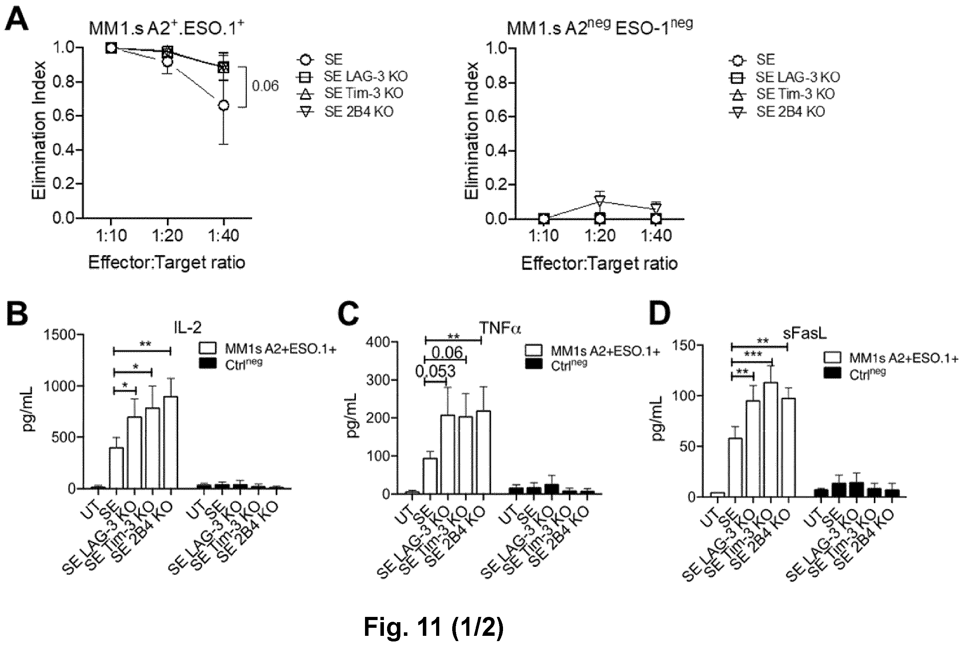

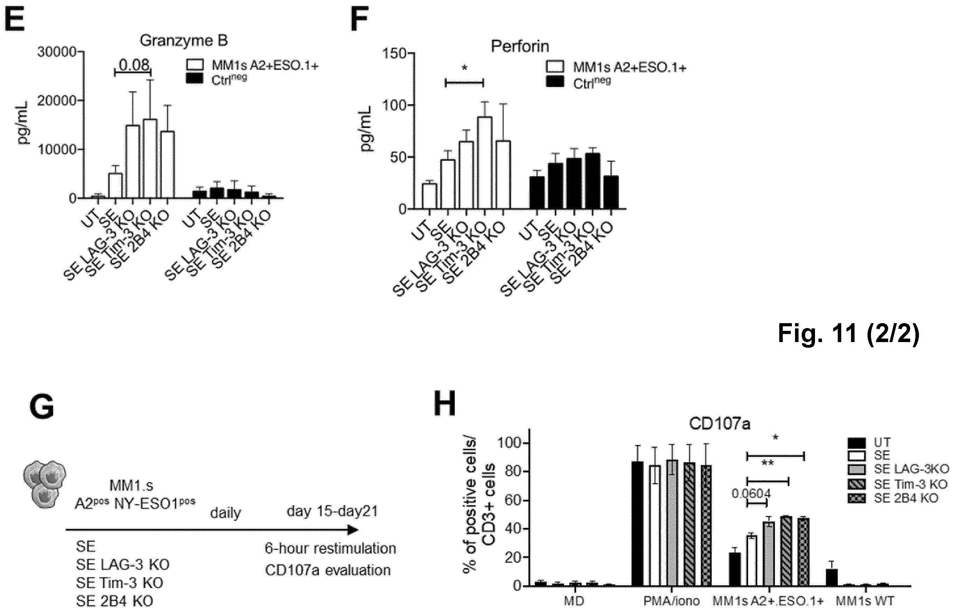

**Figure 11** **Tim-3- and 2B4-disrupted SE T cells display higher degranulation capacity than SE T cells upon chronic stimulation** Elimination index of SE, SE LAG-3 KO T cells, SE Tim-3 KO T cells and SE 2B4 KO T cells co-cultured with HLA-A2^{pos} NY-ESO^{pos} (left panel) or HLA-A2^{neg}NY-ESO-1^{neg} (right panel) MM1.S multiple myeloma cells at decreasing effector:target ratio **(A).** Quantification of IL-2 **(B),** TNFα **(C),** sFasL **(D),** Granzyme B **(E)** and perforin **(F)** produced by SE, SE LAG-3 KO T cells, SE Tim-3 KO T cells, SE 2B4 KO T cells or unmanipulated T cells (UT) upon exposure to HLA-A2^{pos} NY-ESO^{pos} MM1.S cells or to medium (Ctrl^{neg}). Schematic representation of the T cell exhaustion experiment **(G).** Percentage of degranulating CD3+ T cells (measured as CD107a+ cells) in SE (white), SE LAG-3 KO T cells (light gray), SE Tim-3 KO T cells (gray with diagonal bars), SE 2B4 KO T cells (gray with black squares) or unmanipulated T cells (black) challenged for 2 weeks with HLA-A2^{pos} NY-ESO^{pos} MM1.S tumor cells **(H).**
   Data are shown as mean ± SEM; *: p value <0.05; **: p-value < 0.01; ***: p-value < 0.001. Elimination index=1-[number of living target cells co-cultured with redirected T cell population/number of living target cells co-cultured with untransduced cells]
**Figure 12** **Tim-3- and 2B4-disrupted SE T cells control tumor growth and display a more activated and less exhausted phenotype than SE cells when challenged with aggressive multiple myeloma in vivo**
   Schematic representation of the experiment **(A).** Tumor burden (evaluated as total body bioluminescent signal) in mice treated with SE (circles), SE Tim-3-KO T cells (triangles), SE 2B4 KO T cells (upside-down triangles) or unmanipulated (UT, asterisk) T cells or left untreated (UT, black rhombus) **(B).** Absolute quantification of CD3+ T cells in the peripheral blood of mice treated with SE (circles), SE Tim-3-KO T cells (triangles), SE 2B4 KO T cells (upside-down triangles) or unmanipulated (UT, black rhombus) T cells **(C).** Percentage of HLA-A2^{pos} or NY-ESO-1^{pos} tumor cells (identified as GFP+ cells) in the bone marrow of mice treated with SE, SE Tim-3-KO T cells, SE 2B4 KO T cells or unmanipulated (UT) T cells or left untreated (nill). Each symbol represents individual mice **(D).** Frequency of CD3+ T cells infiltrating the bone marrow of mice treated with SE, SE Tim-3-KO T cells, SE 2B4 KO T cells or unmanipulated (UT) T cells **(E).** Frequency of activated (HLA-DR+ PD1+, HLA-DR+ PD-1-), exhausted (HLA-DR-PD-1+) or non-activated (HLA-DR- PD-1-) T cells in the bone marrow treated with SE, SE Tim-3-KO T cells, SE 2B4 KO T cells or unmanipulated (UT) T cells **(F).** Meta-cluster composition of SE, SE Tim-3-KO T cells, SE 2B4 KO T cells or unmanipulated (UT) T cells infiltrating the bone marrow of treated mice **(G).** Normalized expression of analysed markers in each meta-cluster **(H).** In panel H, Vbeta represents the specific Vb 13.1 of the NY-ESO-1 specific TCR. Data are shown as mean ± SEM; *: p value <0.05; **: p-value < 0.01; ***: p-value < 0.001. #: p-value < 0.05 (SE Tim-3 KO vs SE 2B4 KO)

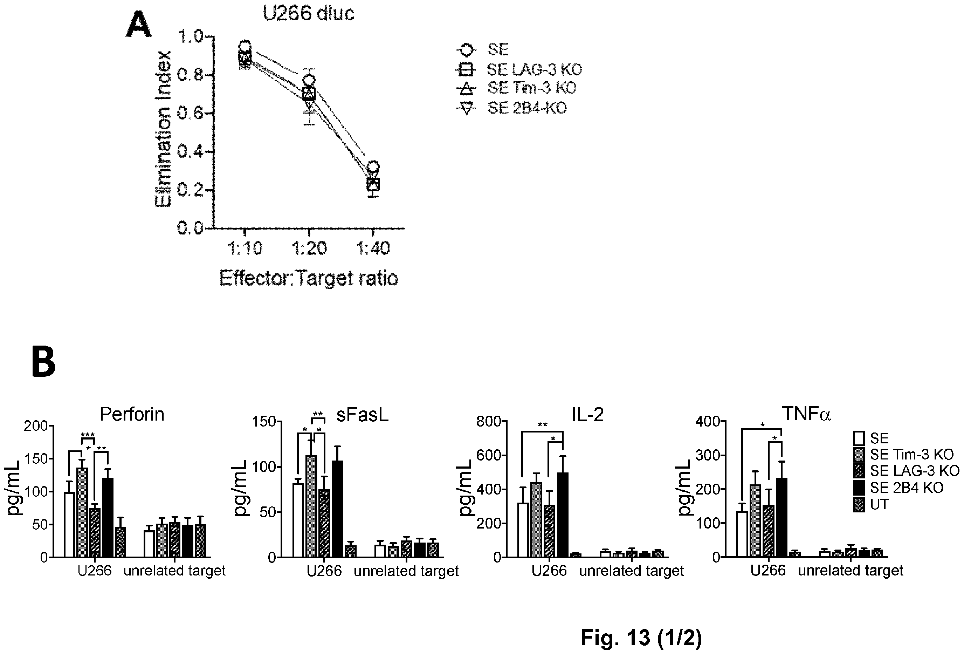

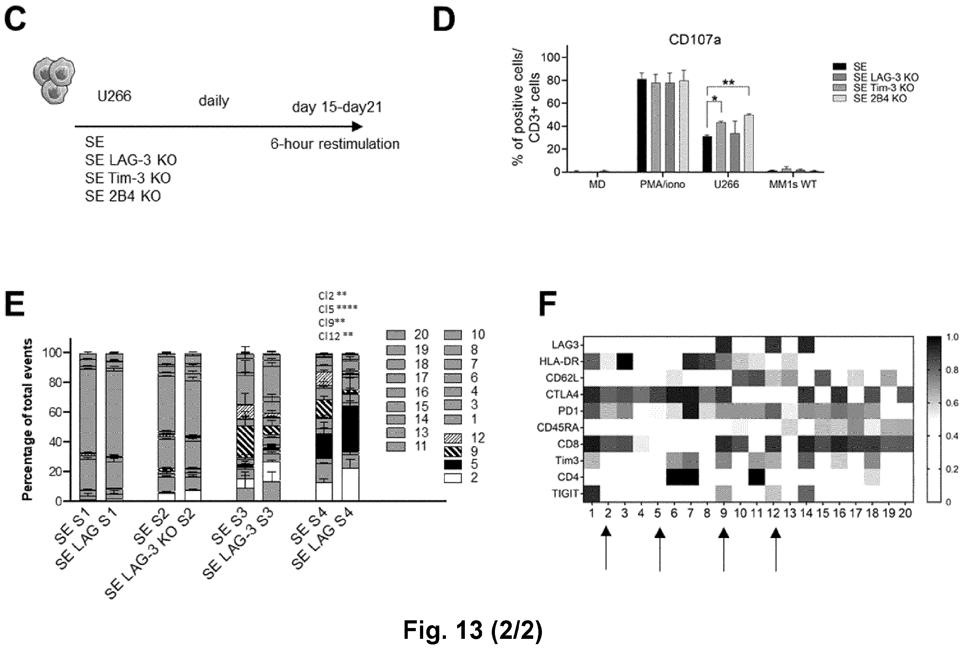

**Figure 13** **LAG-3 disruption in SE T cells prevent the upregulation of other inhibitory receptors (IRs) upon chronic stimulation**
   Elimination index of SE (circles), SE LAG-3 KO T cells (squares), SE Tim-3 KO T cells (triangles) and SE 2B4 KO T cells (upside-down triangles) co-cultured with HLA-A2^{pos} NY-ESO^{pos} U266 multiple myeloma cells at decreasing effector:target ratios **(A).** Quantification of Perforin, sFasL, IL-2 and TNFα produced by SE (white), SE LAG-3 KO T cells (gray with diagonal lines), SE Tim-3 KO T cells (gray), SE 2B4 KO T cells (black) or unmanipulated T cells (gray with black squares) challenged with HLA-A2^{pos} NY-ESO-1^{pos} U266 or an HLA-A2^{neg} NY-ESO-1^{neg} unrelated target **(B).** Timeline of the in vitro functional exhaustion experiment **(C).** Percentage of degranulating CD3+ T cells (measured as CD107a+ cells) in SE (black), SE LAG-3 KO T cells (dark gray), SE Tim-3 KO T cells (medium gray) or SE 2B4 KO T cells (light gray) upon chronic target recognition **(D).** Meta-cluster composition of SE or SE LAG-3 KO T cells after each round of stimulation with HLA-A2^{pos} NY-ESO-1^{pos} U266 multiple myeloma cells. Clusters significantly enriched in SE and SE LAG-3 KO cells are identified in white, black or, black diagonal lines, gray diagonal lines **(E).** Normalized expression of analyzed markers in each meta-cluster **(F).** Elimination index=1-[number of living target cells co-culture with redirected T cell population/number of living target cell co-cultured with untransduced cells]. Data are shown as mean ± SEM; *: p value <0.05; **: p-value < 0.01; ****: p-value < 0.0001.

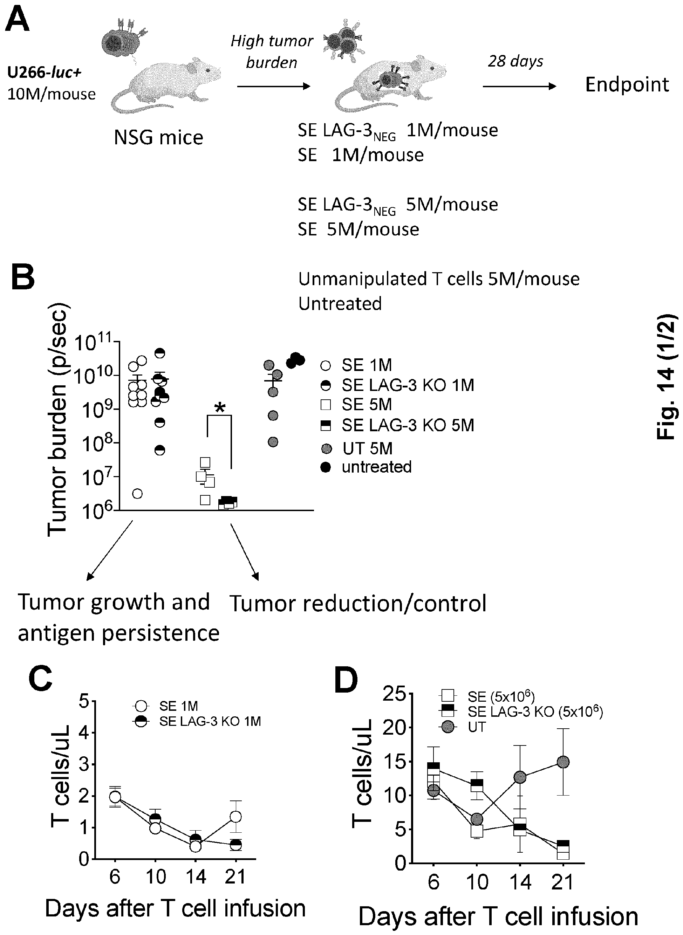

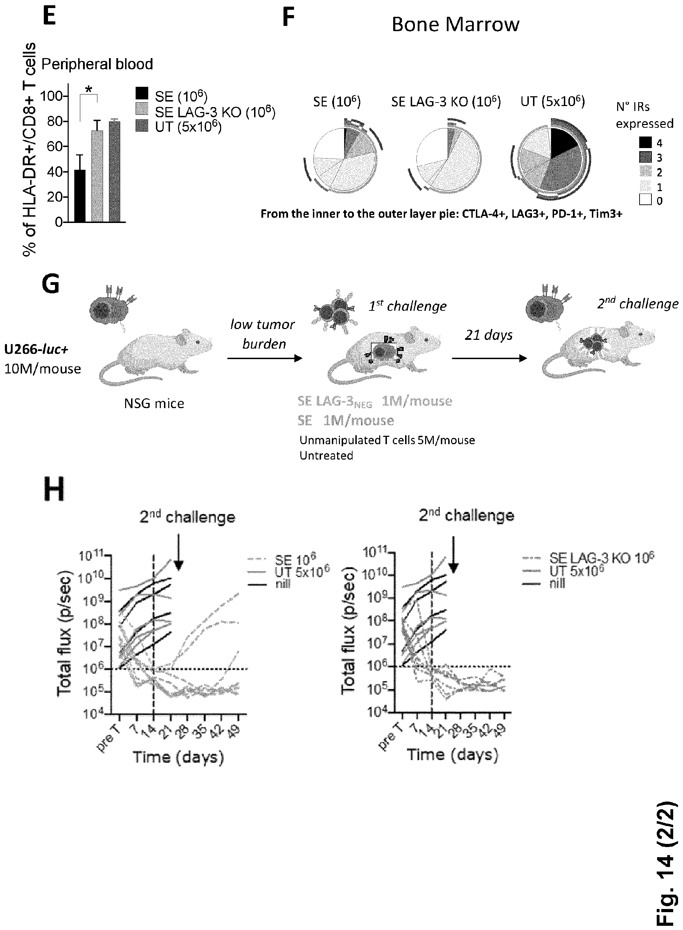

**Figure 14** **SE LAG KO T cells, but not SE LAG-3-competent T cells, efficiently respond upon secondary tumor challenge**
   Schematic representation of in vivo high tumor burden multiple myeloma model (U266) **(A).** Tumor burden (measured by total body bioluminescence) **(B)** and absolute numbers of circulating T cells **(C and D)** in mice treated with a low dose of SE cells, a low dose of SE LAG-3-KO cells, a high dose of SE cells, a high dose of SE LAG-3-KO cells, or injected with a high dose of unmanipulated cells (UT). HLA-DR expression on circulating CD8+ T cells in SE and SE LAG-3 KO low dose cohorts and unmanipulated cells (UT) **(E).** Inhibitory receptors expression in T cells infiltrating the bone marrow of mice treated with low doses of SE or SE LAG-3 KO T cells or injected with unmanipulated cells (UT). The slices in grayscale within the pie indicate the total number of co-expressed inhibitory receptors, while the external pie arches indicate the specific inhibitory receptor expressed **(F).** Schematic representation of re-challenge multiple myeloma (U266) pre-clinical model **(G).** Tumor burden (measured by total body bioluminescence) of mice rechallenged with U266 cells an after treatment with low dose of SE T cells (left panel) or a low dose of SE LAG-3 KO T cells (right panel), or injected with a high dose of unmanipulated cells (UT, solid grey lines) or left untreated (no treatment, black lines). Black arrows indicate the time-point of the second tumor cell infusion. The horizontal dashed lines represent the limit of tumor detection **(H).** Data are shown as mean ± SEM; *: p value <0.05.

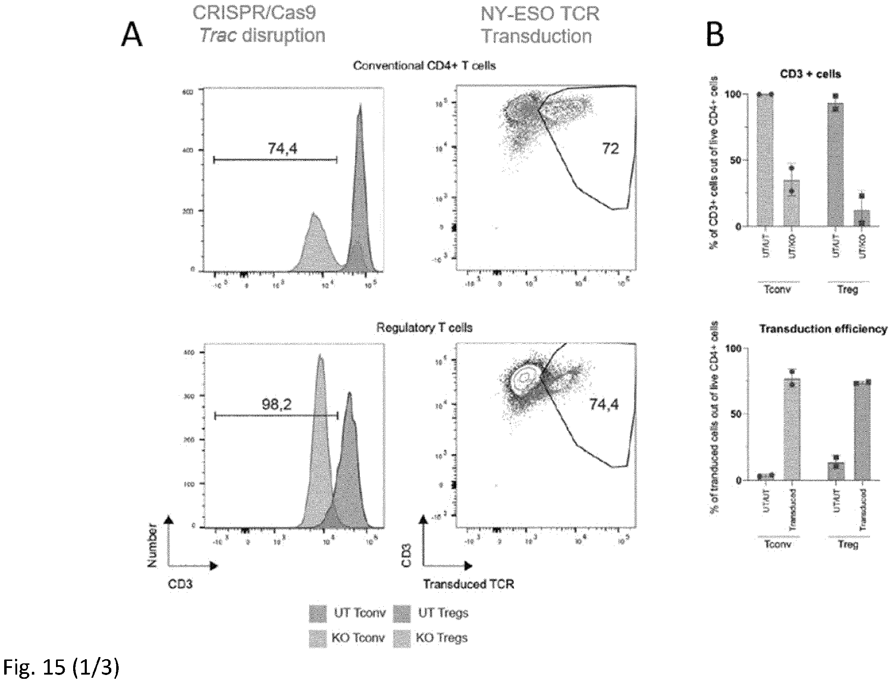

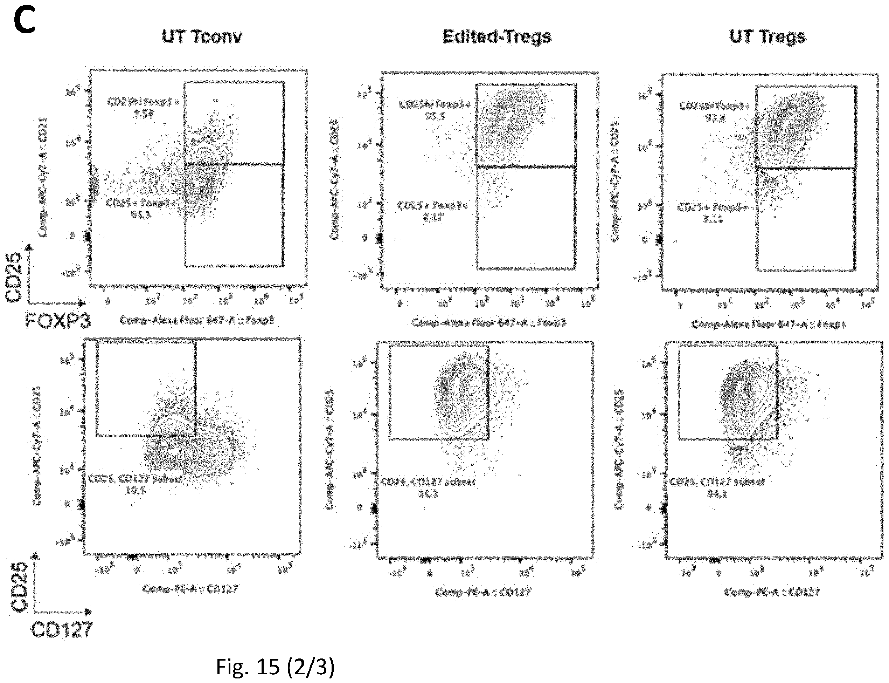

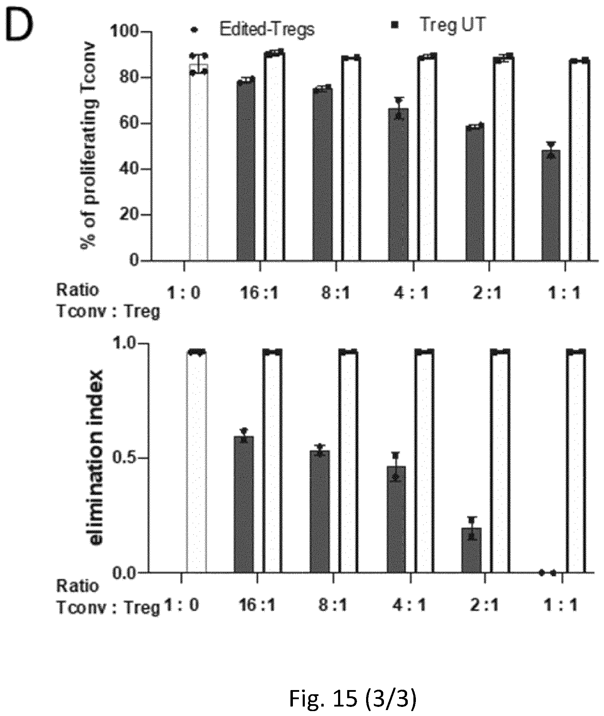

**Figure 15****. Generation of edited Tregs and *in vitro* validation.** Representative FACS plots of Tconv (top plots) and Tregs (bottom plots) are shown. The 2 histograms on the left show CD3 surface expression in not electroporated untransduced (UT) Tconv and Tregs (dark grey) compared to TRAC KO untransduced Tconv and Tregs (lighter grey). Contour plots on the right show the surface expression of CD3 and of NYESO1-specific TCR (measured as surface expression of the TCR Vb chain), in UT Tconv and Tregs (dark grey) compared to edited Tconv and Tregs (lighter grey) **(A).** Percentage of *Trac* disruption (top panel) and transduction efficiency (bottom panel) in Tconv (left columns) and Tregs (right columns). Data reported as mean ± SEM (n=2) **(B).** Representative FACS plots of UT Tconv (left panel), Edited Tregs (central panel) and UT Tregs (right panel) displaying the expression of CD25, Foxp3 and CD127 **(C).** Percentage of proliferating edited Tconv (top graph) and cell line elimination index (lower graph) in the presence of UT Tregs (white columns) or edited Tregs (grey columns) as assessed via FACS analysis **(D).** Data reported as mean ± SEM (n=2).

### Detailed Description of the Invention

It is a further object of the invention an isolated guide ribonucleic acid (gRNA) comprising at least one guide sequence which directs a nuclease to a sequence comprised in:
- Exon 2 of Tim 3 (e.g. g1-g4 Tim-3);
- Exon 3 of Tim-3 (e.g. g5-g10 Tim-3);
- Exon 3 of LAG-3 (e.g. g3, g18 LAG-3);
- Exon 1 (e.g. g33, g10, g16 LAG-3);
- Exon 1 of 2B4 (e.g. g1-g3 2B4);
- Exon 2 (g9-g11 2B4);
- Exon 1 of PD1 (e.g. g1, g2 PD1);
- Exon 2 of PD1 (e.g. g3, g4 PD1);
- Exon 2 of CD39 (e.g. g4 and g5 CD39);
- Exon 1 of TRAC (e.g. g1 TRAC); or
- a shared common region of exon1 between TRBC1 and TRBC2 (e.g. g1-g3 for TRBC1/TRBC2)
and combinations thereof.

It is a further object of the invention an isolated guide ribonucleic acid (gRNA) comprising at least one guide sequence which directs a nuclease to an inhibitory receptor (IR) sequence and/or to a TCR α (TRAC) and/or β chain (TRBC1/2) constant region sequence, said inhibitory receptor (IR) sequence and/or to a TCR α (TRAC) and/or β chain (TRBC1/2) constant region sequence being selected from SEQ ID Nos:28-41, 44, 46, 47, 49-58. The inhibitory receptor (IR) sequence and/or TCR α (TRAC) and/or β chain (TRBC1/2) constant region sequence preferably doesn't comprise the PAM sequence and therefore corresponds to nt. 1-20 of a sequence selected from SEQ ID Nos: 28-41, 44, 46, 47, 49-58.

Said inhibitory receptor (IR) is preferably selected from the group consisting of: Tim-3, LAG-3, 2B4, PD-1 and CD39.

An object of the present invention is also an isolated guide ribonucleic acid (gRNA) comprising at least one guide sequence complementary to the genomic region shown in Table 5, according to coordinates from human reference genome hg38, or complementary to sequences in the close vicinity of the genomic coordinate listed in Table 5, e.g. complementary to sequences that comprise 15 consecutive nucleotides ±10 nucleotides of a genomic coordinate listed Table 5.

Another object of the invention is an isolated guide ribonucleic acid (gRNA) comprising at least one guide sequence which directs a nuclease to an inhibitory receptor (IR) target sequence and/or to a TCR α (TRAC) and/or β chain (TRBC1/2) constant region target sequence, said guide sequence corresponding to an inhibitory receptor (IR) sequence and/or TCR α (TRAC) and/or β chain (TRBC1/2) constant region sequence comprised, comprising or consisting of a sequence selected from SEQ ID Nos: 28-41, 44, 46, 47, 49-58. The inhibitory receptor (IR) sequence and/or TCR α (TRAC) and/or β chain (TRBC1/2) constant region sequence preferably doesn't comprise the PAM sequence and therefore corresponds to nt. 1-20 of a sequence selected from SEQ ID Nos: 28-41, 44, 46, 47, 49-58.

A further object of the invention is an isolated guide ribonucleic acid (gRNA) comprising a guide sequence selected from the group consisting of SEQ ID NOs:1-27.

A further object of the invention is an isolated guide ribonucleic acid (gRNA) comprising the following sequences:
UCUCCGAGAGCCCGUAGAACGUUUUAGAGCUAUGCU (SEQ ID NO: 112),
CAAACACAGCGACCUCGGGUGUUUUAGAGCUAUGCU (SEQ ID NO:113),
UGACAGCGGAAGUGGUUGCGGUUUUAGAGCUAUGCU (SEQ ID NO:114),
GAGAAUCAAAAUCGGUGAAUGUUUUAGAGCUAUGCU (SEQ ID NO:115),
AGUUGAGAAACCCCGCCUACGUUUUAGAGCUAUGCU (SEQ ID NO:116),
CACCGCGGCGCGGUACUCGCGUUUUAGAGCUAUGCU (SEQ ID NO:117),
GCUCAGCACCGUGUAGCGGCGUUUUAGAGCUAUGCU (SEQ ID NO:118),
CUAAAUGGGGAUUUCCGCAAGUUUUAGAGCUAUGCU (SEQ ID NO:119),
AUCCCCAUUUAGCCAGUAUCGUUUUAGAGCUAUGCU (SEQ ID NO:120) or
GAGUCACAUUCUCUAUGGUCGUUUUAGAGCUAUGCU (SEQ ID NO:121).

In the present invention are also included the corresponding protein encoded from orthologous or homologous genes, functional mutants, functional derivatives, functional fragments or analogues, isoforms of the proteins herein mentioned.

The present invention also includes the sequences mentioned herein in the reverse orientation, from 3' to 5' and the complementary sequence and the reverse complement sequence.

In the composition of the invention, the term gRNA or guide sequence includes the nucleic acid encoding it and the corresponding DNA sequence (i.e. a sequence having T instead of U nucleotides).

A further object of the invention is a recombinant lentivirus, retrovirus, adenovirus, or adeno-associated virus (AAV) vector comprising a viral genome comprising a promoter sequence and a polynucleotide sequence encoding a genome editing means operably linked to the promoter sequence,
wherein said genome editing means comprises CRISPR/Cas9 comprising a Cas9 protein and at least one guide RNA (gRNA) as defined above or at least one nucleic acid as above defined or the CRISPR-Cas system as above defined.

Another object of the invention is a cell comprising the nucleic acid or a CRISPR/Cas9 system, or a vector as above defined.

A further object of the invention is a kit comprising at least one gRNA as defined above or a nucleic acid as defined above and optionally further comprising a CRISPR/Cas9 protein or nucleic acid encoding the CRISPR/Cas9 protein, preferably the kit comprises the CRISPR/Cas9 system as above defined.

The nucleic acid molecule of the invention may further comprise a second nucleic acid, which is optionally a second expression cassette, encoding an antigen receptor (CAR) or a TCR.
Examples of antigens relevant for cancer immunotherapy to which the recombinant receptor as above defined may specifically bind are: EpCAM, HER-2, WT1, NY-ESO-1, K-RAS, TGF-bRII, RNF43, KRAS/NRAS, PIK3CA, DOT1L, VAMP7, IFT43, CCRL2, CTDP1, NDC80, AVL9, NOP16, CTDP1, UTP20, C2CD4A, PTGFRN, SULF1, TACC2, AGR2, ANXA2, CEACAM6, DKK1, EGFR, ENOl, EPCAM, LGALS3, MET, MSLN, MUC1, MUC4, MUC16, MUC5AC, SERPINB3, TMPRSS4, TSPAN1, ECT2, TOP2A, TWIST1, Cyclin A1, Cathepsin G, EZH2, RHAMM, PRAME, PROTEINASE 2, NEUTROPHIL ELASTASE, SURVIVIN, BAALC, HBG2, MAGE-A2, hTERT. An example of antigen relevant for autoimmune diseases to which the recombinant receptor as above defined may specifically bind is vimentin and citrullinated vimentin.

Preferably the Cas9 molecule is a Cas9 engineered with one or more nuclear localization sequences.

Preferably the Cas9 molecule is a S. pyrogenes or S. aureus Cas9 molecule.

The guide RNA(s) of the invention may be comprised in an RNP. The guide RNA and the RNA-guided DNA binding agent, such as a Cas nuclease, e.g., a Cas cleavase, Cas nickase, or dCas DNA binding agent, preferably Cas9, composition is preferably comprised in a ribonucleoprotein (RNP) or "RNP complex".

Preferably, the gRNA is delivered to a cell as part of a RNP. More preferably, the gRNA is introduced in a cell with an mRNA encoding the RNA-guided DNA nuclease.

The gRNA and compositions of the invention may be introduced in the cell by any means known to the expert in the art, e.g., by electroporation, transfection, biolistics, lipid nanoparticle, liposomes virosomes, immunoliposomes, microinjection, viral vector, naked nucleic acid (e.g., naked DNA/RNA), artificial virions, or agent- enhanced uptake of DNA.

It is another object of the invention a method of producing a genetically engineered immune cell, comprising introducing into an immune cell at least one gRNA or the composition or the nucleic acid molecule as defined above. Preferably such methods comprise the steps disclosed in Cieri *et al,* 2013 or in WO2007/017915, both herein incorporated by reference, enabling the generation of an engineered cellular product highly enriched in long-living memory stem T cells (TSCM) and central memory (TCM) lymphocytes, resistant to inhibitory signals and specific for an antigen of choice. In alternative embodiments, the cellular product to be genetically engineered can be a selected population of mixed T naive (TN) and stem memory T cells (TSCM) or effector T cells.

Preferably the nucleic acid molecule of the invention is inserted into a vector, preferably a lentiviral vector, more preferably a mono- or bi-directional vector.

Preferably the introduction of the gRNA or of the composition of the invention in a cell comprises contacting the immune cell with the gRNA in vitro, more preferably it comprises electroporation. The above method may further comprise introducing into the immune cell a nucleic acid molecule encoding a recombinant protein, preferably a recombinant receptor.

Preferably the immune cell is a primary cell from a subject.

Preferably the immune cell is a human cell, such as a white blood cell or a human peripheral blood mononuclear cell.

The cell of the invention comprising a polynucleotide of the invention or a vector of the invention may further comprise a TCR or a CAR or a TRUCK or a protein involved in T cell function and survival or a cell marker.

In one embodiment, the cell is a T-cell, a lymphocyte, or a stem cell, optionally wherein the T-cell, the lymphocyte, or the stem cell is selected from the group consisting of CD4 cells, CD8 cells, naive T-cells, memory stem T-cells, central memory T-cells, double negative T- cells, effector memory T-cells, effector T-cells, ThO cells, TcO cells, Th1 cells, Tc1 cells, Th2 cells, Tc2 cells, Th17 cells, Th22 cells, gamma/delta T-cells, natural killer (NK) cells, natural killer T (NKT) cells, cytokine-induced killer (CIK) cells, hematopoietic stem cells and pluripotent stem cells.

In one embodiment, the cell is a T-cell which has been isolated from a subject.

In one embodiment, an endogenous gene encoding a TCR a chain and/or an endogenous gene encoding a TCR b chain in the cell is disrupted, preferably such that the endogenous gene encoding a TCR a chain and/or the endogenous gene encoding a TCR b chain is not expressed. In one embodiment, the endogenous gene encoding a TCR a chain and/or the endogenous gene encoding a TCR b chain is disrupted by insertion of an expression cassette comprising a polynucleotide sequence encoding a TCR or a CAR or a TRUCK.

In one embodiment, IR gene as disclosed above is disrupted by integration of an expression cassette, wherein the expression cassette comprises a polynucleotide sequence encoding a TCR or a CAR or a TRUCK or a protein involved in cell function and/or in cell expansion and/or in cell survival or a cell marker.

In another aspect, the invention provides a method of preparing a cell, which comprises the step of introducing the vector of the invention into a cell in vitro, ex vivo or in vivo, for example by transfection or transduction.

In another aspect, the invention provides a method of preparing a cell, which comprises the step of transducing a cell in vitro, ex vivo or in vivo with one or more vectors of the invention.

In one embodiment, the cell to be transduced with the one or more vectors is selected from the group consisting of T-cells, lymphocytes or stem cells, such as hematopoietic stem cells or induced pluripotent stem cells (iPS), optionally the T-cell, the lymphocyte or the stem cell may be selected from the group consisting of CD4 cells, CD8 cells, ThO cells, TcO cells, Th1 cells, Tc1 cells, Th2 cells, Tc2 cells, Th17 cells, Th22 cells, gamma/delta T-cells, natural killer (NK) cells, natural killer T (NKT) cells, invariant NKT (iNKT) cells, double negative T-cells, naive T-cells, memory stem T-cells, central memory T-cells, effector memory T-cells, effector T cells, cytokine- induced killer (CIK) cells, mucosal associated invariant T cell (MAIT), hematopoeitic stem cells and pluripotent stem cells.

In one embodiment, the method comprises the step of T-cell editing, which comprises disrupting an endogenous gene encoding a TCR a chain and/or an endogenous gene encoding a TCR b chain with the gRNA of the invention.

In one embodiment, the method comprises the step of targeted integration of an expression cassette into the endogenous gene encoding the TCR a chain and/or the endogenous gene encoding the TCR b chain disrupted with the gRNA of the invention, wherein the expression cassette comprises a polynucleotide sequence encoding a TCR or a CAR a TRUCK.

In another aspect, the invention provides the cell of the invention or a cell prepared by the method of the invention for use in adoptive cell transfer, preferably adoptive T-cell transfer, optionally wherein the adoptive T-cell transfer is allogenic adoptive T-cell transfer, autologous adoptive T-cell transfer, or universal non-alloreactive adoptive T-cell transfer.

In another aspect, the invention provides a chimeric molecule comprising the TCR or a CAR or a TRUCK, or a portion thereof, conjugated to a non-cellular substrate, a toxin and/or an antibody. In one embodiment, the non-cellular substrate is selected from the group consisting of nanoparticles, exosomes and other non-cellular substrates.

Preferably the immune cell is CD3 positive.

Preferably the immune cell is a T cell (e.g., a CD4+or CD8+ T cell, CAR-T cell, NK T cell, alpha beta T cell or gamma delta T cell) or NK cell.

The above method is preferably performed on a plurality of immune cells.

Another object of the invention is an immune cell or cell population produced by the above method.

It is another object of the invention a method of altering a T cell comprising contacting the T cell with a gRNA or composition or nucleic acid molecule as defined above.

In the context of the present invention, the T cell may be from a subject suffering from cancer, e.g., a cancer selected from the group consisting of: a leukemia, lymphoma, chronic lymphocytic leukemia (CLL), lymphoma, chronic lymphocytic leukemia (CLL), B cell acute lymphocytic leukemia (B-ALL), acute lymphoblastic leukemia (ALL), acute myeloid leukemia, myelodysplasia, myeloproliferative neoplasm, Hodgkin's lymphoma, non-Hodgkin's lymphoma (NHL), diffuse large cell lymphoma (DLCL), indolent B cell lymphoma, refractory follicular lymphoma, mantle cell lymphoma, multiple myeloma, renal cell carcinoma (RCC), B cell malignancies, colon cancer, neuroblastoma, cervical carcinoma, colorectal cancer, breast cancer, bone cancer, ovarian cancer, skin cancer, melanoma, sarcoma, prostate cancer, lung cancer, liver cancer, esophageal cancer, hepatocellular carcinoma, pancreatic cancer, gastric cancer, astrocytoma, brain cancer, epithelial cancers, renal cell carcinoma (RCC), Ewing sarcoma, neuroblastoma, pancreatic adenocarcinoma, mesothelioma, glioblastoma, osteosarcoma, synovial sarcoma, head and neck cancer, medulloblastoma,

Alternatively, the T cell may also be from healthy donors.

Preferably the contacting with the agent is in vitro or ex vivo.

Preferably the method according to the invention further comprises contacting the T cell with a nucleic acid encoding a recombinant receptor, under conditions to introduce the nucleic acid into the T cell.

It is an object of the invention an altered T cell or cell population produced by the method as defined above.

It is therefore an object of the invention an engineered cell in which at least one IR gene selected from LAG-3, TIM-3, 2B4, CD39 and/or PD-1 and optionally at least one TRAC and/or TRCB1/2 gene were disrupted by the gRNA(s) or compositions as defined above.

It is a further object of the invention a method of enhancing cytotoxicity, inhibiting exhaustion, and/or enhancing infiltration in tumors, and/or enhancing expansion, and/or altering differentiation and/or enhancing persistence of an immune cell population, the method comprising contacting the immune cell population with a gRNA or composition or nucleic acid according to the invention.

Preferably the contacting is effective to induce genetic disruption (e.g., gene knockout) of at least one IR gene selected from LAG-3, TIM-3, 2B4, CD39 and/or PD-1 and optionally of at least one TRAC and/or TRCB1/2 in the immune cells to provide a cell population characterized by one or more of the following: (1) at least about 50%, 60%, 65%, 70%, 75%, 80%, 85%, 90%or 95% of cells in the cell population do not express the endogenous IR polypeptide and optionally TRAC and/or TRCB1/2; do not contain a contiguous IR gene and/or a functional IR gene; (2) a IR gene knockout efficiency in the cell population of at least about 50%, 60%, 65%, 70%, 75%, 80%, 85%, 90%or 95%, e.g., as determined by a method in accordance with the Western Blot assay and/or flow cytometry and/or immunofluorescence and/or immunohistochemistry and/or molecular analysis (e.g. surveyor assay, droplet digital PCR) and/or as described herein; (3) the percentage of cells in the cell population expressing LAG-3, TIM-3, 2B4, CD39 and/or PD-1 on cell surface, as determined by flow cytometry, is lower than that in the immune cell population measured prior to the contacting.

The method preferably further comprises selecting immune cells containing the genetic disruption (e.g., gene knockout) of the above IR genes and optionally of TRAC and/or TRCB1/2.

The method may comprise expanding immune cells containing the genetic disruption (e.g., gene knockout) preferably wherein the expanding is in vitro, ex vivo, or in vivo.

Preferably the immune cell population is a T cell population (e.g., a CAR T cell population).

Another object of the invention is a method of producing an immune cell (e.g., altering a T cell), comprising (a) obtaining an immune cell (e.g., T cell) from a human patient or a healthy subject; (b) introducing gRNA or composition or nucleic acid molecule as defined above; and (c) incubating and optionally expanding the immune cell (e.g., T cell) to provide a gene disrupted immune cell (e.g., T cell) population.

It is a further object of the invention a method of enhancing cytotoxicity, inhibiting exhaustion, and/or enhancing infiltration in tumors, and/or enhancing expansion, and/or altering differentiation and/or enhancing effector functions, and/or enhancing persistence of an immune cell population, comprising (a) obtaining an immune cell (e.g., T cell) population from a human patient; (b) introducing the gRNA or composition of nucleic acid molecule as defined above in the immune cell (e.g., T cell) population; and (c) incubating and optionally expanding the immune cell (e.g., T cell) to provide a gene disrupted immune cell (e.g., T cell) population that has enhanced cytotoxicity, reduced exhaustion, and/or enhanced infiltration in tumors and/or enhanced expansion, and/or altered differentiation and/or enhanced cytokine production and/or enhanced secretion of effector molecules and/or enhanced persistence compared to the cell population prior to introducing the agent.

It is a further object of the invention a cell population comprising the cells as above defined.

The contacting of the immune cell (e.g., T cell) with a nucleic acid encoding a recombinant receptor is preferably carried out under conditions to introduce the nucleic acid into the immune cell, which can occur simultaneously or sequentially in any order to the introducing of the gRNA. Preferably the human patient suffers from cancer, wherein the cancer is a leukemia, lymphoma, chronic lymphocytic leukemia (CLL), lymphoma, chronic lymphocytic leukemia (CLL), B cell acute lymphocytic leukemia (B-ALL), acute lymphoblastic leukemia (ALL), acute myeloid leukemia, myelodysplasia, myeloproliferative neoplasm, Hodgkin's lymphoma, non-Hodgkin's lymphoma (NHL), diffuse large cell lymphoma (DLCL), indolent B cell lymphoma, refractory follicular lymphoma, mantle cell lymphoma, multiple myeloma, renal cell carcinoma (RCC), B cell malignancies, colon cancer, neuroblastoma, cervical carcinoma, colorectal cancer, breast cancer, bone cancer, ovarian cancer, skin cancer, melanoma, sarcoma, prostate cancer, lung cancer, liver cancer, esophageal cancer, hepatocellular carcinoma, pancreatic cancer, gastric cancer, astrocytoma, brain cancer, epithelial cancers, renal cell carcinoma (RCC), Ewing sarcoma, neuroblastoma, pancreatic adenocarcinoma, mesothelioma, glioblastoma, osteosarcoma, synovial sarcoma, head and neck cancer or medulloblastoma,

Preferably the T cell of the invention is a primary T cell from a cancer patient or from a subject, a CAR-T cell, a CAR NK cell, or an NK cell.

As used herein the term "disrupting" refers to reducing, limiting, preventing, silencing, or abrogating expression of a gene.

The methods of preparing a cell (e.g. a T-cell) of the invention may comprise the step of targeted integration of an expression cassette into an endogenous gene (e.g. an endogenous TCR a chain gene and/or an endogenous TCR b chain gene). As used herein the term expression cassette refers to a polynucleotide sequence (e.g. a DNA polynucleotide sequence) comprising one or more polynucleotide sequences encoding one or more genes of interest such that said genes of interest are capable of expression. Endogenous sequences may facilitate expression from the expression cassette, and/or transcription control sequences within the expression cassette may facilitate expression. For example, the expression cassette may comprise a polynucleotide sequence of the invention, or a polynucleotide sequence encoding a TCR or a CAR or a TRUCK, operably linked to an expression control sequence, e.g. a promoter or an enhancer sequence. The one or more genes of interest may be located between one or more sets of restriction sites. Suitably, the restriction sites may facilitate the integration of the expression cassette into, e.g., a vector, a plasmid, or genomic DNA (e.g. host cell genomic DNA).

For example, an expression cassette of the invention may be transferred from a first polynucleotide sequence, e.g. on a vector, to another by 'cutting', e.g. excising, the expression cassette using one or more suitable restriction enzymes and 'pasting', e.g. integrating, the expression cassette into a second polynucleotide sequence.

The expression cassette may comprise a recombinant polypeptide, such as a receptor. The expression cassette may comprise a polynucleotide encoding one or more TCRs or CARs or TRUCKs. The expression cassette may further comprise an antibiotic resistance gene or other selectable marker gene that allows cells that have successfully integrated the expression cassette into their DNA to be identified. The expression cassette may further comprise a gene encoding for a protein involved in cell function and/or cell expansion and/or survival or a cell marker. The polynucleotide sequences comprised in the expression cassette may be operably linked to expression control sequences, e.g. a suitable promoter or enhancer sequence. The person skilled in the art will be able to select suitable expression control sequences.

The polynucleotides and vectors of the invention may be transferred into specific T-cell subsets, including CD4 and or CD8, naive, memory stem T cells, central memory, effector memory or effector cells, or in other cellular subsets such as to promote different in vivo length of persistence and function in the cells of the invention.

The polynucleotides and vectors of the invention may also be transferred into T-cell subsets such as naive, memory stem T cells, central memory cells, effector memory cells, effectors, regulatory T cells.

The polynucleotides and vectors of the invention may also be transferred into T-cell subsets with different polarizations, such as ThO/TcO, Th1/Tc1, Th2/Tc2, Th17, Th22 or others, depending on the cytokine background most appropriate to target a particular tumor type.

Furthermore, the polynucleotides and vectors of the invention may be transferred in other cellular subsets, including gamma/delta T-cells, NK cells, NKT cells, cytokine-induced killer (CIK) cells, MAIT, iNKT cells, hematopoietic stem cells or other cells, in order to obtain the therapeutic effect.

Further provided by the invention is a method of preparing a cell, which comprises the step of transducing a cell in vitro or ex vivo with a vector of the invention. Various methods for transduction of a cell with a vector are known in the art (see e.g. Sambrook et al).

The invention also provides a method of producing a T-cell expressing a TCR by inducing the differentiation of a stem cell which comprises a polynucleotide or a vector as defined above.

A population of cells may be purified selectively for cells that exhibit a specific phenotype or characteristic, and from other cells which do not exhibit that phenotype or characteristic, or exhibit it to a lesser degree. For example, a population of cells that expresses a specific marker (e.g. CD3, CD4, CD8, CD25, CD127, CD152, CXCR3, or CCR4) may be purified from a starting population of cells. Alternatively, or in addition, a population of cells that does not express another marker may be purified.

By "enriching" a population of cells for a certain type of cells it is to be understood that the concentration of that type of cells is increased within the population. The concentration of other types of cells may be concomitantly reduced.

Purification or enrichment may result in the population of cells being substantially pure of other types of cell.

Preferably, the nucleic acid encoding the Cas9 protein is codon-optimized for expression in human cells. The single-chain guide RNA, including the crRNA and the tracrRNA, can be an in vitro transcribed.

Preferably the target DNA sequence comprises the nucleotides complementary to the guide RNA and a trinucleotide protospacer adjacent motif (PAM).

Also included in the present invention are truncated forms of the gRNA herein defined.

A further object of the invention is a composition comprising a guide RNA according to the invention and at least one delivery means selected from GalNAC, polymers, liposomes, peptides, aptamers, antibodies, viral vectors, transposons, folate or transferrin.

Another object of the invention is a method for treating cancer in a subject in need thereof, the method comprising:
(a) providing a non-naturally occurring nuclease system comprising one or more vectors comprising:
   i) a promoter operably linked to at least one nucleotide sequence encoding a CRISPR system guide RNA (gRNA), wherein the gRNA hybridizes with a target sequence of a DNA molecule in a cell of the subject, and wherein the DNA molecule encodes one or more gene products expressed in the cell; and
   ii) a regulatory element operable in a cell operably linked to a nucleotide sequence encoding a genome-targeted nuclease, wherein components (i) and (ii) are located on the same or different vectors of the system, wherein the gRNA targets and hybridizes with the target sequence and the nuclease cleaves the DNA molecule to alter expression of the one or more gene products; and
(b) administering to the subject a therapeutically effective amount of the system, wherein the gRNA is as defined above.

Preferably, the system is packaged into RNP or into two adeno-associated virus (AAV) vectors and/or inactivates one or more gene products.

Preferably the Cas9 protein is codon optimized for expression in the cell.

A further object of the invention is a method of altering expression of one or more gene products in a cell, wherein the cell comprises a DNA molecule encoding the one or more gene products, the method comprising introducing into the cell a non-naturally occurring nuclease system comprising one or more vectors comprising:
a) a promoter operably linked to at least one nucleotide sequence encoding a nuclease system guide RNA (gRNA), wherein the gRNA hybridizes with a target sequence of the DNA molecule; and
b) a regulatory element operable in the cell operably linked to a nucleotide sequence encoding a genome-targeted nuclease,
   wherein components (a) and (b) are located on the same or different vectors of the system,
   wherein the gRNA targets and hybridizes with the target sequence and the nuclease cleaves the DNA molecule to alter expression of the one or more gene products
   and wherein the gRNA is as defined above. Preferably the system is CRISPR.

Preferably the system is packaged in RNP or into two adeno-associated virus (AAV) vectors.

Preferably the system inactivates one or more gene products.

Preferably the genome-targeted nuclease is SpCas9. Preferably the cell is a eukaryotic or non-eukaryotic cell, preferably a mammalian or human cell.

Preferably, the one or more gene products are IRs such as LAG-3, TIM-3, 2B4, CD39, PD-1 and/or TRAC or TRBC. LAG-3 gene presents preferably the sequence as described in NCBI, with accession no. Gene ID: 3902 NC_0000.12, GRCh38.p13 primary assembly. TIM-3 gene presents preferably the sequence as described in NCBI, with accession no. **Gene** ID: **84868** NC_000005.10, GRCh38.p13 primary assembly. 2B4 gene presents preferably the sequence as described in NCBI, with accession no. **Gene ID: 51744** NC_00000.1.11, GRCh38.p13 primary assembly. CD39 gene presents preferably the sequence as described in NCBI, with accession no. Gene ID: 953 NC_000010.11, GRCh38.p13 primary assembly. PD-1 gene presents preferably the sequence as described in NCBI, with accession no. **Gene ID: 5133** NC_000002.12 , GRCh38.p13 primary assembly. TRAC gene presents preferably the sequence as described in NCBI, with accession no. **Gene ID: 28755** NC_000014.9, GRCh38.p13). TRBC1 gene presents preferably the sequence as described in NCBI, with accession no. **Gene ID: 6957** NC_000007.14, GRCh38.p13. TRBC2 gene presents preferably the sequence as described in NCBI, with accession no. **Gene ID: 6957** NC_000007.14, GRCh38.p13.

Preferably the expression of the one or more gene products is decreased or abolished.

Preferably the Cas9 protein comprises an amino acid sequence set forth in the Database UniProtKB/Swiss-Prot, with the Accession number Q99ZW2, Streptococcus pyogenes serotype M1 (SpCas9). Preferably, the Cas9 protein is a recombinant S. pyogenes Cas9 nuclease, purified from an E. coli strain expressing the nuclease, containing nuclear localization sequence (NLS) and C-terminal 6-His tag.

In an embodiment of the invention, from one to five of the terminal nucleotides at 5' end and/or 3' end of the guide RNA may be chemically modified to enhance stability, preferably three terminal nucleotides at 5' end and/or 3' end of the guide RNA are chemically modified to enhance stability, preferably the chemical modification is modification with 2'-O-methyl 3'phosphorothioate.

In the recombinant adeno-associated virus vector according to the invention the gRNA comprises polynucleotide sequence set forth in any of SEQ ID NO:1-27 or nt. 1-20 of a sequence selected from SEQ ID Nos: 28-41, 44, 46, 47, 49-58 or comprises a polynucleotide sequence having 90% or more homology with a polynucleotide sequence set forth in any of SEQ ID NOs: 1-27 or to nt. 1-20 of a sequence selected from SEQ ID Nos: 28-41, 44, 46, 47, 49-58 or comprises nucleic acid encoding said gRNAs, and can be combined with a Cas9 protein as defined above.

Other objects of the invention are DNA or RNA vectors encoding any of the guide RNAs comprising any one or more of the guide sequences described herein.

The nucleic acid encoding the Cas9 protein may be introduced into the mammalian cell before introducing the single-chain guide RNA into the mammalian cell.

In the context of the present invention, the terms "guide RNA", and "gRNA" and "gRNA molecule" are used interchangeably. The gRNA comprises or consists a CRISPR RNA (crRNA) and a trRNA (tracrRNA). In some embodiment, the crRNA and trRNA are disposed on a single RNA molecule (single guide RNA (sgRNA)). Alternatively, they are disassociated on separate RNA molecules (dual guide RNA (dgRNA)).

In the context of the present invention, the term" guide sequence" refers to a sequence within the crRNA or trRNA that recognizes, e.g., is complementary to, to a target sequence, e.g. in a gene, that directs a guide RNA to a target sequence for cleavage by a nuclease. A "guide sequence" may also be indicated as a "targeting sequence," or a "spacer sequence. The degree of complementarity or identity between a guide sequence and its corresponding target sequence may be about 75%, 80%, 85%, 90%, 95%, 96%, 97%, 98%, 99%, or 100%.

The guide sequence is typically about 20-base pair long, but shorter or longer sequences are also included in the present invention (e.g., sequences 15-, 16-, 17-, 18-, 19-, 21-, 22-, 23-, 24-, or 25-base pairs long).

The tracr may comprise nucleic acid sequence that binds specifically to Cas9.

The term "target sequence" refers to a sequence of nucleic acid to which the gRNA directs a nuclease for cleavage. The target sequence is preferably disposed on genomic DNA. Preferably, a Cas protein may be directed by a guide RNA to a target sequence, where the guide RNA hybridizes with and the nuclease cleaves the target sequence. Target sequences comprise both the positive and negative strands of genomic DNA (i.e., the sequence given and the sequence's reverse complement), as a nucleic acid substrate for a Cas protein is a double stranded nucleic acid. The target sequence may be adjacent to (either on the same strand or on the complementary strand of DNA) a protospacer adjacent motif (PAM) sequence recognized by a protein having nuclease or other effector activity or epigenetic activity, e.g., a PAM sequence recognized by Cas9.

Accordingly, the guide sequence may be complementary, for example fully complementary, to a target sequence and the guide sequence may direct a guide RNA (e.g., in a RNP) to bind to the reverse complement of a target sequence provided herein. Thus, in some embodiments, where the guide sequence binds the reverse complement of a target sequence, the guide sequence is identical to the first 20 nucleotides of the target sequence (e.g., the target sequence not including the PAM) except for the substitution of U for T in the guide sequence. The length of the guide sequence may correspond to the length of the target sequence.

Preferably the guide RNA is a dual guide or a single guide. Preferably the guide RNA comprises a crRNA, a trRNA, or a crRNA and a trRNA. Preferably the at least one guide sequence is encoded on a vector. Preferably the vector comprises a first guide sequence and a second guide sequence. Alternatively, a first guide sequence and a second guide sequence are encoded on different vectors.

The first guide sequence and the second guide sequence may be controlled by the same promoter and/or regulatory sequence.

Preferably the guide sequence is complementary to a target sequence in the positive strand of a target gene.

Alternatively, the guide sequence is complementary to a target sequence in the negative strand of a target gene.

Preferably a first guide sequence and second guide sequence are complementary to a first target sequence and a second target sequence in opposite strands of a target gene.

The guide RNA may be chemically modified.

The composition of the invention may further comprise a nuclease, preferably a Cas protein, more preferably the Cas protein is from the Type-I, Type-II, or Type-III CRISPR/Cas system. The Cas protein is preferably Cas9 or Cpfl or nickase.

The nuclease may be modified.

The guide RNA and the Cas protein may form a "ribonucleoprotein" (RNP). "Ribonucleoprotein" (RNP) or "RNP complex" refers to a guide RNA together with an RNA-guided DNA binding agent, such as a Cas cleavase, nickase, or dCas DNA binding agent (e.g., Cas9).

The kit according to the invention may also comprise at least one nuclear localization signal, at least one cell penetrating peptide, at least one marker domain, or combination thereof.

Preferably, the pharmaceutical composition or the gRNAs of the invention may be administered to the subject by implantation, injection, or virally or by ex vivo gene therapy.

A further therapeutic agent may be administered in combination to the subject, wherein said further therapeutic agent is for the treatment and/or prevention of cancer.

The gRNA may also be modified as disclosed in Hendel A et al., Nat biotech 2015.

The term "vector" refers to a nucleic acid molecule capable of transporting another nucleic acid to which it has been linked. Vectors include, but are not limited to, nucleic acid molecules that are single-stranded, double-stranded, or partially double-stranded; nucleic acid molecules that comprise one or more free ends, no free ends (e.g. circular), nucleic acid molecules that comprise DNA, RNA, or both; and other varieties of polynucleotides known in the art. One type of vector is a "plasmid," which refers to a circular double stranded DNA loop into which additional DNA segments can be inserted, such as by standard molecular cloning techniques. Another type of vector is a viral vector, wherein virally-derived DNA or RNA sequences are present in the vector for packaging into a virus (e.g. retroviruses, replication defective retroviruses, lentiviruses, replication defective lentiviruses, adenoviruses, replication defective adenoviruses, and adeno-associated viruses). Viral vectors also include polynucleotides carried by a virus for transfection into a host cell.

Certain vectors are capable of autonomous replication in a host cell into which they are introduced (e.g. bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively-Linked. Such vectors are referred to herein as "expression vectors." Common expression vectors of utility in recombinant DNA techniques are often in the form of plasmids.

Recombinant expression vectors can comprise a nucleic acid of the presently disclosed subject matter in a form suitable for expression of the nucleic acid in a host cell, which means that the recombinant expression vectors include one or more regulatory elements, which may be selected on the basis of the host cells to be used for expression, that is operatively-Linked to the nucleic acid sequence to be expressed.

The terms "polynucleotide", "nucleotide", "nucleotide sequence", "nucleic acid" and "oligonucleotide" are used interchangeably. They refer to a polymeric form of nucleotides of any length, either deoxyribonucleotides or ribonucleotides, or analogs thereof.

In aspects of the presently disclosed subject matter the terms "chimeric RNA", "chimeric guide RNA", "guide RNA", "single guide RNA" and "synthetic guide RNA" are used interchangeably and refer to the polynucleotide sequence comprising the guide sequence. The term "guide sequence" refers to the about 20 bp sequence within the guide RNA that specifies the target site and may be used interchangeably with the terms "guide" or "spacer".

As used herein the term "variant" should be taken to mean the exhibition of qualities that have a pattern that deviates from what occurs in nature.

"Complementarity" refers to the ability of a nucleic acid to form hydrogen bond(s) with another nucleic acid sequence by either traditional Watson-Crick or other non-traditional types. A percent complementarity indicates the percentage of residues in a nucleic acid molecule which can form hydrogen bonds (e.g., Watson-Crick base pairing) with a second nucleic acid sequence (e.g., 5, 6, 7, 8, 9, 10 out of 10 being 50%, 60%, 70%, 80%, 90%, and 100% complementary). "Perfectly complementary" means that all the contiguous residues of a nucleic acid sequence will hydrogen bond with the same number of contiguous residues in a second nucleic acid sequence. "Substantially complementary" as used herein refers to a degree of complementarity that is at least 60%, 65%, 70%, 75%, 80%, 85%, 90%, 95%. 97%, 98%, 99%, or 100% over a region of 8, 9, 10, 11, 12, 13, 14, 15, 16, 17, 18, 19, 20, 21, 22, 23, 24, 25, 30, 35, 40, 45, 50, or more nucleotides, or refers to two nucleic acids that hybridize under stringent conditions.

Preferably, in the context of the present invention, nucleic acid molecules are completely complementary, if they are at least 80%, 85%, 90%, 95%, 99% complementary.

As used herein, "stringent conditions" for hybridization refer to conditions under which a nucleic acid having complementarity to a target sequence predominantly hybridizes with the target sequence, and substantially does not hybridize to non-target sequences.

Stringent conditions are generally sequence-dependent, and vary depending on a number of factors, in general, the longer the sequence, the higher the temperature at which the sequence specifically hybridizes to its target sequence. Non-limiting examples of stringent conditions are described in detail in Tijssen (1993), Laboratory Techniques In Biochemistry And Molecular Biology -Hybridization With Nucleic Acid Probes Part 1, Second Chapter "Overview of principles of hybridization and the strategy of nucleic acid probe assay", Elsevier, N.Y.

"Hybridization" refers to a reaction in which one or more polynucleotides react to form a complex that is stabilized via hydrogen bonding between the bases of the nucleotide residues. The hydrogen bonding may occur by Watson Crick base pairing, Hoogstein binding, or in any other sequence specific manner. The complex may comprise two strands forming a duplex structure, three or more strands forming a multi stranded complex, a single self-hybridizing strand, or any combination of these. A hybridization reaction may constitute a step in a more extensive process, such as the initiation of PGR, or the cleavage of a polynucleotide by an enzyme. A sequence capable of hybridizing with a given sequence is referred to as the "complement" of the given sequence.

As used herein, "expression" refers to the process by which a polynucleotide is transcribed from a DNA template (such as into and mRNA or other RNA transcript) and/or the process by which a transcribed mRNA is subsequently translated into peptides, polypeptides, or proteins. Transcripts and encoded polypeptides may be collectively referred to as "gene product." If the polynucleotide is derived from genomic DNA, expression may include splicing of the mRNA in a eukaryotic cell.

"CRISPR system" refers collectively to transcripts and other elements involved in the expression of or directing the activity of CRISPR-associated ("Cas") genes, including sequences encoding a Cas gene, a guide sequence (also referred to as a "spacer" in the context of an endogenous CRISPR system), or other sequences and transcripts from a CRISPR locus. In some embodiments, one or more elements of a CRISPR system is derived from a particular organism comprising an endogenous CRISPR system, such as Streptococcus pyogenes. In general, a CRISPR system is characterized by elements that promote the formation of a CRISPR complex at the site of a target sequence (also referred to as a protospacer in the context of an endogenous CRISPR system).

In the context of formation of a CRISPR complex, "target sequence" refers to a sequence to which a guide sequence is designed to have complementarity, where hybridization between a target sequence and a guide sequence promotes the formation of a CRISPR complex. Full complementarity is not necessarily required, provided there is sufficient complementarity to cause hybridization and promote formation of a CRISPR complex. A target sequence may comprise any polynucleotide, such as DNA or RNA polynucleotides. In some embodiments, a target sequence is located in the nucleus or cytoplasm of a cell. In some embodiments, the target sequence may be within an organelle of a eukaryotic cell, for example, mitochondrion or chloroplast. A sequence or template that may be used for recombination into the targeted locus comprising the target sequences is referred to as an "editing template" or "editing polynucleotide" or "editing sequence". In aspects of the presently disclosed subject matter, an exogenous template polynucleotide may be referred to as an editing template. In an aspect of the presently disclosed subject matter the recombination is homologous recombination.

In some embodiments, a vector comprises one or more insertion sites, such as a restriction endonuclease recognition sequence (also referred to as a "cloning site"). In some embodiments, one or more insertion sites (e.g. about or more than about 1, 2, 3, 4, 5, 6, 7, 8, 9, 10, or more insertion sites) are located upstream and/or downstream of one or more sequence elements of one or more vectors. When multiple different guide sequences are used, a single expression construct may be used to target CRISPR activity to multiple different, corresponding target sequences within a cell. For example, a single vector may comprise about or more than about 1, 2, 3, 4, 5, 6, 7, 8, 9, 10, 15, 20, or more guide sequences. In some embodiments, about or more than about 1, 2, 3, 4, 5, 6, 7, 8, 9, 10, or more such guide-sequence-containing vectors may be provided, and optionally delivered to a cell.

In general, a guide sequence is any polynucleotide sequence having sufficient complementarity with a target polynucleotide sequence to hybridize with the target sequence and direct sequence-specific binding of a CRISPR complex to the target sequence. In some embodiments, the degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith- Waterman algorithm, the Needleman-Wunsch algorithm, algorithms based on the Burrows- Wheeler Transform (e.g. the Burrows Wheeler Aligner), ClustalW, Clustal X, BLAT, Novoalign (Novocraft Technologies, ELAND (Alumina, San Diego, Calif), SOAP(available at soap.genomics.org.cn), and Maq (available at maq.sourceforge.net). In some embodiments, a guide sequence is about or more than about 5, 10, 11, 12, 13, 14, 1 5, 16, 17, 18, 19, 20, 21, 22, 23, 24, 25, 26, 27, 28, 29, 30, 35, 40, 45, 50, 75, or more nucleotides in length. In some embodiments, a guide sequence is less than about 75, 50, 45, 40, 35, 30, 25, 20, 15, 12, or fewer nucleotides in length.

In some embodiments, the target sequence may be 60%, 65%, 70%,75%, 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8% homologous to the nucleotide sequences set forth in the sequences herein disclosed.

The term "homologous" refers to the "% homology" and is used interchangeably herein with the term "% identity" herein, and relates to the level of nucleic acid sequence identity when aligned using a sequence alignment program. For example, as used herein, 80% homology means the same thing as 80% sequence identity determined by a defined algorithm, and accordingly a homologue of a given sequence has greater than 80% sequence identity over a length of the given sequence.

Exemplary levels of sequence identity include, but are not limited to about, 80%, 81%, 82%, 83%, 84%, 85%, 86%, 87%, 88%, 89%, 90%, 91%, 92%, 93%, 94%, 95%, 96%, 97%, 98%, 99.1%, 99.2%, 99.3%, 99.4%, 99.5%, 99.6%, 99.7%, 99.8% or more sequence identity to the nucleotide sequences set forth in the sequences herein discloses.

The phrase "pharmaceutically-acceptable carrier" as used herein means a pharmaceutically-acceptable material, composition or vehicle, such as a liquid or solid filler, diluent, excipient, or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; and (22) other non-toxic compatible substances employed in pharmaceutical formulations.

Included in the present invention are also nucleic acid sequences derived from the nucleotide sequences herein mentioned, e.g. functional fragments, mutants, variants, derivatives, analogues, and sequences having a % of identity of at least 80% with the sequences herein mentioned.

The term "functional" may be understood as capable of maintaining the same activity. "Fragments" are preferably long at least 10, 11, 12, 13, 14, 15, 16, 17, 18, 19, 20 nt. "Derivatives" may be recombinant or synthetic. The term "derivatives" also refers to longer or shorter polynucleotides and/or having e.g. a percentage of identity of at least 85%, 86%, 87%, 88%, 89%, 90%, 91%, 92%, 93%, 94%, 95%, 96%, 97%, 98%, more preferably of at least 99% with the sequences herein disclosed. In the present invention "at least 70 % identity" means that the identity may be at least 70%, or 75%, or 80%, or 85 % or 90% or 95% or 100% sequence identity to referred sequences. This applies to all the mentioned % of identity. Preferably, the % of identity relates to the full length of the referred sequence. Also within the scope of the subject invention are polynucleotides which have the same nucleotide sequences of a polynucleotide exemplified herein except for nucleotide substitutions, additions, or deletions within the sequence of the polynucleotide, as long as these variant polynucleotides retain substantially the same relevant functional activity as the polynucleotides specifically exemplified. Thus, the polynucleotides disclosed herein should be understood to include mutants, derivatives, variants and fragments, as discussed above, of the specifically exemplified sequences. The subject invention also contemplates those polynucleotide molecules having sequences which are sufficiently homologous with the polynucleotide sequences of the invention so as to permit hybridization with that sequence under standard stringent conditions and standard methods (Maniatis, T. et al, 1982). Polynucleotides described herein can also be defined in terms of more particular identity and/or similarity ranges with those exemplified herein. The sequence identity will typically be greater than 60%, preferably greater than 75%, more preferably greater than 80%, even more preferably greater than 90%, and can be greater than 95%. The identity and/or similarity of a sequence can be 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91 , 92, 93, 94, 95, 96, 97, 98, or 99% or greater as compared to a sequence exemplified herein.

A sequence that is at least 90% identical to a sequence include a sequence that is at least 90, 91 , 92, 93, 94, 95, 96, 97, 98, or 99% or 100% identical compared to a sequence exemplified herein. Unless otherwise specified, as used herein percent sequence identity and/or similarity of two sequences can be determined using the algorithm of Karlin and Altschul (1990), modified as in Karlin and Altschul (1993). Such an algorithm is incorporated into the NBLAST and XBLAST programs of Altschul et al. (1990). BLAST searches can be performed with the NBLAST program, score = 100, wordlength = 12, to obtain sequences with the desired percent sequence identity. To obtain gapped alignments for comparison purposes, Gapped BLAST can be used as described in Altschul et al. (1997). When utilizing BLAST and Gapped BLAST programs, the default parameters of the respective programs (NBLAST and XBLAST) can be used. See NCBI/N1H website.

Also included in the present invention are RNA sequences corresponding to the herein disclosed sequences, DNA sequences corresponding to the herein disclosed sequences, sequences complementary to such RNA or DNA sequences or to the herein disclosed sequences, reverse complementary sequence to such RNA or DNA sequences or to the herein disclosed sequences. In the context of the present invention the term "comprising" includes also "consisting of" or "consisting essentially of" and the term "consisting of" also includes the terms "comprising" or "consisting essentially of'.

### Sequences

### Guide sequences (in parenthesis the name of the guide and the target gene):

5' - GAGUCACAUUCUCUAUGGUC - 3' (g4 Tim-3) (SEQ ID NO:1),
5' - AAUGUGGCAACGUGGUGCUC -3' (g2 Tim-3) (SEQ ID NO:2),
5' - CUAAAUGGGGAUUUCCGCAA -3' (g1 Tim-3) (SEQ ID NO:3),
5 '- AUCCCCAUUUAGCCAGUAUC - 3' (g3 Tim-3) (SEQ ID NO:4),
5' - CUCUCUGCCGAGUCGGUGCA - 3' (g5 Tim-3) (SEQ ID NO:5),
5'- GUGAAGUCUCUCUGCCGAGU -3' (g6 Tim-3) (SEQ ID NO:6),
5'- UGCCCCAUGCAUAGUUACCU -3' (g7 Tim-3) (SEQ ID NO:7),
5' - AGGUCACCCCUGCACCGACU -3' (g8 Tim-3) (SEQ ID NO:8),
5' - UGGCCCAGGUAACUAUGCAU -3' (g9 Tim-3) (SEQ ID NO:9),
5' - CCAAGGAUGCUUACCACCAG -3' (g10 Tim-3) (SEQ ID NO:10),
5' - CACCGCGGCGCGGUACUCGC -3' (g3 Lag-3) (SEQ ID NO:11),
5' - GCUCAGCACCGUGUAGCGGC - 3' (g18 Lag-3) (SEQ ID NO:12),
5'- ACCUUCGUCUGUAUGCUGUU 3' (g1 2B4) (SEQ ID NO:13),
5' -ACCAAACAGCAUACAGACGA 3' (g2 2B4) (SEQ ID NO:14),
5'- AGUUGAGAAACCCCGCCUAC -3' (g9 2B4) (SEQ ID NO:15),
5' - GUUGAGAAACCCCGCCUACA -3' (g10 2B4) (SEQ ID NO:16),
5' - UUGAGAAACCCCGCCUACAG -3' (g11 2B4) (SEQ ID NO:17),
5' - UGUAGCACCGCCCAGACGAC -3' (g1 PD-1) (SEQ ID NO:18),
5' - CGUCUGGGCGGUGCUACAAC -3' (g2 PD-1) (SEQ ID NO:19),
5' - AGGUGCCGCUGUCAUUGCGC -3' (g3 PD-1) (SEQ ID NO:20),
5'- CCUGCUCGUGGUGACCGAAG -3' (g4 PD-1) (SEQ ID NO:21),
5' - AAGGAUGGCUAGGAUAUUCU -3' (g4 CD39) (SEQ ID NO:22),
5' - AAGAAUAUCCUAGCCAUCCU -3' (g5 CD39) (SEQ ID NO:23),
5' - GAGAAUCAAAAUCGGUGAAU -3' (g1 TRAC) (SEQ ID NO:24),
5'- UCUCCGAGAGCCCGUAGAAC 3' (g1 TRBC1/TRBC2) (SEQ ID NO:25),
5'- CAAACACAGCGACCUCGGGU -3' (g2 TRBC1/TRBC2) (SEQ ID NO:26),
5'- UGACAGCGGAAGUGGUUGCG -3' (g3 TRBC1/TRBC2) (SEQ ID NO:27)

### Table 5

**Table 5: guide sequences and chromosomal coordinates.**

| **SEQ ID NO:** | **GUIDE SEQUENCE** | **GENOMIC COORDINATES (hg38)** |
|---|---|---|
| **1** | **GAGUCACAUUCUCUAUGGUC** | chr5:157106717-157106736 |
| **2** | **AAUGUGGCAACGUGGUGCUC** | chr5:157106817-157106836 |
| **3** | **CUAAAUGGGGAUUUCCGCAA** | chr5:157106752-157106771 |
| **4** | **AUCCCCAUUUAGCCAGUAUC** | chr5:157106760-157106779 |
| **5** | **CUCUCUGCCGAGUCGGUGCA** | chr5:157104717-157104736 |
| **6** | **GUGAAGUCUCUCUGCCGAGU** | chr5:157104710-157104729 |
| **7** | **UGCCCCAUGCAUAGUUACCU** | chr5:157104648-157104667 |
| **8** | **AGGUCACCCCUGCACCGACU** | chr5:157104727-157104746 |
| **9** | **UGGCCCAGGUAACUAUGCAU** | chr5:157104654-157104673 |
| **10** | **CCAAGGAUGCUUACCACCAG** | chr5:157104680-157104699 |
| **11** | **CACCGCGGCGCGGUACUCGC** | chr12:6773924-6773943 |
| **12** | **GCUCAGCACCGUGUAGCGGC** | chr12:6773780-6773799 |
| **13** | **ACCUUCGUCUGUAUGCUGUU** | chr1:160841820-160841839 |
| **14** | **ACCAAACAGCAUACAGACGA** | chr1:160841824-160841843 |
| **15** | **AGUUGAGAAACCCCGCCUAC** | chr1:160841462-160841481 |
| **16** | **GUUGAGAAACCCCGCCUACA** | chr1:160841461-160841480 |
| **17** | **UUGAGAAACCCCGCCUACAG** | chr1:160841460-160841479 |
| **18** | **UGUAGCACCGCCCAGACGAC** | chr2:241858792-241858811 |
| **19** | **CGUCUGGGCGGUGCUACAAC** | chr2:241858790-241858809 |
| **20** | **AGGUGCCGCUGUCAUUGCGC** | chr2:241852696-241852715 |
| **21** | **CCUGCUCGUGGUGACCGAAG** | chr2:241852918-241852937 |
| **22** | **AAGGAUGGCUAGGAUAUUCU** | chr10:95823264-95823283 |
| **23** | **AAGAAUAUCCUAGCCAUCCU** | chr10:95823263-95823282 |
| **24** | **GAGAAUCAAAAUCGGUGAAU** | chr14:22547576-22547595 |
| **25** | **UCUCCGAGAGCCCGUAGAAC** | chr7:142791989-142792008 |
| | | chr7:142801336-142801355 |
| **26** | **CAAACACAGCGACCUCGGGU** | chr7:142791716-142791735 |
| **27** | **UGACAGCGGAAGUGGUUGCG** | chr7:142791963-142791982 |
| | | chr7:142801310-142801329 |

**Tim-3 gene Gene ID: 84868** (NC_000005.10, GRCh38.p13 primary assembly)
   *Exon2* 
5’-
GGTCCTCAGAAGTGGAATACAGAGCGGAGGTCGGTCAGAATGCCTATCTGCCCTGC
TTCTACACCCCAGCCGCCCCAGGGAACCTCGTGCCCGTCTGCTGGGGCAAAGGAGC
CTGTCCTGTGTTTGAATGTGGCAACGTGGTGCTCAGGACTGATGAAAGGGATGTGA
ATTATTGGACATCCAGATACTGGCTAAATGGGGATTTCCGCAAAGGAGATGTGTCC
CTGACCATAGAGAATGTGACTCTAGCAGACAGTGGGATCTACTGCTGCCGGATCCA
AATCCCAGGCATAATGAATGATGAAAAATTTAACCTGAAGTTGGTCATCAAACCAG
-3’ (SEQ ID NO:101)
   *Exon 3* 
5’ -
CCAAGGTCACCCCTGCACCGACTCGGCAGAGAGACTTCACTGCAGCCTTTCCAAGG
ATGCTTACCACCAGGGGACATGGCCCAGGTAACTATGCATGGGGCAGAGGAGGG -
3’(SEQ ID NO:102)
**LAG-3 gene** Gene ID: 3902 (NC_000012.12, GRCh38.p13)
   *Exon3* 
5’ -
TGGCCCGCCCGCTGCCGCCCCCGGCCATCCCCTGGCCCCCGGCCCTCACCCGGCGG
CGCCCTCCTCCTGGGGGCCCAGGCCCCGCCGCTACACGGTGCTGAGCGTGGGTCCC
GGAGGCCTGCGCAGCGGGAGGCTGCCCCTGCAGCCCCGCGTCCAGCTGGATGAGC
GCGGCCGGCAGCGCGGGGACTTCTCGCTATGGCTGCGCCCAGCCCGGCGCGCGGA
CGCCGGCGAGTACCGCGCCGCGGTGCACCTCAGGGACCGCGCCCTCTCCTGCCGCC
TCCGTCTGCGCCTGGGCCAGGCCTCGA - 3’(SEQ ID NO:103)
   *Exon1* 
5’-
ACAGGGGTGAAGGCCCAGAGACCAGCAGAACGGCATCCCAGCCACGACGGCCACT
 
TTGCTCTGTCTGCTCTCCGCCACGGCCCTGCTCTGTTCCCTGGGACACCCCCGCCCC
CACCTCCTCAGGCTGCCTGATCTGCCCAGCTTTCCAGCTTTCCTCTGGATTCCGGCC
TCTGGTCATCCCTCCCCACCCTCTCTCCAAGGCCCTCTCCTGGTCTCCCTTCTTCTAG
AACCCCTTCCTCCACCTCCCTCTCTGCAGAACTTCTCCTTTACCCCCCACCCCCCAC
CACTGCCCCCTTTCCTTTTCTGACCTCCTTTTGGAGGGCTCAGCGCTGCCCAGACCA
TAGGAGAGATGTGGGAGGCTCAGTTCCTGGGCTTGCTGTTTCTGCAGCCGCTTTGG
GTGGCTCCAG-3'(SEQ ID NO:104)
**2B4 gene Gene ID:** 51744 (NC_000001.11, GRCh38.p13)
   *Exon 1* 
5’-
gatgccagggatcagctgaccatgtggttagcatctcgggagtgcctcttcagttacaaccaaacagcatacagacgaaggttgacagca
ttgcatggaagaagttgctgccctcacaaaatggatttcatcacatattgaagtgggagaatggctctttgccttccaatacttccaatgatag
attcagttttatagtcaagaacttgagtcttctcatcaaggcagctcagcagcaggacagtggcctctactgcctggaggtcaccagtatatc
tggaaaagttcagacagccacgttccaggtttttgtatttg -3’(SEQ ID NO:105)
   *Exon 2* 
5’ -
ataaagttgagaaaccccgcctacaggggcaggggaagatcctggacagagggagatgccaagtggctctgtcttgcttggtctccagg
gatggcaatgtgtcctatgcttggtacagagggagcaagctgatccagacagcagggaacctcacctacctggacgaggaggttgacat
taatggcactcacacatatacctgcaatgtcagcaatcctgttagctgggaaagccacaccctgaatctcactcaggactgtcagaatgccc
atcagg -3’(SEQ ID NO:106)
**PD-1 gene Gene ID:** 5133 (NC_000002.12, GRCh38.p13)
   *Exon1* 
5’ –
agtttcccttccgctcacctccgcctgagcagtggagaaggcggcactctggtggggctgctccaggcatgcagatcccacaggcgccc
tggccagtcgtctgggcggtgctacaactgggctggcggccaggatggttcttag -3’(SEQ ID NO:107)
   *Exon 2* 
5’ -
actccccagacaggccctggaacccccccaccttctccccagccctgctcgtggtgaccgaaggggacaacgccaccttcacctgcag
cttctccaacacatcggagagcttcgtgctaaactggtaccgcatgagccccagcaaccagacggacaagctggccgccttccccgagg
accgcagccagcccggccaggactgccgcttccgtgtcacacaactgcccaacgggcgtgacttccacatgagcgtggtcagggccc
ggcgcaatgacagcggcacctacctctgtggggccatctccctggcccccaaggcgcagatcaaagagagcctgcgggcagagctca
gggtgacag -3’(SEQ ID NO:108)
**CD39 gene Gene ID: 953** (NC_000010.11, GRCh38.p13)
   *Exon2* 
5’ –
agtctaacgtgaagacattttgctccaagaatatcctagccatccttggcttctcctctatcatagctgtgatagctttgcttgctgtggggttga
cccagaacaaagcattgccagaaaacgttaag -3’ (SEQ ID NO:109)
**TRAC gene Gene ID: 28755** (NC_000014.9, GRCh38.p13)
   Exon1 
5’ -
atatccagaaccctgaccctgccgtgtaccagctgagagactctaaatccagtgacaagtctgtctgcctattcaccgattttgattctcaaac
aaatgtgtcacaaagtaaggattctgatgtgtatatcacagacaaaactgtgctagacatgaggtctatggacttcaagagcaacagtgctg
tggcctggagcaacaaatctgactttgcatgtgcaaacgccttcaacaacagcattattccagaagacaccttcttccccagcccag -
3’(SEQ ID NO:110)
**TRBC1/TRBC2 gene Gene ID: 6957** (NC_000007.14, GRCh38.p13)
   *A shared common region of exon1 between TRBC1 and TRBC2*

5’ -
GTGTTCCCACCCGAGGTCGCTGTGTTTGAGCCATCAGAAGCAGAGATCTCCCACAC
CCAAAAGGCCACACTGGTGTGCCTGGCCACAGGCTTCTTCCCTGACCACGTGGAGC
TGAGCTGGTGGGTGAATGGGAAGGAGGTGCACAGTGGGGTCAGCACGGACCCGCA
GCCCCTCAAGGAGCAGCCCGCCCTCAATGACTCCAGATACTGCCTGAGCAGCCGCC
TGAGGGTCTCGGCCACCTTCTGGCAGAACCCCCGCAACCACTTCCGCTGTCAAGTC
CAGTTCTACGGGCTCTCGGAGAATGACGAGTGGACCCAGGATAGGGCCAAACCC -
3’(SEQ ID NO:111)

### Table 4

**Table 4. In the table, the "gRNA sequence+PAM" indicates the sequence on the genomic DNA corresponding to the guide sequence of the gRNA and the PAM sequence. Corresponding guide sequences are reported in Table 5.**

| **Target site** | **gRNA ID (name of the guide and the target gene)** | **gRNA sequence+PAM sequence** | **SEQ ID NO:** |
|---|---|---|---|
| HAVCR2_exon 2 | g4_Tim-3 | GAGTCACATTCTCTATGGTC**AGG** | 28 |
| HAVCR2_exon 2 | g1_Tim-3 | CTAAATGGGGATTTCCGCAA**AGG** | 29 |
| HAVCR2_exon 2 | g3_Tim-3 | ATCCCCATTTAGCCAGTATC**TGG** | 30 |
| LAG-3_exon3 | g3_LAG-3 | CACCGCGGCGCGGTACTCGC**CGG** | 31 |
| 2B4_exon 2 | g9_2B4 | agttgagaaaccccgcctac**agg** | 32 |
| TRAC_exon 1 | g1_TRAC | gagaatcaaaatcggtgaat**agg** | 33 |
| TRBC1/2 | g2_TRBC1/2 | CAAACACAGCGACCTCGGGT**GGG** | 34 |
| HAVC2_exon 2 | g2_Tim-3 | AATGTGGCAACGTGGTGCTC**AGG** | 35 |
| HAVCR2_exon 3 | g5_Tim-3 | CTCTCTGCCGAGTCGGTGCA**GGG** | 36 |
| HAVCR2_exon 3 | g6_Tim-3 | GTGAAGTCTCTCTGCCGAGT**CGG** | 37 |
| HAVCR2_exon 3 | g7_Tim-3 | TGCCCCATGCATAGTTACCT**GGG** | 38 |
| HAVCR2_exon 3 | g8_Tim-3 | AGGTCACCCCTGCACCGACT**CGG** | 39 |
| HAVCR2_exon 3 | g9_Tim-3 | TGGCCCAGGTAACTATGCAT**GGG** | 40 |
| HAVCR2_exon 3 | g10_Tim-3 | CCAAGGATGCTTACCACCAG**GGG** | 41 |
| Lag3_exon 1 | g10_LAG-3 | GCAAAGTGGCCGTCGTGGCT**GGG** | 42 |
| Lag3_exon 1 | g16_LAG-3 | ACAGAGCAAAGTGGCCGTCG**TGG** | 43 |
| Lag3_exon 3 | g18_LAG-3 | GCTCAGCACCGTGTAGCGGC**GGG** | 44 |
| Lag3_exon 1 | g33_LAG-3 | GAACGGCATCCCAGCCACGA**CGG** | 45 |
| 2B4_exon 1 | g1_2B4 | accttcgtctgtatgctgtt**tgg** | 46 |
| 2B4_exon 1 | g2_2B4 | accaaacagcatacagacga**agg** | 47 |
| 2B4_exon 1 | g3_2B4 | ctcccgagatgctaaccaca**tgg** | 48 |
| 2B4_exon 2 | g10_2B4 | gttgagaaaccccgcctaca**ggg** | 49 |
| 2B4_exon 2 | g11_2B4 | ttgagaaaccccgcctacag**ggg** | 50 |
| CD39_exon 2 | g4_CD39 | AAGGATGGCTAGGATATTCT**TGG** | 51 |
| CD39_exon 2 | g5_CD39 | AAGAATATCCTAGCCATCCT**TGG** | 52 |
| TRBC1/2_exon 1 | g1_TRBC1/2 | TCTCCGAGAGCCCGTAGAAC**TGG** | 53 |
| TRBC1/2_exon 1 | g3_TRBC1/2 | TGACAGCGGAAGTGGTTGCG**GGG** | 54 |
| PD-1_exon 1 | g1_PD-1 | TGTAGCACCGCCCAGACGAC**TGG** | 55 |
| PD-1_exon 1 | g2_PD-1 | CGTCTGGGCGGTGCTACAAC**TGG** | 56 |
| PD-1_exon 2 | g3_PD-1 | AGGTGCCGCTGTCATTGCGC**CGG** | 57 |
| PD-1_exon 2 | g4_PD-1 | CCTGCTCGTGGTGACCGAAG**GGG** | 58 |

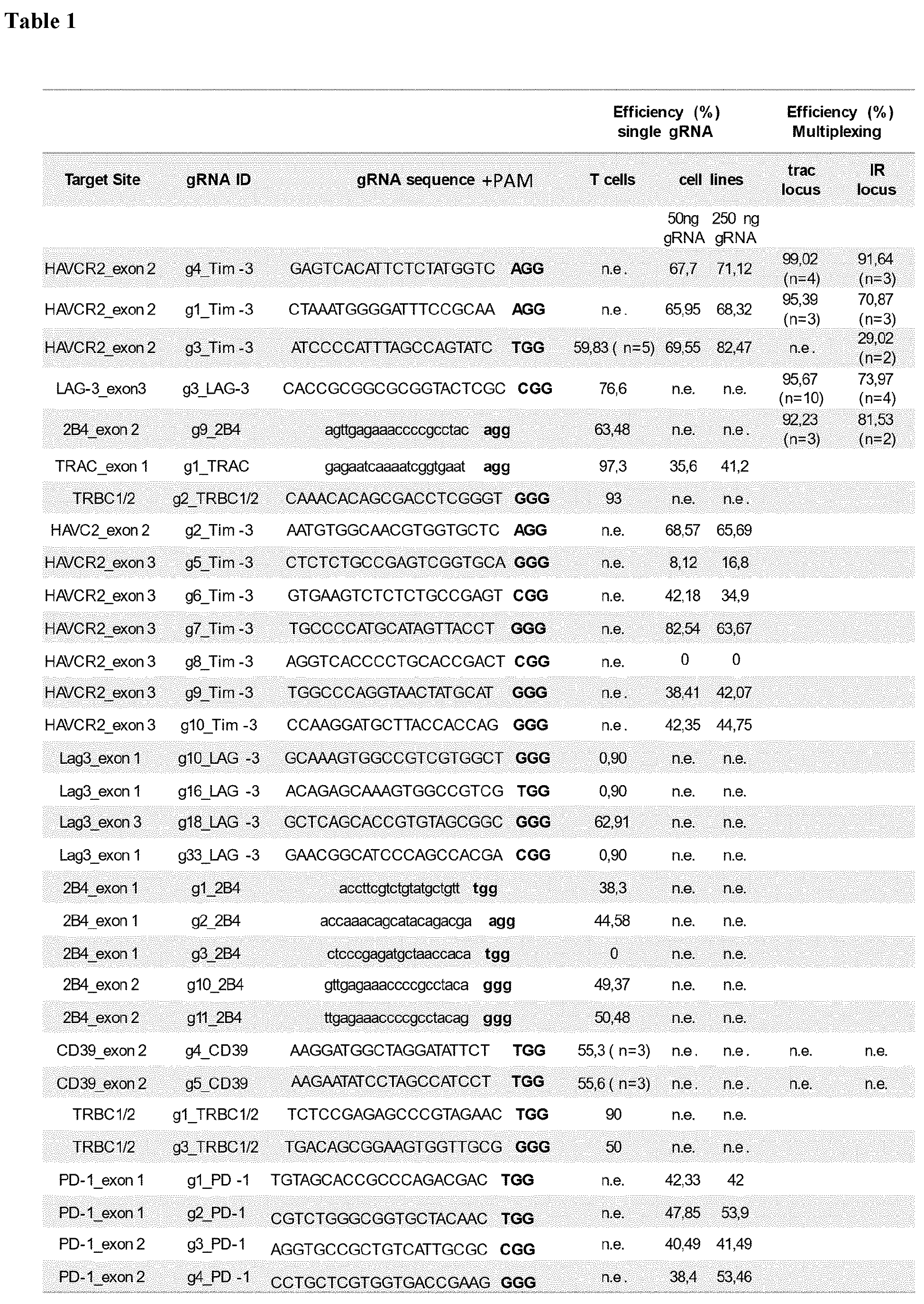

**Table 1. Sequences and score of designed gRNAs targeting Tim-3, LAG-3, PD-1, 2B4, TRAC and TRBCIn** the table the "gRNA sequence+PAM" indicates the sequence on the genomic DNA corresponding to the guide sequence of the gRNA and the PAM sequence. For each guide, the targeting efficiency (measured as % of Non-Homologous-End-Joining, NHEJ) at each target locus is shown. The targeting efficiency of each gRNA in primary T cells or in cell lines is shown. The evaluation of targeting efficiency in multiplexing has been performed in primary T cells. For some gRNAs, the NHEJ frequency is shown as mean of different biological replicates (indicated in brackets). The PAM sequence is indicated in bold.

### Examples

### Example 1

### MATERIALS AND METHODS

### Cell cultures

### Cell lines

K562-iCas9 and Jurkat cell lines were grown in Iscove's Modified Dulbecco's Medium (IMDM, Lonza) and Roswell Park Memorial Institute 1640 Medium (RPMI, Lonza) respectively, supplemented with 10% FBS (fetal bovine serum, BioWhittaker), 1% penicillin/streptomycin (Lonza) and 1% glutamine (Lonza).

Myeloma cell lines MM.1s and U266 were grown with RPMI 1640 supplemented with 10% FBS, 1% penicillin/streptomycin and 1% glutamine (Lonza). Adherent MM.1s were detached using TrypLE Express enzyme (Gibco).

MM1.s were transduced with 3 different lentiviral vectors encoding for HLA-A2, NY-ESO-1 antigen and luciferase. Transduced cells were FACS-sorted to obtain a pure population of HLA-A2^{pos} NY-ESO-1^{pos} luc^{pos} cells with a purity > 98%).

Cells were counted every 3-4 days by Trypan blue dye exclusion and plated at a concentration of 0.5-1 x 10⁶ cells/mL. Cells were incubated at 37°C 5% CO2 in a humidified cell culture incubator.

### Primary T isolation and expansion

Peripheral blood mononuclear cells (PBMCs) were collected from healthy donors, after written informed consent, according to the San Raffaele Scientific Institutional Ethical Committee guidelines. PBMCs were isolated by Ficoll-Hypaque gradient separation (Lymphoprep; Fresenius) and were freshly used. Lymphocytes were enriched and stimulated using anti-CD3/CD28-conjugated magnetic beads in a 3:1 bead/T cell ratio for 6 days. After 6 days of stimulation, we magnetically removed beads, and cells were cultured in X-vivo 15 medium (Lonza) supplemented with 5% FBS (fetal bovine serum, BioWhittaker), 1% penicillin/streptomycin (Lonza) and glutamine (Lonza). T cells were cultured in the presence of IL-7 and IL-15 (5 ng/ml; PeproTech) to preserve early differentiated stem cell memory and central memory phenotype, as previously reported. Medium was replaced every 3-4 days and cells were counted by Trypan blue dye exclusion. Cells were incubated at 37°C 5% CO2 in a humidified cell culture incubator.

### gRNAs screening in cell lines

Guide-RNAs targeting the coding regions of Tim-3, LAG-3, PD-1, Tim-3, 2B4 and TCR alpha and beta chain were designed using the online optimized design tool at http://crispr.mit.edu, developed by Dr. Zhang Feng laboratory at MIT. Guide-RNAs targeting CD39 were designed using CRISPR Design Tools developed by Synthego (https://www.synthego.com/products/bioinformatics/crispr-design-tool). Each gRNA was cloned into a plasmid under the control of the human U6 promoter to drive gRNAs expression

To clone sgRNAs into the vector, we annealed forward and reverse oligonucleotides at 95°C for 6 minutes. List of oligonucleotides used are summarized in table 2.

**Table 2**

| sgRNA | **Oligonucleotide Fw** | **SE Q ID Nos** | **Oligonucleotide Rev** | **SE Q ID Nos** |
|---|---|---|---|---|
| PD-1 g1 | ACCGGTAGCACCGCCCAG ACGAC | **100** | AAACGTCGTCTGGCGGTGC TAC | **74** |
| PD-1 g2 | ACCGGTCTGGGCGGTGCT ACAAC | **59** | AAACGTTGTAGCACCGCCCA GAC | **75** |
| PD-1 g3 | ACCGGGTGCCGCTGTCAT TGCGC | **60** | AAACGCGCAATGACAGCGG CACC | **76** |
| PD-1 g4 | ACCGCTGCTCGTGGTGAC CGAAG | **61** | AAACCTTCGGTCACCACGAG CAG | **77** |
| PD-1 g5 | ACCGGCAGTTGTGTGACA CGGAAG | **62** | AAACGAAGGCACAGTGTGTT GACG | **78** |
| Tim-3 g1 | ACCGTAAATGGGGATTTC CGCAA | **63** | AAACTTGCGGAAATCCCCAT TTA | **79** |
| Tim-3 g2 | ACCGATGTGGCAACGTGG TGCTC | **64** | AAACGAGCACCACGTTGCCA CAT | **80** |
| Tim-3 g3 | ACCGTCCCCATTTAGCCA GTATC | **65** | AAACGATACTGGCTAAATGG GGA | **81** |
| Tim-3 g4 | ACCGAGTCACATTCTCTA TGGTC | **66** | AAACGACCATAGAGAATGTG ACT | **82** |
| Tim-3 g5 | ACCGTCTCTGCCGAGTCG GTGCA | **67** | AAACTGCACCGACTCGGCAG AGA | **83** |
| Tim-3 g6 | ACCGTGAAGTCTCTCTGC CGAGT | **68** | AAACACTCGGCAGAGAGACT TCA | **84** |
| Tim-3 g7 | ACCGGCCCCATGCATAGT TACCT | **69** | AAACAGGTAACTATGCATGG GGC | **85** |
| Tim-3 g8 | ACCGGGTCACCCCTGCAC CGACT | **70** | AAACAGTCGGTGCAGGGGTG ACC | **86** |
| Tim-3 g9 | ACCGGGCCCAGGTAACTA TGCAT | **71** | AAACATGCATAGTTACCTGG GCC | **87** |
| Tim-3 g10 | ACCGCAAGGATGCTTACC ACCAG | **72** | AAACCTGGTGGTAAGCATCC TTG | **88** |
| TRAC g1 | ACCGAGAAATCAAAATCG GTGAAT | **73** | AAACATTCACCGATTTTGAT TTCT | **89** |

The annealed products and linearized backbone were incubated with T4 ligase. Top10 competent bacteria (Invitrogen) was used to amplify ligated products. Briefly, Top10 competent bacteria were transformed with ligated plasmids and plated on Lourie Bertani (LB, Sigma-Aldrich)-Agar plates. Colonies were then growth in liquid LB cultures and plasmidic DNA was extracted using Promega Wizard^{®} plus SV Minipreps DNA kit, according to manufacturer instructions. The plasmids' sequences were checked by Sanger sequencing to identify colonies encoding for in frame gRNAs inserted in the plasmid.

gRNAs cloned into B3_E7 backbone were electroporated in 0,45 * 10⁶ K562-iCas9 cells using Nucleofector 4D (Lonza) and SF Cell Line 4D-Nucleofector^{®} X Kit (Lonza), according to manufacturer's instructions. Cas9 expression was activated into K562-iCas9 cells through doxycycline administration (5 mg/ml) 24 hours before the electroporation. Jurkat cells were electroporated with both the plasmid encoding for Cas9 and the plasmid encoding for the sgRNA.

### Gene modification in primary T cells

### Ribonucleoproteins (RNPs) assembly with in vitro transcribed (IVT) sgRNAs

sgRNAs were synthesized *in vitro* using GeneArt Precision gRNA Synthesis Kit (ThermoFisher Scientific) according to manufacturer's instructions. Briefly, forward and reverse oligonucleotide sequences were designed to constitute a sgRNA DNA template, containing the T7 promoter, the sequence encoding for the target-specific gRNA and the constant region of crRNA/tracrRNA. Precision gRNA Synthesis Kit can be used to generate a gRNA DNA template containing a T7 promoter by PCR. Subsequently, *in vitro* transcription (IVT) of the gRNA template followed by spin column purification is performed to obtain sgRNAs and residual DNA template was removed by DNase I digestion. sgRNAs were capped using 3'-O-Me-m7G(5')ppp(5')G RNA Cap Structure Analog (ARCA; NEB) and a dephosphorylation step was added in order to reduce the toxicity of the sgRNAs. sgRNAs concentrations were measured using the Qubit RNA HS Assay Kit by Qubit^{™} 3.0 fluorometer, according to manufacturer's instructions (ThermoFisher Scientific).

Ribonucleoproteic complex (RNP) were pre-assembled mixing *in vitro* transcribed sgRNAs and the Cas9 (Alt-R^{®} Cas9 enzyme, 61 µM stock, IDT) in a 1:1 ratio (40 pmol) for 20 minutes at room temperature.

### Ribonucleoproteins (RNPs) assembly with commercial gRNAs

Commercial crRNA (Alt-R^{®} CRISPR-Cas9 crRNA, IDT) and tracrRNA (Alt-R^{®} CRISPR-Cas9 tracrRNA, IDT) were purchased by Integrated DNA technology (IDT). crRNA:tracrRNA were mixed in equimolar concentrations and heated at 95°C for 5 min to form a duplex with a final 100 µM concentration. crRNA:tracrRNA duplex and Cas9 enzyme were assembled in a 120:104 pmol ratio for 20 minutes at RT, according to manufacturer's instructions. For reactions using commercial sgRNA plus enhancer, 1 uL of Enhancer (100 uM; Alt-R^{®} Electroporation Enhancer, IDT) was added to the reaction. Commercial sgRNA targeting CD39 were purchased from Synthego. sgRNAs and Cas9 enzyme (Synthego) were assembled in a or 100:20 or 180:20 pmol ratio for 10 minutes at RT.

### IR disruption and TCR redirection in primary T cells

Stimulated human CD3+ cells were electroporated 48 hours after stimulation. Two million cells / condition were resuspended in P3 primary buffer (Lonza) and assembled RNPs targeting one or more coding sequences of selected genes were simultaneously added to the cell suspension. Cells were electroporated using Nucleofector 4D (Lonza) according to manufacturer's instructions. 24 hours later, electroporated cells were counted and transduced with previously validated lentiviral vector encoding for HLA-A2 restricted NY-ESO-1 specific TCR. The expression of the exogenous TCR was evaluated by flow cytometry through anti-Vβ13.1 antibody (FITC, Beckman Coulter) or with APC-conjugated dextramer (Immudex).

### Non-Homologous End Joining evaluation

### DNA extraction

Targeted cell lines or T lymphocytes were collected overtime to evaluate the frequency of NHEJ at each targeted genomic locus. DNA was extracted through QIAamp DNA Mini or Micro kits (Qiagen), depending on the number of cells and according to manufacturer's instructions. DNA was eluted in Buffer AE (10 mM Tris-Cl, 0,5 mM EDTA, ph 9.0), quantified by Nanodrop and stored at -20°C until use.

### Surveyor assay

Surveyor^{®} Mutation Detection Kits (IDT) was performed according to manufacturer's instructions to detect NHEJ frequencies. Specifically, validated primer pairs were used to amplify by PCR a fragment of approximately 400bp of our edited gene of interest. The following primer pairs were used.

**Table 3**

| | | | |
|---|---|---|---|
| PD-1 | Exon 1 | Fw: AGTTTCCCTTCCGCTCAC | SEQ ID NO.90 |
| | | Rev: CCTGACCCGTCATTCTACAG | SEQ ID NO.91 |
| | Exon 2 | Fw: GAGGGATGTGAGCAGGTG | SEQ ID NO.92 |
| | | Rev: GCATCTCTGTCCTCTAGCTC | SEQ ID NO.93 |
| Tim-3 | Exon 2 | Fw: GCCTATCTGCCCTGCTTCTA | SEQ ID NO.94 |
| | | Rev: TTACAGGCTCCCGTAACCAT | SEQ ID NO.95 |
| | Exon 3 | Fw: TATCCAGGCAGGTTTGGAAG | SEQ ID NO.96 |
| | | Rev: ATCCAATGTGTCAGTTCCCA | SEQ ID NO.97 |
| TRAC | Exon 1 | Fw: GGGC AAAGAGGGAAAT GAGA | SEQ ID NO.98 |
| | | Rev: CAATGGATAAGGCCGAGACC | SEQ ID NO.99 |

After genomic amplifications, PCR product were denaturated and annealed in order to form heteroduplexes containing a mismatch at the point of mutations. This mispairing will be recognized by the surveyor nuclease, which will cleave the heteroduplex and generate fragment of different length. The editing frequency, at genomic level, was evaluated analyzing fragments lengths and amounts through Agilent 2100 Bioanalyzer (Agilent Technologies) at Center for Translational Genomics and Informatics in our Institute, according to manufacturer's instruction. The NHEJ percentage was evaluated as follows: [(1st fragment + 2nd fragment) / (wild-type fragment + 1st fragment + 2nd fragment)]* 100. All the terms of the expression are expressed as pg/uL

### Droplet digital PCR (ddPCR)

NHEJ assessment in IR_{NEG} TCR redirected cells was performed using ddPCR NHEJ Genome Edit Detection Assays (Bio-Rad) using custom assays to amplify Tim-3, LAG-3, 2B4, TRAC or TRBC1/2 targeted loci, according to manufacturer's instructions. Briefly, 10 ng of DNA per well were used with a total of 20 uL of reaction mix, containing primers and probes. Auto-DG (Biorad) was used to automatically perform oil droplets generation, according to manufacturer's instructions. After droplet generation, plates were sealed and PCR were performed with the following thermal cycling conditions: 10 minutes of enzyme activation at 95°C, 30 seconds of denaturation at 94°C, 3 minutes of annealing/extension at 62°C, 10 minutes of enzyme deactivation at 98°C. Denaturation and annealing/extension were repeated for 40 cycles with a ramp rate of 2°C/second. Data were acquired using QuantaSoft Software and analyzed QuantaSoft Analysis Pro Software.

### ICE analysis

DNA was extracted from edited and control cells and amplified by PCR the target sequence including the cut site. The PCR product was run on a 1% agarose gel to verify the presence of a single fragment of the correct size. The PCR-purified product was submitted for Sanger Sequencing, and sequences were run on the Inference of CRISPR Editing (ICE) algorithm (Synthego) to assess the effect of CD39 KO at the genetic level.

### Functional in vitro assays

### IR ligands evaluation on tumor cell lines

Galectin-9 and HMGB1 secretions in cell culture medium were quantified using Galectin-9 ELISA kit (RayBiotech) or HMGB1 ELISA kit (IBL International) according to manufacturer's instructions. CD48 and HLA-DR expressions were quantified using fluorochrome conjugated monoclonal antibodies (anti-CD48 APC-Vio 770 Miltenyi; anti-HLA-DR PE Biolegend). U266 and MM1.s cells were stimulated with IFNγ (600 UI/mL) or left unstimulated and IR ligands were analyzed 48 hours after the stimulation.

### Antigen-specific killing assay

Target cells (T) (either U266 or MM1.s) were co-cultured with effector (E) SE-IR_{KO} cells or SE-IR_{COMP} cells at decreasing E:T ratio. Wild type MM.1s cell line, which is HLA-A2 negative cell line and does not express NY-ESO-1, and untransduced T lymphocytes were used as controls. After three days, cells were analyzed by flow cytometry. U266 cells were selected as CD138+, HLA-A2^{pos} NY-ESO-1^{pos} MM1.s were selected as GFP positive cells, whereas wild type MM.1s are CD38+. Number of residual U266, MM.1s or effector cells were counted according to the number of cells acquired at flow cytometry and the total well volume.

Elimination index for anti-tumor activity evaluation was calculated as follows: 1 - (number of residual target cells in presence of redirected T cells/number of residual target cells in presence of untransduced T cells).

In chronic stimulation experiments, effector cells were stimulated daily with either HLA-A2^{pos} NY-ESO-1^{pos}MM1.s or U266 cells. After 15-21 days, effector cells were restimulated with target cells for 6-hours in a 1:1 effector to target (E:T) ratio and CD107a+ CD3+ T cells were quantified by flow cytometry. Medium and HLA-A2^{neg} NY-ESO-1^{neg} MM1.s cells were used as negative controls. The stimulation with 50 ng/ml PMA (Sigma-Aldrich) and 1mg/mL ionomycin (Sigma-Aldrich) was used as positive control.

### Quantification of cytokines production in antigen-specific stimulation

Cytokines production were analyzed upon antigen-specific stimulation, as described above. 24 hours upon antigen-specific challenge, supernatants of effector:target ratio of 1:10 conditions were collected and stored al -20°C until analysis. A panel of 13 different cytokines was analyzed using LEGENDplex human Th panel (13-plex) (Biolegend) according to manufacturer's instruction. Briefly, 20uL of supernatant was analyzed to absolutely quantify IL-2, IL-4, IL-5, IL-6, IL-9, IL-10, IL-13, IL-17A, IL-17F, IL-21, IL-22, IFN-γ and TNF-α in the medium.

Samples and standard tubes were acquired by flow cytometry. Data were analyzed using LEGENDplex data analysis software (Vigentech).

### In vivo models of multiple myeloma

The protocol was approved by the Institutional Animal Care and Use Committee (IACUC). At day 0, 6-8 weeks old NOD/Scid mice (Charles-River Italia) received sub-lethal total body irradiation and were infused intravenously with 10x10⁶ luciferase⁺ U266 cell line. For MM1.s tumor model, 6-8 weeks old NOD/Scid mice (Charles-River Italia) were infused intravenously with 2x10⁶ HLA-A2^{pos}NY-ESO-1^{Pos} luc^{pos}. Mice were clinically monitored and weighed 3 times per week. Tumor growth was monitored by total body bioluminescent imaging. Briefly, bioluminescence imaging (BLI) was performed with IVIS SpectrumCT System. Mice were infected intraperitoneally with 150mg luciferin/kg 10 minutes before BLI. BLI image analysis was performed by measuring the total flux (photons/seconds) within regions with lesions. Images were acquired and analyzed with Living Image 4.4.

Upon specific tumor engraftment (according to the experimental settings), mice were treated with different doses of SE-IR_{KO} cells, SE-IR_{COMP} T cells or unmanipulated cells. Human chimerism and activation of T cells on peripheral blood were assessed weekly by flow cytometry. Mice were weekly monitored and in the presence of limping or weight loss greater than 5% were sacrificed. At sacrifice, spleen and bone marrow were harvested and extensive phenotypic, activation and exhaustion analysis of human T cell was performed by flow cytometry. In U266 re-challenge mouse model, 10x10⁶ U266 were infused intravenously in mice with a tumor burden lower than 10⁶ photons/sec.

### Multiparametric flow cytometry and intracellular staining experiments

For experiments described in figure 1 and 5, cells were labeled with fluorescent antibodies specific for CD3 (Biolegend), CD4 (Biolegend), CD8 (BD), Tim-3 (Miltenyi), Lag-3 (Miltenyi), PD-1 (Biolegend), BTLA (Biolegend), 2B4 (Biolegend), TIGIT (eBioscience, PE/Cy7), CD39 (Biolegend)).

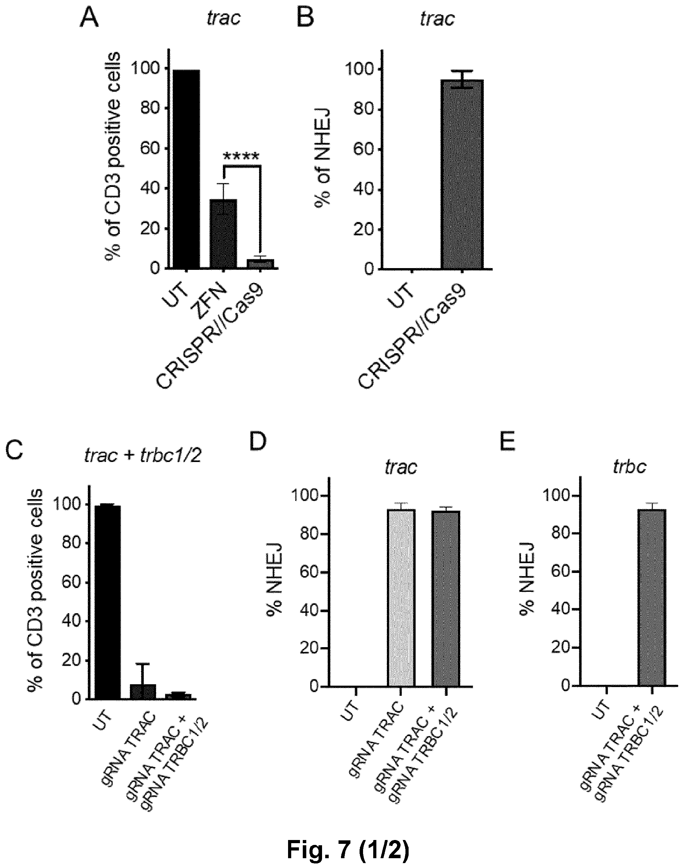

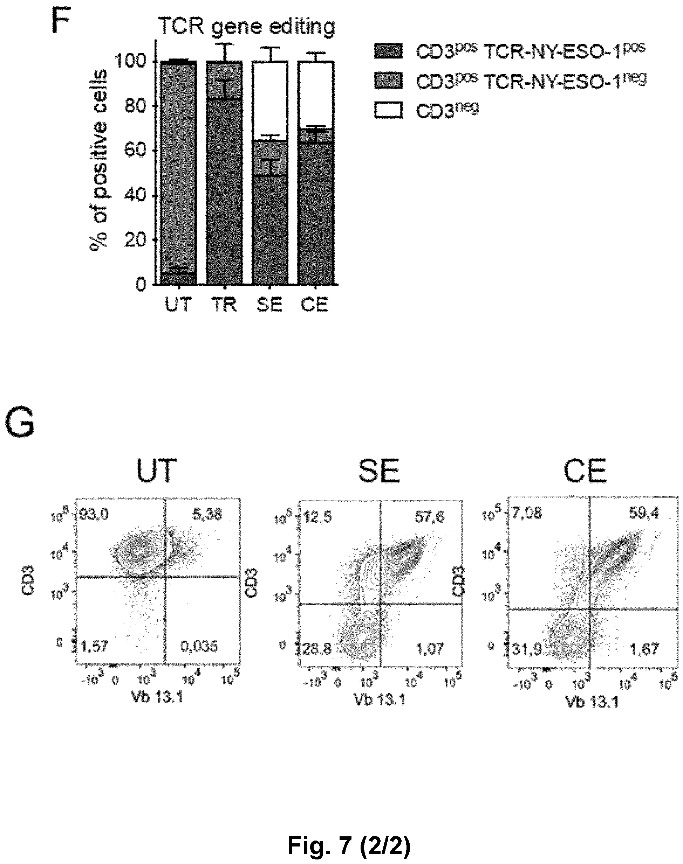

For the experiments in figures 7-13, cells were stained with fluorochrome conjugated monoclonal antibodies specific for CD3 (Biolegend), CD8 (Biolegend), CD4 (Biolegend), CD45RA (Biolegend), CD62L (Biolegend), PD-1 (Biolegend), CTLA-4 (Biolegend), TIGIT (Biolegend), Tim-3 (Miltenyi), LAG-3 (Miltenyi), 2B4 (Biolegend), CD138 (BeckmanCoulter), CD38 (Biolegend), HLA-DR (Biolegend). T cells redirected against NY-ESO-1 antigen were detected using FITC conjugated anti-Vb13.1 specific antibody (Beckman Coulter) or with HLA-A*0201-restricted NY-ESO-I_{SLLMWITQV}-specific dextramers (APC, Immudex).

For intracellular staining, anti-CD107a antibody (eBioscience) was added to cell culture. Cells were stained with surface antibodies, washed and fixed and permeabilized with FIX & PERM Cell Fixation & Permeabilization Kit (ThermoFisher), according to manufacturer's instructions. For *in vivo* experiments, 50 uL of whole blood were stained with fluorochrome conjugated monoclonal antibody specific for mouseCD45 (Biolegend), humanCD45 (Biolegend), 2B4 (Biolegend), CD3 (Biolegend), CD8 (Biolegend), CD45RA (Biolegend), CD62L (Biolegend), CTLA-4 (Biolegend), PD-1 (Biolegend), Tim-3 (Miltenyi), LAG-3 (Milyenyi), HLA-DR (BD Bioscience). After surface staining, red blood cells were lysed with ACK (Ammonium-Chloride-Potassium) buffer for 10 minutes at room temperature. Human T cell were counted on peripheral blood using Flow Count fluorescent beads (Beckman Coulter), according to manufacturer's instruction.

For each experiment, dead cells were excluded by DAPI positive staining. Data were acquired using a BD FACS Canto II, LSR Fortessa or Symphony (BD Biosciences) and analyzed with FlowJo version 10 software (TreeStar). Data were processed using FlowJo version 9.6.3 (TreeStar).

### High-dimensional flow cytometry analysis

Unbiased high-dimensional analysis was performed using cytoChain algorithm (Manfredi, Abbati et al under revision). Briefly, each sample was cleaned using flow-iQC package of FlowAI.Total cleaned events were scaled using arcSinh function (inverse hyperbolic sine) with fixed cofactor at 150 or with FlowVS. The samples from were concatenated in a single flowFrame and FlowSOM algorithm was employed for hierarchical clustering. The heatmaps were built by means of a hierarchical cluster analysis *(hclust* of the stats R package) on a set of dissimilarities calculated with *dist* (stats package with an Euclidean method setting).

### Statistical analysis

Statistical analyses were performed with Prism 8 (GraphPad Software). Mann-Whitney test was used when comparing two independent groups. Two-way ANOVA was used when comparing across two or more different subsets. For all comparisons, two-sided P values were used, and P < 0.05 was considered statistically significant.

### TCR gene editing in regulatory T cells

Human regulatory T cells (Tregs) and conventional T cells (Tconv) were separated from PBMCs as CD4⁺CD25⁺ and CD4⁺CD25⁻, respectively. Tregs were stimulated with aCD3/aCD28 beads and cultured in the presence of IL-2; Tconv were stimulated with aCD3/aCD28 beads (Life Technologies) and cultured in the presence of IL7/IL15. Two days after stimulation, Tregs and Tconv were electroporated with RNP complexes for TRAC disruption. *TRAC*-disrupted Tregs (KO-Tregs) and *TRAC*-disrupted Tconv (KO-Tconv) were transduced with a lentiviral vector (LV) encoding for the HLA-A*02:01-restricted NY-ESO-1 specific TCR 12 and 24 hours after electroporation, respectively. On day 6 beads were detached from Tconv and *TRAC* disruption efficiency was assessed on KO-Tregs and KO-Tconv by flow cytometry. At day 12 LV transduction efficiency was assessed by flow cytometry. aCD3/aCD28 beads were detached from Tregs on day 14. Edited Tregs (KO-Tregs transduced with the NY-ESO1-specific TCR) and untransduced (UT) Tregs were serially diluted and co-cultured with fixed numbers of edited Tconv (Tconv transduced with the NY-ESO1-specific TCR) and a HLA-A*02:01+ NY-ESO1+ GFP+ U266 target cell line for 72 hours. Prior to seeding, edited Tconv were stained with Cell Tracer (Thermofisher) to track their proliferation. Percentage of proliferating Tconv in the presence of UT-Treg or edited Tregs was assessed via flow cytometry. Elimination index by edited Tconv was assessed via flow cytometry and normalized on U266-GFP cell line cultured alone.

### RESULTS

In the last years, several evidences proved the superior ability of long-term persistence of early differentiated T_{SCM}/T_{CM} after vaccination or hematopoietic stem cell transplantation for the treatment of primary immunodeficiencies or hematological malignancies and/or adoptive T cell therapy with engineered T cells. Considering their unique characteristics of self-renewal and long-term persistence, T_{SCM} represent the ideal T cell subset to be used for adoptive T cell therapy. The present inventors contributed to the field developing a protocol for the *in vitro* generation and expansion of T_{SCM}. Since the present inventors envisage to employ T_{SCM} for their T cell product, they investigated the dynamics of IRs expression on primary T cells activated with this protocol. Briefly, primary T lymphocytes from healthy donors were enriched and activated with anti-CD3/anti-CD28-coated magnetic beads and cultured with low doses (5ng/ml each) of IL-7 and IL-15 to preserve an early differentiated memory phenotype. The expression of Tim-3, LAG-3, PD-1, CTLA-4, 2B4, TIGIT and BTLA was analyzed in resting conditions before stimulation and at different time points after polyclonal stimulation. The inventors separately analyzed the expression of inhibitory receptors on both CD4 and CD8 T cells.

The inventors observed two different patterns of IRs expression after T cell activation. Almost all activated T cells expressed Tim-3, LAG-3 and PD-1 in the first week after activation **(****Figure 1A****).** Of note, Tim-3 was the only IR with the most stable expression, as it continued to be expressed by almost 100% of CD8 T cells and 60% of CD4 T cells 17 days after activation, while LAG-3 and PD-1 expression rapidly decreased after the first week. On the contrary, CTLA-4, BTLA, 2B4 and TIGIT were expressed by a lower proportion of activated cells. Specifically, activated CD8⁺ T cells upregulated CTLA-4, but they expressed at very low levels BTLA, 2B4 and TIGIT. Contrariwise, CD4⁺ T cells did not express TIGIT, but they upregulated CTLA-4, BTLA and 2B4 **(****Figure 1A****).**

Considering the data obtained from the analysis of T cells infiltrating the bone marrow of AML patients relapsing after HSCT (Noviello, Manfredi et al., Nat. Comm. 2019; Toffalori et al, Nat. Med 2019) or infiltrating solid tumors (Canale et al., cancer res 2018) and considering the kinetics of IR-expression in T cells stimulated with our protocol to generate early differentiated T_{SCM} and T_{CM}-based cellular products, we decided to test the selective disruption of Tim-3, LAG-3, PD-1, 2B4 or CD39 either alone or in combination with a TCR gene editing protocol for the generation of tumor-specific T cells resistant to exhaustion signals.

### Screening of CRISPR/Cas9 molecules in cell lines allows the selection of gRNAs with high gene disruption efficiency.

### Design of sgRNAs

Since the inventors need to disrupt multiple genes simultaneously (the endogenous TCR and at least one inhibitory receptor) for the generation of TCR-edited tumor-specific T cells devoid of inhibitory molecules (SE-IR_{KO} T cells), they chose recently described CRISPR/Cas9 technology to perform gene disruptions. This technology harbors several advantages over other nucleases (such as Zinc Finger Nucleases or TALEN) already successfully employed for genome editing. Remarkably, their easy design coupled with the possibility to disrupt multiple genes in a single step of cell manipulation qualify this technology as the best approach for inventors' strategy.

The inventors designed a panel of gRNAs targeting a unique genomic sequence at Tim-3, LAG-3, PD-1, 2B4 and CD39 loci. Simultaneously, they designed gRNAs targeting the genomic sequence of both TCR α and β chain constant regions (TRAC and TRBC1/2, respectively). They used cripr.mit.edu online tool to design sgRNAs. The algorithm scanned the genomic coding sequence and identified all possible gRNAs, according to NGG position in the genome. The output from the algorithm was a list of all possible guides in the query sequence, ranked by fidelity of on-target activity computed as 100% minus a weighted sum of off-target hit-scores in the target genome. Off-target site were predicted considering total number of mismatches, mismatch absolute position and mean pairwise distance between mismatches. gRNAs with a score lower than 70 were excluded to minimize off-target sites. Sequences are summarized in tables 1, 4 and 5. Targeted genomic sites for each gene are summarized in figure 1B.

### Screening of CRISPR/Cas9 molecules in cell lines allows the selection of gRNAs with high gene disruption efficiency.

To assess the cut efficiency of each gRNA, inventors screened gRNAs in a K562 cell line expressing an inducible form of Cas9 (iCas9-K562). They initially screened gRNAs targeting PD-1, Tim-3 and TRAC genes. They cloned each gRNA into B3_E.7-Human U6_gRNA-mod_AMP vector under the control of U6 promoter. Then, they electroporated two different plasmid amounts (50ng and 250ng) into iCas9-K562 cells 24 hours after the induction of Cas9 expression. One week after electroporation, they determined Non-Homologous End Joining (NHEJ) percentage at targeted genomic sites by surveyor assay. All gRNAs targeting PD-1 displayed approximately 40% of NHEJ, except for g4 that displayed 53,46% NHEJ at the highest plasmid concentration **(****Figure 2A** **and Table 1).** Conversely, for Tim-3 disruption inventors identified two gRNAs with absent or very low NHEJ activity (g8 and g5, respectively). All the other gRNAs screened for Tim-3 disruption displayed at least 40% of genomic disruption, with g3 showing 82,47% of disruption at highest concentration **(****Figure 2B** **and Table 1).**

For TRAC disruption, inventors tested the designed gRNA both in iCas9-K562 and Jurkat cell lines. In iCas9-K562 cell line, the gRNA induced gene disruption in 41% cells. When tested in Jurkat cells, inventors also co-transfected a plasmid encoding for the Cas9 gene. In this case, they observed very low gene disruption at the TRAC locus **(****Figure 2C** **and Table 1)** and speculated that this could be derived from high toxicity caused by co-transfection of two plasmidic DNA simultaneously.

Inventors then tried to correlate the frequency of NHEJ obtained in this screening phase with the gRNA score obtained from the algorithm used for gRNA design. Interestingly, they noticed that gRNA scores did not correlate with cut efficiency **(****Figure 2****, D, E and F).**

Given the results collected in the screening phase using iCas9-K562 and Jurkat cell lines, g4 targeting PD-1, g3 targeting Tim-3 and g1 targeting TRAC were selected as the best performing ones. In addition, the targeting efficiency of all the designed gRNAs specific for LAG-3, Tim-3, 2B4, TRAC, TRBC1/2 and CD39 were screened in primary T cells.

### Different reagents for CRISPR/Cas9 ribonucleoprotein production display different editing efficiency in primary T cells

CRISPR/Cas9 molecules can be delivered to the cells with different strategies: (i) both sgRNA and Cas9 DNA cloned into viral vectors; this process is limited by high toxicity and by the permanent Cas9 integration into the genome which is not recommended for primary cells due to undesired off-target toxicity; (ii) Cas9 mRNA co-delivered together with the sgRNA; Cas9 activity would be transient and there will be no need for viral production, but free mRNA in lymphocytes causes high toxicity, and the time that Cas9 mRNA takes to be translated into a functional protein could be sufficient for sgRNA to be already degraded thus limiting the efficiency of the editing; (iii) Cas9 protein and gRNAs pre-assembled *in vitro* to form a ribonucleoproteic complex; this strategy allows for transient Cas9 activity, high efficiency and reduced toxicity. Considering pros and cons of all approaches, inventors decided to test ribonucleoprotein (RNP) for CRISPR/Cas9 delivery in primary T cells.

To select the optimal reagents based on high disruption efficiency in T cells, they tested different protocols for RNPs preparation. Specifically, they tested *in vitro* transcribed (IVT) gRNAs (unique RNA molecule encoding for crRNA and tracrRNA) and commercial chemically modified dual gRNAs (crRNA and tracrRNA splitted in two separated RNA fragments). In addition, inventors also tested commercial gRNAs in combination with the enhancer, a small negatively charged DNA molecule aiding RNPs entry into the cells. Inventors tested gRNAs targeting Tim-3 and TRAC and they observed that the three tested protocols displayed variable efficiencies. Commercial gRNAs in combination with enhancer provided the highest knock-out efficiency at both genomic loci compared to commercial gRNA used in the absence of the enhancer or to IVT gRNAs, reaching up to 98% of NHEJ at the TRAC locus. **(****Figure 3****).** Thus, they selected commercial gRNAs with the enhancer for subsequent experiments.

Interestingly, inventors noticed that gene disruption efficiencies were higher in T cells using RNPs compared to plasmids delivery used in the screening phase in K562-iCas9 and in Jurkat cell lines. Thus, they confirmed that RNPs are the best suitable reagents for T cell gene editing. In addition, they speculate that this may be also due to differential gene regulation between activated T cells and cell lines, as it is proved that chromatin accessibility impact of CRISPR/Cas9 activity. Thus, for subsequent gene editing reagents, inventors decided to test all the designed gRNAs directly in activated primary T cells. They designed and tested gRNAs targeting LAG-3 (5 gRNAs), 2B4 (6 gRNAs) and Tim-3 (3 gRNAs). Results showed that three gRNAs did not display targeting activity at all (g10, g16 and g33 for LAG-3, g3 for 2B4), while g3 targeting LAG-3, g3 targeting Tim-3 and g9 targeting 2B4 displayed respectively 76%, 63% and 63% of NHEJ, respectively **(****Figure 4****).** For TCR disruption, inventors screened 1 gRNA targeting TRAC chain and 3 gRNAs targeting a sequence of the constant region common for TRBC1 and TRBC2 chains. Results showed that g1 targeting TRAC displayed 97,3% of NHEJ, while g1, g2 and g3 targeting TRBC1/2 displayed 90%, 93% and 50% of NHEJ, respectively **(****Figure 4****).** Considering these results, g3 for LAG-3, g9 for 2B4, g3 for Tim-3, g1 for TRAC and g2 for TRBC1/2 gene disruption were selected for all subsequent experiments.

### Efficient entpdl (CD39) disruption in primary T cells by CRISPR/Cas9

The entpd1 gene, encoding for CD39, is located on chromosome 10q24.1 and is composed of 10 exons. To disrupt CD39, the inventors screened two different sgRNAs, designed in exon 2 to reduce the likelihood of obtaining a truncated protein (sg4, sg5).

When disrupting CD39 with sg4 and sg5, each delivered as a ribonucleoprotein complex with the Cas9 in a ratio of 5:1, the inventors observed only a 15% efficiency, which remained stable also when forcing CD39 upregulation with OKT3 and IL-2 **(****Figure 5****, A and B).**

Since enhancing the quantity of Cas9 could lead to off-target events possibly affecting the transcriptome, the team tried to maintain stable the amount of Cas9 while increasing the sgRNA quantity to reach a sgRNA:Cas9 ratio of 9:1. When only 30% of control cells were positive for CD39, a 25% efficiency was calculated, but after further T cell activation up to 50% knock-out efficiency was observed **(****Figure 5****, C and D).** The CD39 disruption was confirmed at the genetic level.

An in silico tool analyzing the Inference of CRISPR Edits (ICE, Synthego) was used to provide a quantitative assessment of the knock-out efficiency by comparing the Sanger Sequencing traces of amplicons generated from edited and control cells. As expected, the traces of the control and edited cells (dark gray and light gray, respectively) were superimposable in the sequence region located before the cut site, while they started to be discordant at the cut site and in its downstream genomic region **(****Figure 6A****).** The ICE analysis showed a knock-out efficiency up to 45% and 30% for sg4 and sg5, respectively, when used in a 9:1 ratio with Cas9, and an indel score of 50% and 60%, respectively **(****Figure 6, B****).** It is important to underline that, while the knock-out score only includes sequences harboring a frameshift mutation or a large (+21 bp) fragment deletion, the indel score comprehends all detected sequences that are different from the wild type. This explains why sg5 displays an indel score higher than its knock-out score. On the contrary, for sg4 the knock-out and indel score were comparable, indicating that the majority of insertions and deletions resulted in functional gene disruption.

### CRISPR/Cas9 and lentiviral vectors allow for complete TCR-gene editing

The present inventors previously developed a complete TCR gene editing protocol based on the genetic disruption of the endogenous TCR alpha and beta genes with ZFN and followed by lentiviral-mediated transfer of a tumor-specific TCR. Complete TCR gene edited lymphocytes proved safer and more effective than conventional TCR gene transferred cells *in vitro* and in animal models of acute myeloid leukemia. Nevertheless, this protocol requires two steps of gene disruptions followed by two steps of viral vector transductions, resulting in more than 40 days of T cell culture and 4 genomic manipulations. As CRISPR/Cas9 system allows for multiple gene disruption in a single reaction, inventors envisage to employ this technology to optimize their already developed TCR gene editing protocol. First, inventors compared TRAC disruption in T cells using CRISPR/Cas9 with they previously validated Zinc Finger Nucleases. Inventors evaluated TRAC knock-out by CD3 expression as surrogate marker for TCR complex disruption and they confirmed flow cytometry data by NHEJ analysis at the TRAC locus. Inventors obtained higher efficiency in TRAC knock-out using CRISPR/Cas9 compared to ZFN, with a mean of 95% and 65,5% of CD3 negative cells with CRISPR/Cas9 and ZFN, respectively. The phenotypic result obtained with CRISPR/Cas9 was confirmed by NHEJ at the TRAC locus **(****Figure 7****, A and B).**

To improve the clinical feasibility of TCR gene editing, the present inventors have previously demonstrated that the single alpha chain disruption with ZFN followed by the transduction with a lentiviral vector encoding for a tumor-specific TCR allows to abrogate endogenous TCR repertoire in a single step of T cell activation and 21 days of cell culture. Although this optimized protocol for single TCR gene editing (SE) abate the mispairing between the remaining endogenous β chain and the tumor specific TCR, alloreactivity is not completely abrogated. Thus, inventors decided to further optimize complete TCR gene editing with CRISPR/Cas9. Inventors simultaneously delivered RNPs targeting both alpha and beta TCR chains and compared TCR gene disruption efficiencies with that observed on single TCR alpha disrupted cells. When tested by flow cytometry, more than 97% of TCR alpha and beta chain disrupted cells failed in expressing CD3 on cell surface **(****Figure 7C****).** However, CD3 expression does not allow for precise quantification of each TCR α and β chain gene disruption, since the disruption of one of the 2 genes is sufficient to impair surface translocation of CD3. Thus, inventors analyzed the percentage of NHEJ at both TRAC and TRBC1/2 *loci.* As shown in figure 7D and E, they observed comparable NHEJ frequency at trac or trbc1/2 *loci* in single alpha and complete alpha and beta chain disrupted cells. Overall, these data confirmed that multiple gene disruption with CRISPR/Cas9 is feasible in one step of manipulation and display the same high level of gene disruption at both genomic sites.

To redirect T cell against tumor-antigens, inventors transduced single alpha and complete alpha and beta chain disrupted cells with a lentiviral vector encoding for a TCR specific for the HLA-A*0201 restricted NY-ESO-1₁₅₇₋₁₆₅ epitope. They transduced T cells with such lentiviral vector 24 hours after TCR endogenous gene disruption. Since it has been proved that RNPs activity is strongly reduced 12 hours after the delivery into the cells, inventors envisage that 24 hours will be sufficient to avoid RNPs mediated cleavage of tumor-specific TCR encoded by the viral vector. Inventors evaluated the percentage of transduced cells detecting by flow cytometry the specific Vβ of the NY-ESO-1 TCR (Vβ 13.1). Inventors obtained high levels of transduction, with a median of 64% of TRAC+TRBC disrupted cells (CE) redirected against the NY-ESO-1 antigen. In comparison, in TRAC disrupted cells (SE) a slight decrease of Vβ^{pos} cells and a concomitant slight increase in CD3pos Vβ^{neg} cells were observed **(****Figure 7****, F and G),** but these differences were not statistically significant.

Overall, these experiments proved that CRISPR/Cas9 in combination with lentiviral vectors allows to develop complete TCR gene edited cells in a single step of manipulation, one round of T cell activation and with a higher efficiency compared to inventors' previously validated ZFN-based protocol.

### TCR gene editing and inhibitory receptors disruptions can be efficiently combined in human memory stem T cells using CRISPR/Cas9 and lentiviral vectors

To generate memory stem T cells redirected against tumor antigens and resistant to exhaustion signals, inventors combined TCR gene editing with IRs disruptions. They simultaneously electroporated in an equimolar ratio RNPs targeting one IR (Tim-3, LAG-3 or 2B4) and RNPs targeting the TRAC locus. 24 hours post electroporation a lentiviral vector encoding for the TCR specific for the HLA-A2-restricted NY-ESO-1₁₅₇₋₁₆₅ epitope was delivered **(****Figure 8A****).** Surprisingly, g3 targeting Tim-3 proved less efficient when co-delivered in multiplexing with TRAC targeting gRNA **(****Figure 8****, B and C).** As inventors obtained less than 50% of CD3^{neg}Tim3^{neg} cells with these two gRNAs combination, they decided to test other 2 gRNAs targeting Tim-3. Inventors tested the new gRNAs both alone and in combination with TRAC knock-out **(****Figure 8D****).** As shown in figure 8D, they obtained a mean of 76% and 94% CD3^{neg}Tim-3^{neg} cells using g1 or g4 respectively. These data were confirmed by molecular analysis **(****Figure 8E****).** Thus, inventors decided to discard g3 and to employ g4 and g1 for multiplexing knock-out experiments.

Inventors tested g3, g9 and g4 targeting LAG-3, 2B4 and Tim-3, respectively. Conversely to what observed for Tim-3 **(****Figure 8F****),** gRNAs targeting LAG-3 and 2B4 were equally efficient when used alone or in combination with the selected TRAC specific gRNA **(****Figure 8F****).**

More in detail, inventors obtained 77%, 92% and 82% of NHEJ at the LAG-3, Tim-3 and the 2B4 loci respectively in double TRAC-IR knock-out cells **(****Figure 8F****).** TRAC disruptions were maintained higher than 90% in all multiplexing conditions **(****Figure 8F****).**

When transduced with the lentiviral vector encoding for the NY-ESO-1 specific TCR, inventors obtained more than 50% of TCR redirection in Tim-3, LAG-3 or 2B4 knock-out cells, thus obtaining a high frequency of IR_{KO} cells redirected against NY-ESO-1 **(****Figure 9A****).**

Inventors did not observe any difference in the expansion capacity of all engineered T cell populations **(****Figure 9B****).** In addition, phenotypic analysis of SE-IR_{KO} cells showed a comparable memory composition to single edited-IR competent (SE-IR_{COMP}) counterparts and all cell populations proved highly enriched in early differentiated cells **(****Figure 9C****).**

Overall, these data demonstrate that CRISPR/Cas9 and lentiviral vectors can be easily combined to generate *in vitro* high numbers of early differentiated T cells redirected against tumor antigens and resistant to inhibitory signals. Of note, CRISPR-Cas9-mediated Tim-3, LAG-3 or 2B4 disruption does not alter the expansion capacity nor the differentiation phenotype of TCR-edited T cells.

### Tim-3- and 2B4-disrupted SE T cells retain high degranulation capacity upon chronic stimulation

Inventors then tested the effect of IR-disruptions in TCR redirected cells upon antigen-specific stimulation in short term co-culture assays.

To investigate if multiple myeloma cells engage Tim-3-, LAG-3- and 2B4-mediated pathways, inventors analyzed the expression of IR ligands in two multiple myeloma cell lines (U266 and MM1s), in both resting conditions and upon IFNγ stimulation. The Gal-9 and HMGB1 release in cell culture medium, the upregulation of MHC-II and CD48 and the flipping of phosphatidyl-serin in the outer layer of cell membrane were quantified. MM1s cells express high levels of all tested IR ligands, while U266 expressed 1log less of CD48 compared to MM1s, and the level of IR ligands remains stable upon IFNγ exposure **(****Figure 10****).** To test the effect of Tim-3, LAG-3 and 2B4 disruption in tumor-specific T cells upon target recognition, inventors challenged SE-IR_{COMP} or SE-IR_{KO}T cells with MM1.s either wild-type (HLA-A2^{neg}NY-ESO-1^{neg}) or transduced to express HLA-A2, the NY-ESO-1 antigen and luciferase **(****Figure 11A****).** In a 3 days co-culture assay, inventors found that the disruption of Tim-3, LAG-3 and 2B4 did not impact on both the killing ability and the specificity of TCR-edited T cells. Interestingly SE-IR_{COMP} T cells showed a slightly reduced anti-tumor response compared to all tested SE-IR_{KO} T cells at limiting effector : target ratio (Figure 11A). In parallel, inventors quantified the production of pro-inflammatory cytokines and effector molecules produced by the developed cellular products upon target recognition. Inventors observed that Tim-3-, LAG-3- and 2B4-disrupted TCR-edited cells produced higher amount of IL-2, TNFα and sFasL compared to SE-IR_{COMP} cells **(****Figure 11****, B-D).** Concomitantly, SE-Tim-3_{KO} T cells produced higher amount of Perforin and a slightly increased amount of Granzyme B compared to SE-IR_{COMP} T cells **(****Figure 11****, E and F).**

To demonstrate that the lack of Tim-3-, LAG-3- and 2B4-mediated inhibitory signals in tumor-specific T cells might boost effector functions in chronically stimulated cells, inventors compared the anti-tumor responses of SE-IR_{COMP} and SE-IR_{KO} T cells upon repeated tumor recognition. T cells were stimulated daily for 15-21 days with target cells and the frequency of CD107a+ CD3+ T cells upon 6-hours restimulation was quantified **(****Figure 11G****).** Of note, inventors observed that only SE-Tim-3_{KO} and SE-2B4_{KO} retained a statistically significant higher capacity to degranulate after chronic stimulation compared to SE-IR_{COMP} cells. The frequency of degranulating cells was higher, but not statistically significant, also in SE-LAG-3_{KO} T cells **(****Figure 11H****).** Overall, these results indicate that the lack of Tim-3 and 2B4 in tumor-specific T cells might sustain a prolonged effector function upon chronic antigen recognition.

### Tim-3- and 2B4-disrupted SE T cells exert higher immunological pressure than SE-IR_{COMP} T cells in a stress test multiple myeloma mouse model

To further validate all the engineered cellular products, the anti-tumor responses of SE-Tim-3_{KO} and SE-2B4_{KO} T cells was evaluated in vivo. Thus, luciferase⁺ A2⁺ NY-ESO-1⁺ MM1.s cells were infused in non-irradiated NSG mice; mice were treated at high tumor burden occurrence (total flux > 10⁸ p/sec) with 5x10⁶ of SE-IR_{COMP}, SE-Tim3_{KO} or SE-2B4_{KO}. Mice left untreated (nill) or infused with unmanipulated cells (UT) were used as control **(****Figure 12A****).**

SE-IR_{COMP} and SE-IR_{KO} cells exerted an anti-tumor activity, with a similar kinetics, in the first week after infusion, but then failed to control tumor growth **(****Figure 12B****).** Concomitantly, the peak of T cell expansion was observed at day 3 post-infusion, and then CD3+ cells progressively decreased in all groups of treatment. At the peak of expansion, circulating SE-2B4_{KO} cells were significantly lower compared to SE-IR_{COMP} cells **(****Figure 12C****).**

As a measure of the immunological pressure exerted by the cellular products on cancer cells, inventors measured the expression of HLA-A2 and NY-ESO-1 in tumor cells harvested at sacrifice. Surprisingly, all mice treated with SE-TIM-3_{KO} and 80% of mice treated with SE-2B4_{KO} T cells showed a large fraction of NY-ESO1^{neg} tumor cells in the bone marrow. In addition, 2 mice treated with SE-2B4_{KO} T cells displayed a fraction of tumor cells with reduced HLA-A2 expression. Conversely, in SE group, only one mouse displayed HLA-A2 negative tumor cells and in 2 out of 5 mice multiple myeloma cells were still NY-ESO-1^{pos} **(****Figure 12D****).** Altogether, these data suggest the presence of a high immunological pression exerted by SE-IR_{KO} T cells on tumor cells.

In line with these observations, CD3+ cells expanded at significantly higher levels in the bone marrow of SE-2B4_{KO} treated mice compared to mice treated with SE-IR_{COMP} T cells. A slight increase (but not statistically significant) of CD3+ cells was also observed in the bone marrow of mice treated with SE-Tim-3_{KO} T cells **(****Figure 12E****).** A significantly higher proportion of activated CD3+ T cells (HLA-DR+PD1+) were observed in the bone marrow of mice treated with SE Tim-3-KO T cells and a similar trend was observed in mice treated with SE 2B4_{KO} cells **(****Figure 12F****).** Notably, unsupervised high dimensional analysis of T cells infiltrating the bone marrow showed a statistically significant enrichment of a cluster (cluster 15) composed by early differentiated (CD45RA⁺CD62L⁺) Vβ13.1⁺ (NY-ESO-1 TCR) activated CD8+ T cells in both SE Tim3_{KO} and SE 2B4_{KO} treated mice compared to SE-IR_{COMP} cells **(****Figure 12****, G and H).**

### In a second multiple myeloma model, Tim-3- and 2B4-disrupted SE T cells retain high degranulation capacity upon chronic stimulation, while Lag-3-disruption prevents the overexpression of additional IRs

The impact of Tim-3, LAG-3 or 2B4 disruption in tumor-specific T cells was assessed in a different multiple myeloma model. SE-IR_{COMP} and SE-IR_{KO} T lymphocytes were challenged with U266 cells, a multiple myeloma cell line that physiologically expresses HLA-A2 and NY-ESO-1 antigen. As shown in figure 13A, SE and SE-IR_{KO} T cells displayed comparable killing activity in all tested effector:target ratio. In U266 tumor model, only SE Tim-3_{KO} and SE 2B4_{KO} T cells produced significantly higher amount of Perforin and sFasL compared to SE-LAG-3_{KO} and SE-IR_{COMP} cells in short-term antigen stimulation **(****Figure 13B****).** In addition, only SE 2B4_{KO} cells produced significantly higher amount of IL-2 and TNFα compared to SE-LAG-3_{KO} and SE-IR_{COMP} T cells **(****Figure 13B****).** A similar increase was also observed in SE-Tim3_{KO} cells, although this delta was not statistically significant **(****Figure 13B****).**

To demonstrate whether SE-IR_{KO} T cells retain effector functions upon chronic stimulation in this second multiple myeloma model, SE-IR_{COMP} and SE-IR_{KO} T cells were daily co-cultured with target cells. After 15-21 days, the degranulation capacity of all cellular products was quantified by flow cytometry upon short-term restimulation with target cells. **(****Figure 13C****).** In line with data observed with the MM1.s model, only SE-Tim3 _{KO} and SE-2B4_{KO} T cells retain a significantly higher capacity to degranulate compared to SE-IR_{COMP} T cells **(****Figure 13D****).**

Additionally, the phenotype of SE-IR_{COMP} and SE-LAG-3_{KO} T cells was evaluated by multiparametric flow cytometry upon chronic stimulation with U266 tumor cells. After 4 rounds of stimulation, unsupervised high-dimensional analysis revealed a significant enrichment of clusters 9 and 12 represented by CD8+ T_{CM} and T_{EM} cells expressing multiple (5) IRs in SE-IR_{COMP} condition compared to the SE-LAG-3_{KO} condition **(****Figure 13****, E and F).** Furthermore, cluster 2 and cluster 5 composed by T cells expressing only 2 IRs (PD1+ and CTLA4+) were significantly enriched in SE-LAG-3_{KO} cells compared to SE cells **(****Figure 13****, E and** F). These observations suggest that the disruption of LAG-3 might counteract the upregulation of additional inhibitory receptors in a chronic stimulation setting.

### SE LAG-3_{KO} display a significantly longer anti-tumor response and display a less exhausted phenotype than SE LAG-3_{COMP} cells

To investigate the anti-tumor activity of SE-LAG-3_{KO} and SE-IR_{COMP} T cells, endpoint *in vivo* experiments were performed. 10x10⁶ luciferase⁺ U266 cells were injected in sub-lethally irradiated NSG mice; mice with a high-tumor burden were treated with 10⁶ or 5x10⁶ SE or SE LAG-3_{KO} cells. Unmanipulated cells (5x10⁶ dose) were also infused as controls. Mice were sacrificed 3 weeks after T-cell infusion **(****Figure 14A****).** Tumor growth was monitored by total body bioluminescence imaging. Inventors observed that the dose of 10⁶ was not sufficient for controlling tumor growth independently from the cellular product infused. On the contrary, 5x10⁶ SE T cells induced a partial tumor control, and 5x10⁶ SE LAG-3_{KO} T cells completely eradicated the tumor **(****Figure 14B****).** Circulating T cells were characterized by flow cytometry. As expected, in mice receiving the low dose T cells, inventors observed low frequencies of circulating human lymphocytes. In mice treated with the high dose of T cells, lymphocytes had accumulated more **(****Figure 14****, C-D).** Specifically, low dose of SE LAG-3_{KO} cells displayed a significantly higher proportion of activated (HLA-DR+) cells compared to SE, indicative of a more activated phenotypic profile **(****Figure 14E****).** At sacrifice, a higher proportion of 10⁶ SE cells infiltrating the bone marrow co-expressed multiple IRs compared to 10⁶ SE LAG-3_{KO} cells, indicating that LAG-3 disruption in T cell redirected against NY-ESO-1 prevents the upregulation of other inhibitory receptors, even in stressed conditions of tumor outgrowth **(****Figure 14F****).**

Since repeated antigen encounter might trigger T-cell exhaustion, the anti-tumor responses of SE and SE LAG-3_{KO} cells were compared in a tumor rechallenge experiment. NSG mice were infused with U266 and treated mice in a minimal residual disease setting **(****Figure 14G****).** Mice were treated with either SE or SE LAG-3_{KO} cells. Untreated mice and mice injected with 5 x10⁶ unmanipulated cells were used as controls **(****Figure 14G****).** Tumor burden was evaluated by total body bioluminescence imaging. As shown in Figure 14H, all edited cellular products completely eradicated the tumors in the first 3 weeks upon treatment. Circulating tumor-specific T cells were re-challenged with a second infusion of tumor cells at day 21. Interestingly, SE LAG-3_{KO} cells, but not SE-IR_{COMP} cells, were able to completely prevent the second engraftment of the tumor, thus indicating an enhanced T cell fitness of SE LAG-3_{KO} cells upon secondary antigen encounter *in vivo* **(****Figure 14H****)**

Overall, these data indicate that Tim-3 and 2B4 disruption in TCR-edited cells enhance their effector functions and induce a high production of pro-inflammatory cytokines, while LAG-3 disruption prevent the upregulation of additional IRs upon chronic target recognition. Thus, these evidences support our hypothesis that IRs disruption could boost anti-tumor responses and might prevent functional exhaustion. In addition, these data suggest that the disruption of each individual inhibitory receptor might produce different effects on T cell function and signaling. Inventors speculate that these differential effects might be linked to the context-dependent role of inhibitory receptors.

### TCR gene editing in regulatory T cells

Inventors applied the TRAC disruption protocol to Regulatory T cells (Tregs). Gene disruption efficiency was 80% in Treg cells **(****Figure 15A****,** left panels). After TRAC disruption and transduction with a lentiviral vector encoding for a NY-ES01-specific TCR, inventors obtained more than 70% of edited Tregs **(****Figure 15A****,** right panels and **Figure 15B****).** TRAC disruption did not modify Treg phenotype, as assessed by cytofluorimetric analysis of CD25, Foxp3 and CD127 expression **(****Figure 15C****).** Finally, edited Tregs maintained their functional abilities. More in details, they not only were able to efficiently suppress Tconv proliferation in the presence of the antigen, but were also able to inhibit Tconv killing capacity **(****Figure 15D****).**

### DISCUSSION

Inventors herein implemented the CRISPR/Cas9 technological platform into their TCR gene editing protocol (Provasi Nat Med, Mastaglio Blood). This is a much more flexible genome editing platform, adaptable to simultaneous multiple gene targeting. CRISPR/Cas9 nucleases have dramatically changed the scenario of genome editing, raising incredible benefits. The CRISPR/Cas9 system harbors major advantages over already validated ZFNs and TALENs because it only requires: (i) an RNA molecule complementary to target DNA and assembled with Cas9 nucleases; (ii) the recognition of target DNA based on Watson&Crick rules of base pairing. At difference from ZFNs and TALENs, that require complex protein design and validation, CRISPR/Cas9 is user-friendly, relatively easy to use, and allows to simultaneously target more than one locus in a single manipulation step. As inventors' aim is to simultaneously disrupt both the endogenous TCR and at least one inhibitory receptor gene, CRISPR/Cas9 represents the perfect tool for our purposes.

Inventors successfully designed and developed gRNA for TCR α and β chains disruption. With an efficiency of >90%, these reagents proved more effective than previously validated ZFNs in inducing TCR disruption. Of note, CRISPR/Cas9 reagents allowed to simultaneously disrupt both TCR chains, with more than 90% disruption at both genomic loci, as assessed at molecular level. Thus, our approach further improves the TCR gene editing protocol, allowing to obtain complete TCR gene editing within a single step of multiple gene disruption, followed by one step of gene insertion with no requirements of cell sorting.

Once infused in patients, tumor-specific T cells are expected to migrate to the tumor, recognize cancer cells and kill them. However, the tumor microenvironment is a complex ecosystem that shapes T cell responses. Tumor specific T cells, chronically stimulated by cancer cells in an immunosuppressive TME, acquire an exhaustion signature. A hallmark of this condition is the upregulation on the cell surface of multiple inhibitory receptors, including CTLA-4, PD-1, Tim-3, LAG-3 and 2B4. Cancer cells exploit these inhibitory pathways to dampen T cell responses. Here inventors selectively disrupt multiple IRs with designed gRNAs to be implemented in their TCR-gene editing protocol, with the ultimate aim of producing TCR redirected cells resistant to at least one exhaustion signal. One theoretical limitation of this approach might be the fact that IR are not only expressed by exhausted cells, but also by activated cells, in combination with other activation markers, such as MHC class II, CD25 or CD69. However, inventors herein show that neither Tim3, nor Lag3, nor 2B4 are required for T cell expansion, activation, differentiation and acquisition of effector functions, since none of these functions were reduced in IR KO cells compared to IR competent counterparts.

Anti-CTLA-4 and anti-PD-1 monoclonal antibodies were the first ICB tested in clinical trials for the treatment of both solid and hematological malignancies and showed impressive clinical results in terms of tumor control and overall survival. However, some limitations emerged from these trials. First, due to the intrinsic heterogeneity within the exhausted T cell pool, ICB display in some cases only partial efficacy. In addition, despite their efficacy, the use of ICB is often limited by the occurrence of autoimmune related adverse events. This secondary unwanted reaction is mainly due to the effect of ICS on the entire T cell repertoire, rather than only on tumor-specific T cells. Additionally, due to the constitutional expression of IR receptors on Tregs, ICB dampen Treg functionality, thus fostering autoimmune conditions. The present approach aims at overcoming these limitations: since in our cellular products inhibitory receptors are disrupted only on TCR gene edited cells, the inhibitory brakes are unleashed only on such tumor-specific cells.

Inventor here successfully developed gRNAs to genetically disrupt Tim-3, LAG-3, 2B4 and the endogenous TCR (both α and β chains). In order to develop cellular products highly enriched in IR-disrupted cells, inventors designed and tested multiple gRNAs. We initially validated the gRNAs in K562-iCas9 cell line and assessed gene disruption by NHEJ in order to select the gRNAs with the highest cut efficiency. However, when tested in primary human T cells, gRNAs performed with a different efficiency compared to that observed in cell lines. As CRISPR/Cas9 DNA binding is strictly dependent on chromatin accessibility (Knight et al, 2015), we speculated that the discrepancy in gene disruption efficiencies might depend on differential gene expressions among the two cellular types. Thus, inventors reasoned that gRNAs should be directly screened on the final target cell subpopulation (in this case primary human T cells) to be efficient.

The simultaneous editing of multiple genes poses the risk of off-target recognition and translocations between the genomic regions targeted by the gRNA. Although inventors did not observe changes in the expansion, differentiation, phenotype and function of edited T cells, neither in vitro nor in vivo, ongoing experiments are currently tackling these 2 relevant issues for future clinical implementation.

A growing number of evidences show that objective clinical responses obtained with adoptive immunotherapies are strongly dependent on the levels of T cell engraftment, expansion and persistence (Robbins et al, 2015), which strictly rely on the composition of the infused T cell product (Busch et al, 2016). Since our purpose is to develop long-lasting cellular products, we envisage to genetically engineer T cells with an early differentiated phenotype. We took advantage of a peculiar memory subpopulation, namely memory stem T cells (T_{SCM}). Several studies dissecting the memory ontogeny in vivo in a wide spectrum of clinical conditions indicate that T_{SCM} cells are endowed with stemness, high-proliferative potential and superior long-term persistence (Fuertes Marraco et al, 2015b; Oliveira et al, 2015; Biasco et al, 2015; Cieri et al, 2015; Roberto et al, 2015; Cianciotti et al, 2019). Given their unique characteristics, T_{SCM} cells represent the ideal subset to exploit for ACT.

Here, inventors optimized reagents and culture conditions to generate TCR-edited-IR-negative T_{SCM} and T_{CM} cells: they simultaneously disrupted the endogenous TCR α chain in combination with Tim-3, LAG-3 or 2B4 and generated TCRedited-IR_{KO} cells by transduction with a lentiviral vector encoding for an HLA-A2 restricted NY-ESO-1 specific TCR. Unexpectedly, inventors observed a decrease in the gene targeting efficiency when the already validated gRNA targeting Tim-3 was tested in multiplexing. This required the testing and the validation of additional gRNAs in combination with the TCR gene editing procedure. This observation indicates that, although the CRISPR/Cas9 system is easy-to-use and efficient in multiple gene targeting, some level of reagents optimization is still required in multiplexing. All the other validated gRNAs maintained the same levels of gene disruption efficiencies when tested in multiplexing for the simultaneous TRAC disruption. Upon multiplexing testing, inventors were able to identify the best performing gRNAs combinations and obtain high efficiency of Tim-3, LAG-3 and 2B4 gene disruption (at least 80% of NHEJ at IRs loci) in TRAC disrupted T_{SCM} cells and more than 50% of the cells were efficiently redirected against the NY-ESO-1 antigen.

The present data indicate that multiple gene disruptions by CRISPR/Cas9 in combination with TCR gene editing is feasible without altering the phenotype of the cellular product nor its expansion and proliferative capacities. Of note, the present results indicate that TCR-edited-IR_{KO} cells retain an early differentiated phenotype, thus suggesting that Tim-3, LAG-3 and 2B4 disruptions do not impact on the memory differentiation of edited cells.

Although IRs have been extensively investigated in several infectious and cancer models, the role of each individual inhibitory receptor on T cell response has not yet fully elucidated. TCR-edited-IR_{KO} cells developed in this work provide a platform to dissect the role of each IR on T cell mediated response. Currently, no evidences are present in literature about the impact of Tim-3 and 2B4 disruptions on antigen specific T cell responses and only a single paper investigated the effect of LAG-3 disruption in CAR-T cells (Zhang et al, 2017). In this study, the authors reported that LAG-3 disruption did not impact on the T cell fitness and phenotype. However, only 50% of edited cells were LAG-3 disrupted cells. In vitro cytotoxicity assays did not indicate major advantages in LAG-3 disruption. Although in vivo validation shows a slight increase in the survival of mice treated with LAG-3-disrupted CAR-T cells, the data did not show an enhanced tumor control. This work proved that LAG-3 disruption is feasible and tolerated by T cells, but conclusive results on the impact of LAG-3 disruption on CAR-T cells were missing.

Here, inventors dissect the impact of LAG-3, Tim-3 and 2B4 disruption on TCR-edited cells both in vitro and in preclinical models of multiple myeloma. The evaluation of cytotoxicity in short-term assays shows that TCR-edited-Tim-3_{KO}, TCR-edited-LAG-3_{KO} and TCR-edited-2B4 _{KO} cells displayed comparable killing activity to TCR-edited-IR_{COMP} cells. These results indicate that IRs-disrupted T cells retain their killing capacity, thus proving that Tim-3, LAG-3 or 2B4 are not required on T cells for effective effector functions. However, although compared to CARs, TCRs provide a much more physiological stimulation signal to T cells, the NY-ESO-1 specific TCR used in this work display a high affinity for the antigen, thus not properly recapitulating natural occurring low affinity TCR of tumor-specific exhausted cells infiltrating the tumors. Further experiments with low affinity tumor-specific TCR might reveal a different impact of Tim-3, LAG-3 and 2B4 disruptions on tumor recognition.

The analysis of IFN-γ, IL2 and TNF-α producing cells revealed a higher frequency of IL-2ₚₒₛ cells in TCR-edited-Tim-3_{KO} and TCR-edited-2B4 _{KO} cells compared to TCR-edited-LAG3_{KO} and TCR-edited-IR_{COMP} cells. These data are in line with our previous findings that in vitro generate T_{SCM}, due to their early differentiated phenotype, mainly produce IL-2, but not high levels of IFN-γ and TNF-α, upon PMA/ionomycin stimulation (Cieri et al, 2013). The analysis of cytokine producing cells in IRs-disrupted effector cells, a memory subpopulation physiologically more able to produce effector molecules, might be informative.

While it was not observed significant differences in the percentage of cytokine producing cells, the analysis of the amounts of secreted cytokines showed more interesting results. The quantification of cytokines secreted in the medium upon tumor recognition indicates that TCR-edited-Tim-3_{KO} and TCR-edited-2B4_{KO} cells, but not TCR-edited-LAG-3_{KO} cells, produce a higher amount of pro-inflammatory cytokines than TCR-edited-IR_{COMP} cells. These observations indicate that Tim-3 and 2B4, but not LAG-3, disruptions significantly enhance the production of cytotoxic cytokines.

Here, we demonstrated that multiple gene disruptions are feasible and highly efficient with CRISPR/Cas9 reagents and, in combination with TCR redirection through lentiviral vectors, allow the generation of TCR-edited cells devoid of one inhibitory pathway. Inventors also showed that our genetic manipulation did not impact on the phenotype of edited cells, as our cellular products are highly enriched in early differentiated T_{SCM}/T_{CM} cells. Although not fully comprehensive, the present in vitro analysis of TCR-edited-IR_{NEG} cells demonstrated that T cell functions are retained upon IRs disruptions. More interestingly, in vitro data revealed that each IR impacts on distinct aspects of the biology of T cell response.

Modified commercial gRNAs and a high-fidelity version of Cas9 were used, thus it is expected to detect low numbers of off-targets in our cellular products.

### REFERENCES

Biasco L, Scala S, Basso Ricci L, Dionisio F, Baricordi C, Calabria A, Giannelli S, Cieri N, Barzaghi F, Pajno R, Al-Mousa H, Scarselli A, Cancrini C, Bordignon C, Roncarolo MG, Roncarolo MG, Montini E, Bonini C & Aiuti A (2015) In vivo tracking of T cells in humans unveils decade-long survival and activity of genetically modified T memory stem cells. Sci. Transl. Med. 7: 273ra13
Blackburn SD, Shin H, Haining WN, Zou T, Workman CJ, Polley A, Betts MR, Freeman GJ, Vignali DAA & Wherry EJ (2009) Coregulation of CD8+ T cell exhaustion by multiple inhibitory receptors during chronic viral infection. Nat. Immunol. 10: 29-37
Busch DH, Fräßle SP, Sommermeyer D, Buchholz VR & Riddell SR (2016) Role of memory T cell subsets for adoptive immunotherapy. Semin. Immunol. 28: 28-34 Available at: http://dx.doi.org/10.1016/j.smim.2016.02.001
Canale FP, Ramello MC, Núñez N, Furlan CLA, Bossio SN, Serrán MG, Boari JT, Del Castillo A, Ledesma M, Sedlik C, Piaggio E, Gruppi A, Rodriguez EVA & Montes CL (2018) CD39 expression defines cell exhaustion in tumor-infiltrating CD8+ T cells. Cancer Res.
Cianciotti BC, Ruggiero E, Campochiaro C, Oliveira G, Magnani ZI, Baldini M, Doglio M, Tassara M, Manfredi AA, Baldissera E, Ciceri F, Cieri N & Bonini C (2019) CD4 + memory stem T cells recognizing citrullinated epitopes are expanded in patients with Rheumatoid Arthritis and sensitive to TNF-α blockade. Arthritis Rheumatol.: ahed of print Available at: https://onlinelibrary.wiley.com/doi/abs/10.1002/art.41157
Cieri N, Camisa B, Cocchiarella F, Forcato M, Oliveira G, Provasi E, Bondanza A, Bordignon C, Peccatori J, Ciceri F, Stanghellini MTL, Mavilio F, Mondino A, Bicciato S, Recchia A & Bonini C (2013) IL-7 and IL-15 instruct the generation ofhuman memory stem T cells from naive precursors. 121: 573-585
Cieri N, Oliveira G, Greco R, Forcato M, Taccioli C, Cianciotti B, Valtolina V, Noviello M, Vago L, Bondanza A, Lunghi F, Marktel S, Bellio L, Bordignon C, Bicciato S, Peccatori J, Ciceri F & Bonini C (2015) Generation of human memory stem T cells after haploidentical T-replete hematopoietic stem cell transplantation. Blood 125: 2865-2874
Clay TM, Custer MC, Sachs J, Hwu P, Rosenberg SA & Nishimura MI (1999) Efficient transfer of a tumor antigen-reactive TCR to human peripheral blood lymphocytes confers anti-tumor reactivity. J. Immunol. 163: 507-13 Available at: http://www.ncbi.nlm.nih.gov/pubmed/10384155
Fourcade J, Sun Z, Benallaoua M, Guillaume P, Luescher IF, Sander C, Kirkwood JM, Kuchroo V & Zarour HM (2010) Upregulation of Tim-3 and PD-1 expression is associated with tumor antigen-specific CD8+ T cell dysfunction in melanoma patients. J. Exp. Med. 207: 2175-2186
Fuertes Marraco SA, Soneson C, Cagnon L, Gannon PO, Allard M, Maillard SA, Montandon N, Rufer N, Waldvogel S, Delorenzi M & Speiser DE (2015) Long-lasting stem cell-like memory CD8+ T cells with a naive-like profile upon yellow fever vaccination. Sci. Transl. Med. 7***:***
   Gattinoni L, Lugli E, Ji Y, Pos Z, Paulos CM, Quigley MF, Almeida JR, Gostick E, Yu Z, Carpenito C, Wang E, Douek DC, Price DA, June CH, Marincola FM, Roederer M & Restifo NP (2011) A human memory T cell subset with stem cell-like properties. Nat. Med. 17: 1290-1297
Gattinoni L, Speiser DE, Lichterfeld M & Bonini C (2017) T memory stem cells in health and disease. Nat. Med. 23: 18-27
Jin HT, Anderson AC, Tan WG, West EE, Ha SJ, Araki K, Freeman GJ, Kuchroo VK & Ahmed R (2010) Cooperation of Tim-3 and PD-1 in CD8 T-cell exhaustion during chronic viral infection. Proc. Natl. Acad. Sci. U. S. A. 107: 14733-14738
Kassu A, Marcus RA, D'Souza MB, Kelly-McKnight EA, Golden-Mason L, Akkina R, Fontenot AP, Wilson CC & Palmer BE (2010) Regulation of Virus-Specific CD4 + T Cell Function by Multiple Costimulatory Receptors during Chronic HIV Infection . J. Immunol. 185: 3007-3018
Kaufmann DE, Kavanagh DG, Pereyra F, Zaunders JJ, Mackey EW, Miura T, Palmer S, Brockman M, Rathod A, Piechocka-Trocha A, Baker B, Zhu B, Le Gall S, Waring MT, Ahern R, Moss K, Kelleher AD, Coffin JM, Freeman GJ, Rosenberg ES, et al (2007) Upregulation of CTLA-4 by HIV-specific CD4+ T cells correlates with disease progression and defines a reversible immune dysfunction. Nat. Immunol. 8: 1246-1254
Knight SC, Xie L, Deng W, Guglielmi B, Witkowsky LB, Bosanac L, Zhang ET, Beheiry ME, Masson JB, Dahan M, Liu Z, Doudna JA & Tjian R (2015) Dynamics of CRISPR-Cas9 genome interrogation in living cells. Science (80-. ). 350: 823-826
Larkin J, Chiarion-Sileni V, Gonzalez R, Grob JJ, Cowey CL, Lao CD, Schadendorf D, Dummer R, Smylie M, Rutkowski P, Ferrucci PF, Hill A, Wagstaff J, Carlino MS, Haanen JB, Maio M, Marquez-Rodas I, McArthur GA, Ascierto PA, Long G V., et al (2015) Combined nivolumab and ipilimumab or monotherapy in untreated Melanoma. N. Engl. J. Med. 373: 23-34
Leach DR, Krummel MF & Allison JP (1996) Enhancement of antitumor immunity by CTLA-4 blockade. Science (80-.). 271: 1734-1736
Maniatis T, Fritsch EF & Sambrook J (1982) Molecular Cloning: A Laboratory Manual. Cold Spring Harbor laboratory press
Mastaglio S, Genovese P, Magnani Z, Ruggiero E, Landoni E, Camisa B, Schiroli G, Provasi E, Lombardo A, Reik A, Cieri N, Rocchi M, Oliveira G, Escobar G, Casucci M, Gentner B, Spinelli A, Mondino A, Bondanza A, Vago L, et al (2017) NY-ESO-1 TCR single edited stem and central memory T cells to treat multiple myeloma without graft-versus-host disease. Blood 130: 606-618
Nakamoto N, Cho H, Shaked A, Olthoff K, Valiga ME, Kaminski M, Gostick E, Price DA, Freeman GJ, Wherry EJ & Chang KM (2009) Synergistic reversal of intrahepatic HCV-specific CD8 T cell exhaustion by combined PD-1/CTLA-4 blockade. PLoSPathog. 5***:***
   Noviello M, Manfredi F, Ruggiero E, Perini T, Oliveira G, Cortesi F, De Simone P, Toffalori C, Gambacorta V, Greco R, Peccatori J, Casucci M, Casorati G, Dellabona P, Onozawa M, Teshima T, Griffioen M, Halkes CJM, Falkenburg JHF, Stölzel F, et al (2019) Bone marrow central memory and memory stem T-cell exhaustion in AML patients relapsing after HSCT. Nat. Commun. 10: 1-15
Oliveira G, Ruggiero E, Stanghellini MTL, Cieri N, D'Agostino M, Fronza R, Lulay C, Dionisio F, Mastaglio S, Greco R, Peccatori J, Aiuti A, Ambrosi A, Biasco L, Bondanza A, Lambiase A, Traversari C, Vago L, Kalle C Von, Schmidt M, et al (2015) Tracking genetically engineered lymphocytes long-term reveals the dynamics of t cell immunological memory. Sci. Transl. Med. 7: 1-14
Provasi E, Genovese P, Lombardo A, Magnani Z, Liu PQ, Reik A, Chu V, Paschon DE, Zhang L, Kuball J, Camisa B, Bondanza A, Casorati G, Ponzoni M, Ciceri F, Bordignon C, Greenberg PD, Holmes MC, Gregory PD, Naldini L, et al (2012) Editing T cell specificity towards leukemia by zinc finger nucleases and lentiviral gene transfer. Nat. Med. 18: 807-815
Robbins PF, Kassim SH, Tran TLN, Crystal JS, Morgan RA, Feldman SA, Yang JC, Dudley ME, Wunderlich JR, Sherry RM, Kammula US, Hughes MS, Restifo NP, Raffeld M, Lee CCR, Li YF, El-Gamil M & Rosenberg SA (2015) A pilot trial using lymphocytes genetically engineered with an NY-ESO-1-reactive T-cell receptor: Long-term follow-up and correlates with response. Clin. Cancer Res. 21: 1019-1027
Roberto A, Castagna L, Zanon V, Bramanti S, Crocchiolo R, McLaren JE, Gandolfi S, Tentorio P, Sarina B, Timofeeva I, Santoro A, Carlo-Stella C, Bruno B, Carniti C, Corradini P, Gostick E, Ladell K, Price DA, Roederer M, Mavilio D, et al (2015) Role of naive-derived T memory stem cells in T-cell reconstitution following allogeneic transplantation. Blood 125: 2855-2864
Sakuishi K, Apetoh L, Sullivan JM, Blazar BR, Kuchroo VK & Anderson AC (2010) Targeting Tim-3 and PD-1 pathways to reverse T cell exhaustion and restore anti-tumor immunity. J. Exp. Med. 207: 2187-2194
Toffalori C, Zito L, Gambacorta V, Riba M, Oliveira G, Bucci G, Barcella M, Spinelli O, Greco R, Crucitti L, Cieri N, Noviello M, Manfredi F, Montaldo E, Ostuni R, Naldini MM, Gentner B, Waterhouse M, Zeiser R, Finke J, et al (2019) Immune signature drives leukemia escape and relapse after hematopoietic cell transplantation. Nat. Med. 25: 603-611 Available at: http://dx.doi.org/10.1038/s41591-019-0400-z
Wherry EJ & Kurachi M (2015) Molecular and cellular insights into T cell exhaustion. Nat. Rev. Immunol. 15: 486-499
Wolchok JD, Kluger H, Callahan MK, Postow MA, Rizvi NA, Lesokhin AM, Segal NH, Ariyan CE, Gordon RA, Reed K, Burke MM, Caldwell A, Kronenberg SA, Agunwamba BU, Zhang X, Lowy I, Inzunza HD, Feely W, Horak CE, Hong Q, et al (2013) Nivolumab plus Ipilimumab in advanced melanoma. N. Engl. J. Med. 369: 122-133
Zhang Y, Zhang X, Cheng C, Mu W, Liu X, Li N, Wei X & Liu X (2017) CRISPR-Cas9 mediated LAG-3 disruption in CAR-T cells. 11: 554-562

## Claims

1. An isolated guide ribonucleic acid (gRNA) comprising a guide sequence targeting an inhibitory receptor (IR), a TCR α (TRAC) constant region or a β chain (TRBC1/2) constant region target sequence, wherein said guide sequence is selected from the group consisting of SEQ ID NOs: 1-27.

2. An isolated guide ribonucleic acid (gRNA) comprising a guide sequence selected from the group consisting of:
- at least 17, 18, 19, 20 contiguous nucleotides of a sequence selected from SEQ ID NOs: 1-27, or
- a sequence that is at least 90% identical to a sequence selected from SEQ ID NOs: 1-27.

3. A composition comprising at least one gRNA according to any one of previous claims or a nucleic acid molecule encoding said at least one gRNA.

4. The composition of claim 3 comprising:
- at least one gRNA comprising a guide sequence selected from SEQ ID NOs: 1-10, and
- at least one further gRNA comprising a guide sequence selected from SEQ ID NOs:11-23 and optionally
- at least one further gRNA comprising a guide sequence of SEQ ID NO:24 and/or at least one further gRNA comprising a guide sequence selected from SEQ ID NOs:25-27.

5. The composition of claim 3 comprising:
- at least one gRNA comprising a guide sequence selected from SEQ ID NOs:1-23, and
- at least one further gRNA comprising a guide sequence of SEQ ID NO:24 and/or at least one further gRNA comprising a guide sequence selected from SEQ ID NOs:25-27.

6. The composition according to any one of claims 3-5 comprising:
(i) a gRNA comprising a guide sequence of SEQ ID NO:24 and/or a gRNA comprising a guide sequence selected from SEQ ID NOs:25-27, preferably SEQ ID NO:26, and
(ii) a gRNA comprising a guide sequence of SEQ ID NO: 1 or
a gRNA comprising a guide sequence of SEQ ID NO:3 or
a gRNA comprising a guide sequence of SEQ ID NO:11 or
a gRNA comprising a guide sequence of SEQ ID NO:15.

7. The composition according to any one of claims 3-6, further comprising an RNA-guided DNA binding agent or a nucleic acid encoding an RNA-guided DNA binding agent, preferably Cas9.

8. A composition comprising a ribonucleoprotein (RNP) complex comprising the composition of claim 7, preferably further comprising an enhancer.

9. A nucleic acid molecule encoding at least one gRNA as defined in any one of claims 1-2, or at least one gRNA as defined in any one of claims 3-6 or combinations thereof, preferably wherein said nucleic acid is operably linked to a promoter sequence that is recognized by a phage RNA polymerase for in vitro RNA synthesis, preferably wherein said nucleic acid is part of a vector.

10. A Clustered Regularly Interspaced Short Palindromic Repeats (CRISPR)-CRISPR-associated protein 9 (Cas9) system comprising:
a. a Cas9 protein or a nucleic acid molecule encoding a Cas9 protein, and
b. at least one gRNA according to any one of claims 1-2 or the composition of claims 3-8 or at least one nucleic acid molecule according to claim 9,
preferably wherein the system and or a. or b. are comprised in a ribonucleoprotein (RNP) complex, preferably delivered by electroporation, and/or packaged into a viral vector.

11. A vector comprising or encoding at least one gRNA as defined in any one of claims 1-2, or at least one gRNA as defined in any one of claims 3-8 or at least one nucleic acid molecule of claim 9 or comprising a CRISPR-Cas system according to claim 10, preferably said vector is or comprises a plasmid vector or a viral vector, such as a retroviral vector, gammaretroviral vector, herpesvirus vector, lentiviral vector, adenoviral vector or adeno-associated vector.

12. An engineered cell comprising at least one gRNA according to claim 1-2, or the composition of claims 3-8 or the nucleic acid molecule according to claim 9 or the CRISPR/Cas system according to claim 10 or the vector according to claim 11,
preferably said cell being an immune cell, such as a T cell (e.g., a CD2+, a CD3+, a CD4+ or CD8+ T cell, CAR-T cell, NK T cell, iNKT, alpha beta T cell or gamma delta T cell, regulatory T cells), MAIT, more preferably being a memory stem T cells (T_{SCM}), a central memory (T_{CM}) lymphocytes, T naive, mixed T naive (TN) and stem memory T cells (TSCM), effector memory or effector T cells or regulatory T cell.

13. The engineered cell of claim 12, wherein the cell further comprises a recombinant protein, or a polynucleotide encoding a recombinant protein,
or wherein the cell has been or will be engineered to express a recombinant protein, preferably said recombinant protein being a recombinant receptor, more preferably a receptor expressed on the surface of the immune cell receptor,
preferably wherein the recombinant receptor specifically binds to an antigen, and preferably wherein the immune cell is capable of inducing cytotoxicity, proliferating and/or secreting a cytokine upon binding of the recombinant receptor to the antigen, preferably wherein the recombinant receptor is a recombinant T cell receptor (TCR) or a chimeric antigen receptor (CAR) or a T cell redirected for universal cytokine killing (TRUCK).

14. A pharmaceutical composition comprising at least one guide RNA according to any one of claims 1-2 or the composition according to any one of claims 3-8, or at least one nucleic acid molecule of claim 9, a CRISPR/Cas9 system according to claim 10, or at least one vector according to claim 11 or at least one cell according to claim 12 or 13 and at least one pharmaceutically acceptable carrier and/or excipient.

15. An in vitro or ex-vivo method of altering expression of at least one IR gene and optionally of TRAC and/or TRBC1/2 gene, in an isolated cell, said method comprising introducing into an isolated cell a guide RNA according to any one of claims 1-2, or the composition of any one of claims 3-8 or at least one nucleic acid molecule of claim 9, in a CRISPR/Cas9 system, wherein the guide RNA targets the gene and the Cas protein cleaves the genomic loci of the DNA molecules encoding the one or more gene products, whereby expression of the one or more gene products is decreased or abolished,
preferably said cell being an immune cell, such as a T cell (e.g., a CD2+, a CD3+, a CD4+ or CD8+ T cell, CAR-T cell, NK T cell, iNKT, alpha beta T cell or gamma delta T cell, regulatory T cells), MAIT, more preferably being a memory stem T cells (T_{SCM}), a central memory (T_{CM}) lymphocytes, T naive, mixed T naive (TN) and stem memory T cells (TSCM), effector memory or effector T cells or regulatory T cell.

16. The gRNA according to any one of claims 1-2, or the composition of claims 3-8 or the nucleic acid molecule according to claim 9 or the CRISPR/Cas system according to claim 10 or the vector according to claim 11 or the pharmaceutical composition according to claim 14 or the cells of any one of claims 12-13 for use as a medicament, preferably for use in gene therapy, preferably for use in the treatment and/or prevention of a condition selected from the group consisting of: cancer or tumor, an inflammatory disease, an autoinflammatory disease, an autoimmune disease, an allergic disease, an infectious disease, graft versus host disease, organ rejection and/or immune response induced by gene therapy,
preferably the cancer or tumor is selected from the group consisting of: a leukemia, lymphoma, chronic lymphocytic leukemia (CLL), lymphoma, chronic lymphocytic leukemia (CLL), B cell acute lymphocytic leukemia (B-ALL), acute lymphoblastic leukemia (ALL), acute myeloid leukemia, myelodysplasia, myeloproliferative neoplasm, Hodgkin's lymphoma, non-Hodgkin's lymphoma (NHL), diffuse large cell lymphoma (DLCL), indolent B cell lymphoma, refractory follicular lymphoma, mantle cell lymphoma, multiple myeloma, renal cell carcinoma (RCC), B cell malignancies, colon cancer, neuroblastoma, cervical carcinoma, colorectal cancer, breast cancer, bone cancer, ovarian cancer, skin cancer, melanoma, sarcoma, prostate cancer, lung cancer, liver cancer, esophageal cancer, hepatocellular carcinoma, pancreatic cancer, gastric cancer, astrocytoma, brain cancer, epithelial cancers, renal cell carcinoma (RCC), Ewing sarcoma, neuroblastoma, pancreatic adenocarcinoma, mesothelioma, glioblastoma, osteosarcoma, synovial sarcoma, head and neck cancer, medulloblastoma,
preferably the autoimmune disease is selected from the group consisting of: type 1 diabetes mellitus, autoimmune enteropathy, rheumatoid arthritis, systemic lupus erythematosus, multiple sclerosis, autoimmune myositis, psoriasis, Addison's disease, Grave's disease, Sjogren's syndrome, Hashimoto's thyroiditis, myasthenia gravis, vasculitis, pernicious anemia, celiac disease, autoimmune hepatitis, alopecia areata, pemphigus vulgaris, vitiligo, aplastic anemia, autoimmune uveitis, Alopecia Areata, Amyotrophic Lateral Sclerosis (Lou Gehrig's), Ankylosing Spondylitis, Anti-GBM Nephritis, Antiphospholipid Syndrome, Osteoarthritis, Autoimmune Active Chronic Hepatitis, Autoimmune Inner Ear Disease (AIED), Balo Disease, Behcet's Disease, Berger's Disease, Bullous Pemphigoid, Cardiomyopathy, Chronic Fatigue Immune Dysfunction Syndrome, Churg Strauss Syndrome, Cicatricial Pemphigoid, Cold Agglutinin Disease, Colitis Cranial Arteritis, Crest Syndrome, Crohn's Disease, Dego's Disease, Dermatomyositis & JDM, Devic Disease, Eczema, Essential Mixed Cryoglobulinemia, Eoscinophilic Fascitis, Fibromyalgia - Fibromyositis, Fibrosing Alveolitis, Giant Cell Arteritis, Glomerulonephritis, Goodpasture's Disease, Guillain-Barre Syndrome, Hashimoto's Thyroiditis, Hepatitis, Hughes Syndrome, Idiopathic Pulmonary Fibrosis, Idiopathic Thrombocytopenic Purpura, Irritable Bowel Syndrome, Kawasaki's Disease, Lichen Planus, Lupoid Hepatitis, Lupus / SLE, Lyme Disease, Meniere's Disease, Mixed Connective Tissue Disease, Myositis: Juvenile Myositis (JM), Juvenile dermatomyositis (JDM), and Juvenile Polymyositis (JPM), Osteoporosis, Pars Planitis, Pemphigus Vulgaris, Polyglandular Autoimmune Syndromes, Polymyalgia Rheumatica, Polymyositis, Primary Biliary Cirrhosis, Primary Sclerosis Cholangitis, Psoriasis, Raynaud's Syndrome, Reiter's Syndrome, Rheumatic Fever, Rheumatoid Arthritis, Scleritis, Scleroderma, Sticky Blood Syndrome, Still's Disease, Stiff Man Syndrome, Sydenham's Chorea, Takayasus Arteritis, Temporal Arteritis, Ulcerative Colitis, Uveitis, Vasculitis, Wegener's Granulomatosis and Wilson's Syndrome, preferably the autoimmune disease is vasculitis such as catastrophic anti-phospholipid syndrome (also named Asherson's syndrome), Giant Cell Arteritis and anti- ANCA vasculitis or myasthemia gravis, refractory celiac disease, autoimmune uveitis such as Behcet's Disease, pemphigus vulgaris, giant cell myocarditis, Graves' disease, Addison's disease and granulomatosis with polyangiitis,
preferably the inflammatory or autoinflammatory disease is a chronic inflammatory disease, preferably the chronic inflammatory disease is selected from the group consisting of: inflammatory bowel disease, Chron's disease, ulcerative colitis, celiac disease,
more preferably the engineered cell of claim 12 or 13 is for use in cancer immunotherapy.
